# EUROPEAN PATENT SPECIFICATION

(11) **EP 1 042 468 B1**
(45) Date of publication and mention of the grant of the patent: **18.10.2006**
(21) Application number: 98962884.7
(22) Date of filing: 03.12.1998
(51) Int. Cl.: C12N 15/12, C07K 14/705, C07K 16/28, C12N 5/10, G01N 33/68

(54) **LOW-VOLTAGE ACTIVATED CALCIUM CHANNEL COMPOSITIONS AND METHODS**
ZUSAMMENSETZUNGEN VON DURCH NIEDERVOLT AKTIVIERTEN KALZIUMKANÄLEN UND VERFAHREN
COMPOSITIONS A CANAUX CALCIUM ACTIVES A BASSE TENSION ET PROCEDES CORRESPONDANTS

(30) Priority: 03.12.1997 US 984709; 10.11.1998 US 188932
(43) Date of publication of application: 11.10.2000
(73) Proprietor: Merck & Co., Inc., Rahway, New Jersey 07065 (US)
(72) Inventor: WILLIAMS, Mark, Carlsbad, CA 92009 (US); STAUDERMAN, Kenneth, San Diego, CA 92106 (US); HARPOLD, Michael, Santa Fe, NM 87505-4726 (US)
(74) Representative: Horgan, James Michael Frederic
(86) International application number: PCT/US1998/025671
(87) International publication number: WO 1999/028342

(56) References cited:
- WO-A-93/04083
- WO-A-95/04144
- NOONEY JM (REPRINT) ET AL: "Identifying neuronal non-L Ca2+ channels - more than stamp collecting?" TRENDS IN PHARMACOLOGICAL SCIENCES, 10-1997, 18, 363-371, XP002093637
- ERTEL S I ET AL: "Low-voltage-activated T-type Cachannels" TRENDS IN PHARMACOLOGICAL SCIENCES, vol. 18, no. 2, February 1997, page 37-42 XP004055849
- DZHURA IO ET AL: "Characterization of hypothalamic low-voltage-activated Ca channels based on their functional expression in Xenopus oocytes." NEUROSCIENCE, FEB 1996, 70 (3) P729-38, XP002093638 UNITED STATES
- CRIBBS LL ET AL: "Cloning and characterization of alpha1H from human heart, a member of the T-type Ca2+ channel gene family." CIRC RES, JUL 13 1998, 83 (1) P103-9, XP002093640 UNITED STATES
- WILLIAMS ME ET AL: "Structure and functional characterization of a novel human low-voltage activated calcium channel." J NEUROCHEM, FEB 1999, 72 (2) P791-9, XP002106656 UNITED STATES

## Description

### TECHNICAL FIELD

The present invention relates to molecular biology and pharmacology. More particularly, the invention relates to calcium channel compositions and methods of making and using the same.

### BACKGROUND OF THE INVENTION

Calcium channels are membrane-spanning, multi-subunit proteins that allow controlled entry of Ca²⁺ ions into cells from the extracellular fluid. Cells throughout the animal kingdom, and at least some bacterial, fungal and plant cells, possess one or more types of calcium channel. The most common type of calcium channel is voltage dependent. All "excitable" cells in animals, such as neurons of the central nervous system (CNS), peripheral nerve cells and muscle cells, including those of skeletal muscles, cardiac muscles, and venous and arterial smooth muscles, have voltage-dependent calcium channels (VGCCs). "Opening" of a voltage-dependent channel to allow an influx of Ca²⁺ ions into the cells requires a depolarization to a certain level of the potential difference between the inside of the cell bearing the channel and the extracellular environment bathing the cell. The rate of influx of Ca²⁺ into the cell depends on this potential difference.

Calcium channels are multisubunit proteins that contain two large subunits, designated α₁ and α₂, which have molecular weights between about 130 and about 200 kilodaltons ("kD"), and one to three different smaller subunits of less than about 60 kD in molecular weight. At least one of the larger subunits and possibly some of the smaller subunits are glycosylated. Some of the subunits are capable of being phosphorylated. The α₁ subunit has a molecular weight of about 150 to about 170 kD when analyzed by sodium dodecylsulfate (SDS)-polyacrylamide gel electrophoresis (PAGE) after isolation from mammalian muscle tissue and has specific binding sites for various 1,4-dihydropyridines (DHPs) and phenylalkylamines. Under non-reducing conditions (in the presence of N-ethylmaleimide), the α₂ subunit migrates in SDS-PAGE as a band corresponding to a molecular weight of about 160-190 kD. Upon reduction, a large fragment and smaller fragments are released. The *β* subunit of the rabbit skeletal muscle calcium channel is a phosphorylated protein that has a molecular weight of 52-65 kD as determined by SDS-PAGE analysis. This subunit is insensitive to reducing conditions. The *γ* subunit of the calcium channel appears to be a glycoprotein with an apparent molecular weight of 30-33 kD, as determined by SDS-PAGE analysis.

In order to study calcium channel structure and function, large amounts of pure channel protein are needed. Because of the complex nature of these multisubunit proteins, the varying concentrations of calcium channels in tissue sources of the protein, the presence of mixed populations of calcium channels in tissues, difficulties in obtaining tissues of interest, and the modifications of the native protein that can occur during the isolation procedure, it is extremely difficult to obtain large amounts of highly purified, completely intact calcium channel protein.

Because calcium channels are present in various tissues and have a central role in regulating intracellular calcium ion concentrations, they are implicated in a number of vital processes in animals, including neurotransmitter release, muscle contraction, pacemaker activity, and secretion of hormones and other substances. These processes appear to be involved in numerous human disorders, such as central nervous system disorders and cardiovascular diseases. Calcium channels, thus, are also implicated in numerous disorders. A number of compounds useful for treating various cardiovascular diseases in animals, including humans, are thought to exert their beneficial effects by modulating functions of voltage-dependent calcium channels present in cardiac and/or vascular smooth muscle. Many of these compounds bind to calcium channels and block, or reduce the rate of, influx of Ca²⁺ into the cells in response to depolarization of the cell membrane.

The results of studies of recombinant expression of rabbit calcium channel α₁ subunit-encoding cDNA clones and transcripts of the cDNA clones indicate that the α₁ subunit forms the pore through which calcium enters cells. The relevance of the barium currents generated in these recombinant cells to the actual current generated by calcium channels containing as one component the respective α₁ subunits *in vivo* is unclear. In order to completely and accurately characterize and evaluate different calcium channel types, however, it is essential to examine the functional properties of recombinant channels containing all of the subunits as found *in vivo.*

In order to conduct this examination and to fully understand calcium channel structure and function, it is critical to identify and characterize as many calcium channel subunits as possible. Also in order to prepare recombinant cells for use in identifying compounds that interact with calcium channels, it is necessary to be able to produce cells that express uniform populations of calcium channels containing defined subunits.

An understanding of the pharmacology of compounds that interact with calcium channels in other organ systems, such as the CNS, may aid in the rational design of compounds that specifically interact with subtypes of human calcium channels to have desired therapeutic effects, such as in the treatment of neurodegenerative and cardiovascular disorders. Such understanding and the ability to rationally design therapeutically effective compounds, however, have been hampered by an inability to independently determine the types of human calcium channels and the molecular nature of individual subtypes, particularly in the CNS, and by the unavailability of pure preparations of specific channel subtypes to use for evaluation of the specificity of calcium channel-effecting compounds. Thus, identification of DNA encoding human calcium channel subunits and the use of such DNA for expression of calcium channel subunits and functional calcium channels would aid in screening and designing therapeutically effective compounds.

Multiple types of calcium channels have been identified in mammalian cells from various tissues, including skeletal muscle, cardiac muscle, lung, smooth muscle and brain, (see, *e.g.,* Bean, B.P.(1989) *Ann. Rev. Physiol. 51*:367-384 and Hess, P. (1990) *Ann. Rev. Neurosci. 56*:337). The different types of calcium channels have been broadly categorized into four classes, L-, T-, N-, P-, Q and R-type, distinguished by current kinetics, holding potential sensitivity and sensitivity to calcium channel agonists and antagonists. The primary determinant of diversity among calcium channels is the nature of the pore-forming α₁ subunit. Nucleic acid encoding numerous α₁ subunits has been cloned and the encoded subunits expressed. Correlations between α₁ subunits and the operationally defined Ca²⁺ currents have been established. Six gene products α_{1A}-α_{1-E} and α_{1S} participate in the formation of high-voltage activated channels, which include the L, N, P, Q and R-type channels.

DNA encoding human α₁-subunits, including α_{1A}-, α_{1B}-, *α*_{1C}*-, α*_{1D}*-* and α_{1E} subunits and splice variants thereof has been described (see, e.g., U.S. Patent No. 5,429,921, U.S. Patent No. 5,846,756, U.S. Patent No. 5,851,824, published International PCT application No. PCT/US92/06903, and published International PCT application No. PCT/US94/09230). These subunits appear to participate in formation of high voltage calcium (HVA) channels, which in addition to one of these α₁-subunits, includes a β subunit and an α₂-Subunit, including δ, which is linked to α₂ by a disulfide bridge and arises from the same precursor. The distinct biophysical and pharmacological properties of each channel derive primarily form the α₁-subunit, but are modulated by the ancillary subunits, principally the β subunits associated with the channel. β-subunits have been shown to increase the peak current amplitude, to shift activation/inactivation curves toward more hyperpolarized potentials and to alter kinetics of activation and inactivation (see, e.g., Lambert et al. (1997) J. Neurosci. 17:6621-6625). The *α*₂*δ* subunit, which is tissue-specific, increases the current generated by any α₁ subunit and potentiates the stimulatory response of β subunits.

### T-type or LVA channels

Little is known about the channels that have been designated T-channels or LVA (low voltage activated) channels. Low-voltage activated (LVA), i.e., T-type, calcium channels are reportedly found in a variety of cell types. Low-voltage activated (LVA) or T-type calcium channels are also widely distributed in the central and peripheral nervous system and apparently involved in an extensive array of different neuronal processes.

In general it is believed that T-type currents do not differ fundamentally from other Ca²⁺ currents. Like HVA channels, T-type channels are selectively permeable to divalent cations, as long as a minimal concentration of divalent cations is present in the external medium. For LVA (or T-type) currents, this minimal Ca²⁺ concentration is about 25 µm, and for HVA currents it is about 1 µM. T-type current is reported to saturate with a K_{d} of about 10 mM Ca²⁺, which is similar to that reported for HVA currents. The channels, however, appear to exhibit certain differences. They differ in their relative permeability to divalent cations. In general, HVA channels are more permeable to Ba²⁺ than to Ca²⁺; T-type are equally or slightly less permeable to Ba²⁺ than to Ca²⁺. T-type channels also are believed to exhibit slower activation/inactivation and deactivation kinetics and have been reported to exhibit relatively higher sensitivity to Ni²⁺. This type of channel is activated near the resting potential of the membrane, and is believed to be responsible for the generation of repetitive firing activity or intrinsic neuronal oscillations and for Ca²⁺ entry accompanying the spike activity (see, e.g., Huguenard (1996) Annual Rev. Physiol. 58:329-348). Recent data suggests that β-subunits identified to date may not be a constitutive T-type channel subunit (see, Lambert et al. (1997) J. Neurosci. 17:6621-6625). The structure of calcium channels that generate the various LVA currents is unknown. None of the α₁ subunits previously cloned appear to have all properties that have been ascribed to the low voltage-activated T-type (or LVA) channels.

Cribbs LL (1998) Circ Res, 83(1):103-109 describes the cloning and characterization of alpha 1H subunit of the T-type Ca²⁺ channel gene family from human heart (document published of July 1998).

It is an object herein, to provide nucleic acid encoding specific calcium channel subunits that have structural and functional properties that differ from the HVA type channels. It is also an object herein to provide nucleic acid encoding channels that have activities that have been ascribed to T-type channels and to provide eukaryotic cells bearing recombinant tissue-specific or subtype-specific calcium channels. It is also an object to provide assays for identification of potentially therapeutic compounds that act as modulators of calcium channel activity, particularly those specific for channels that exhibit properties of human T-type channels and other types of channels.

### SUMMARY OF THE INVENTION

Isolated and purified nucleic acid fragments that encode calcium channel subunits are provided. The subunits form low-voltage activated (LVA) channels, particularly channels that have properties associated with T-type channels. The subunits and results provided herein, provide a family of α_{1H} subunits corresponding to LVA, or T-type, channels. Channels that contain these subunits have ability to open at low potential difference, but stay open for only moderate time periods. These channels are located in critical physiologic locations, including neurons in the thalamus, hypothalamus, and brain stem, and consequently may be involved in autonomic nervous functions, perhaps involved in regulation of cardiovascular activities such as heart rate, arterial and venous smooth muscle innervation and tone, pulmonary rate and other critical physiologic activities.

DNA encoding these α_{1H} subunits of a animal channels, and RNA, encoding such subunits, made upon transcription of such DNA are provided. In particular, nucleic acid that encodes T-type calcium channels, designated α_{1H}-subunits (designated α_{1F} in the priority document U.S. application Serial No. 08/984,709) of a calcium channel, particularly an animal calcium channel and more particularly a mammalian calcium channel is provided.

Of particular interest herein is the nucleic acid that encodes the α_{1H} subunits of calcium channels, particularly mammalian calcium channels. Nucleic acid encoding exemplary α_{1H} subunits are provided. Nucleic acid encoding two splice variants, designated α_{1H-1} and α_{1H-2}, from human calcium channels is provided. The nucleic acid sequences and encoded amino acids of the exemplified subunits are set forth in SEQ ID Nos. 12 (α_{1H-1}), 15 (α_{1H-1}) and 16 (α_{1H-2}). SEQ ID NOs. 1 2 and 15 differ only in that in amino acid 2230 (bases 6983-6985) is Asp (GAC) in the SEQ ID No. 15 and Glu (GAA) in SEQ ID No. 12.

This nucleic acid can be used to isolate variants, including additional splice variants of the nucleic acid encoding α_{1H} subunits, allelic variants and α_{1H} subunits from other animals, particularly mammals. Such nucleic acid includes DNA encoding an α_{1H-1} subunit that has substantially the same sequence of amino acids as encoded by the DNA set forth in SEQ ID Nos. 12 and 15. This nucleic acid can also be used to isolate DNA encoding α_{1H} subunits from other species, particularly other mammals.

Also provided is nucleic acid that encodes a second splice variant, designated α_{1H-2}, is provided. The nucleic acid sequence of this variant, differs from α_{1H-1} in having a 957 nucleotide deletion, resulting in loss of 319 amino acids (corresponding to amino acids 470-788 of α_{1H-1}).

Also included are any subunits that are encoded by nucleic acid containing nucleotides nt 1506 to nt 2627 of SEQ ID No. 12 or 15 or subunits that are encoded by nucleic acid that hybridizes, preferably under conditions of high stringency, to a probe derived from this region and that encodes a T-channel, which can be identified using methods herein.

The α_{1H} subunit differs from the α_{1A}-α_{1E} calcium channel subunits in a number of aspects. First, the intracellular loop positioned between transmembrane Domains I and II is considerably longer than HVA calcium channels. For instance, as exemplified in SEQ ID Nos. 12 and 15 and described below, the intracellular loop between Domains I and II is greater than 1,100 nt (1122 nt), whereas the corresponding region in HVA calcium channels ranges from 351 to 381 nt in length. Thus, the intracellular loop of α_{1H} contains approximately 370 additional amino acid residues (aa 420 to aa 794 of SEQ ID No. 12) not found in HVA calcium channel α₁ subunits. In addition, the encoded amino acid sequence of this loop region is highly proline rich and contains a poly-HIS region of 9 consecutive histidine residues.

Other distinguishing features of the α_{1H} subunit, include the absence of amino acid residues in the intracellular loop between transmembrane Domains I and II that are known to be critical (e.g., see De Waard *et al.* (1996) FEBS Letters 380:272-276; Pragnell *et al.* (1994) Nature 368:67-70) for the interaction between an α₁ subunit and a β subunit. The α_{1H} subunit also contains a notably large extracellular loop in Domain I between IS5 and IS6. T he HVA α₁ calcium channel subunits provided herein contain 249-270 nucleotide residues in this loop. In contrast, the human α_{1H} subunit contains 426 nucleotide residues in this loop. The intracellular loop between transmembrane Domains III and IV is also slightly larger than the HVA α₁ subunits (186 nt compared to 159-165 nt).

Nucleic acid probes, which can be labeled for detection, containing at least 16, or, if desired, 20 or 30 or more, contiguous nucleotides of α_{1H}-encoding nucleic acid are disclosed Methods using the probes for the isolation and cloning of calcium channel subunit-encoding DNA, including splice variants within tissues and inter-tissue variants are also disclosed. Particularly preferred regions from which to construct probes for the isolation of DNA encoding a human α_{1H} subunit include the nucleic acid sequence encoding the notably long intracellular loop located between transmembrane Domains I and II (e.g., nt 1506 to nt 2627 of SEQ ID Nos. 12 and 15). Probes for isolating DNA encoding a human α_{1H} subunit are preferably 16 contiguous nucleotides in length. In some instances, probes of 30 or 50 nucleotides are used and in other instances probes between 50 to 100 nucleotides are used.

Eukaryotic cells containing heterologous DNA encoding one or more calcium channel subunits, particularly human calcium channel subunits, or containing RNA transcripts of DNA clones encoding one or more of the subunits are provided. A single α_{1H} subunit can form a channel. The requisite combination of subunits for formation of active channels in selected cells, however, can be determined empirically using the methods herein. For example, if a selected α₁ subtype or variant does not form an active channel in a selected cell line, an additional subunit or subunits can be added until an active channel is formed. Other subunits can be added to assess the effects of such addition.

In preferred embodiments, the cells contain DNA or RNA encoding an α_{1H} subunit of an animal, preferably of a mammalian calcium channel. Embodiments in which the cells contain nucleic acid encoding an α_{1H} are of particular interest herein. In other embodiments, the cells contain DNA or RNA encoding additional heterologous subunits, including an α₂δ. The cells may also include nucleic acid encoding a β subunit and/or a γ subunit. In such embodiments, eukaryotic cells stably or transiently transfected with any combination of one, two, three or four of the subunit-encoding DNA clones, such as DNA encoding any of *α*₁*, α*₁ + *β,* α₁ + β + α₂, are provided. The eukaryotic cells provided herein contain heterologous nucleic acid that encodes an α_{1H} subunit and optionally a heterologous α₂₋subunit and/or a β subunit and/or γ subunit.

In preferred embodiments, the cells express such heterologous calcium channel subunits and include one or more of the subunits in membrane-spanning heterologous calcium channels. In more preferred embodiments, the eukaryotic cells express functional, heterologous calcium channels that are capable of gating the passage of calcium channel-selective ions and/or binding compounds that, at physiological concentrations, modulate the activity of the heterologous calcium channel. In certain embodiments, the heterologous calcium channels include at least one heterologous calcium channel subunit. In most preferred embodiments, the calcium channels that are expressed on the surface of the eukaryotic cells are composed substantially or entirely of subunits encoded by the heterologous DNA or RNA. In preferred embodiments, the heterologous calcium channels of such cells are distinguishable from any endogenous calcium channels of the host cell. Such cells provide a means to obtain homogeneous populations of calcium channels. Typically, the cells contain the selected calcium channel as the only heterologous ion channel expressed by the cell.

In certain embodiments the recombinant eukaryotic cells that contain the heterologous DNA encoding the calcium channel subunits are produced by transfection with DNA encoding one or more of the subunits or are injected with RNA transcripts of DNA encoding one or more of the calcium channel subunits. The DNA may be introduced as a linear DNA fragment or may be included in an expression vector for stable or transient expression of the subunit-encoding DNA. Vectors containing DNA encoding human calcium channel subunits are also provided.

The eukaryotic cells that express heterologous calcium channels may be used in assays for calcium channel function or, in the case of cells transformed with fewer subunit-encoding nucleic acids than necessary to constitute a functional recombinant human calcium channel, such cells may be used to assess the effects of additional subunits on calcium channel activity. The additional subunits can be provided by subsequently transfecting such a cell with one or more DNA clones or RNA transcripts encoding human calcium channel subunits.

The recombinant eukaryotic cells that express membrane spanning heterologous calcium channels may be used in methods for identifying compounds that modulate calcium channel activity. In particular, the cells are used in assays that identify agonists and antagonists of calcium channel activity in humans and/or assessing the contribution of the various calcium channel subunits to the transport and regulation of transport of calcium ions. Because the cells constitute homogeneous populations of calcium channels, they provide a means to identify agonists or antagonists of calcium channel activity that are specific for each such population.

The cells provided herein may be used to assess T-type channel function and tissue distribution and to identify compounds that modulate the activity of T-type channels. Because T-type channels are operative in neurons in the thalamus, hypothalamus, and brain stem, and may be involved in autonomic nervous functions, in regulation of cardiovascular activities such as heart rate, arterial and venous smooth muscle innervation and tone, pulmonary rate and other fundamental processes, assays designed to assess such activities and assays the identify modulators of these activities provide a means to understand fundamental physiological processes and also a means to identify new drug candidates for an array of disorders.

Assays that use the eukaryotic cells for identifying compounds that modulate calcium channel activity are also provided. In practicing these assays the eukaryotic cell that expresses a heterologous calcium channel, containing at least one subunit encoded by the DNA provided herein, is in a solution containing a test compound and a calcium channel selective ion, the cell membrane is depolarized, and current flowing into the cell is detected. If the test compound is one that modulates calcium channel activity, the current that is detected is different from that produced by depolarizing the same or a substantially identical cell in the presence of the same calcium channel-selective ion but in the absence of the compound. In preferred embodiments, prior to the depolarization step, the cell is maintained at a holding potential which substantially inactivates calcium channels which are endogenous to the cell. Also in preferred embodiments, the cells are mammalian cells, most preferably HEK cells, or amphibian oöcytes.

Cells that express T-channels or LVA channels may be used in assays that screen for compounds that have activity as modulators, particularly antagonists, of the activity of these channels.

Transcription based assays for identifying compounds that modulate the activity of calcium channels (see, U.S. Patent Nos. 5,436,128 and 5,401,6291, particularly calcium channels that contain an α_{1H} subunit are provided. These assays use cells that express calcium channels, particularly calcium channels containing an α_{1H}-subunit, and more preferably an α_{1H}-subunit encoded by heterologous DNA, and also contain nucleic acid encoding a reporter gene construct containing a reporter gene in operative linkage with one or more transcriptional control elements that is regulated by a calcium channel. The assays are effected by comparing the difference in the amount of transcription of a the reporter gene in the cells provided herein in the presence of the compound with the amount of transcription in the absence of the compound, or with the amount of transcription in the absence of the heterologous calcium channel, whereby compounds that modulate the activity of the heterologous calcium channel in the cell are identified. The reporter gene is any such gene known to those of skill in the art, including, but not limited to the gene encoding bacterial chloramphenicol acetyltransferase, the gene encoding firefly luciferase, the gene encoding bacterial luciferase, the gene encoding β-galactosidase or the gene encoding alkaline phosphatase, and the transcriptional control element is any such element known to those of skill in the art, including, but not limited to serum responsive elements, cyclic adenosine monophosphate responsive elements, the *c-fos* gene promoter, the vasoactive intestinal peptide gene promoter, the somatostatin gene promoter, the proenkephalin promoter, the phosphoenolpyruvate carboxykinase gene promoter or the nerve growth factor-1 A gene promoter and elements responsive to intracellular calcium ion levels.

Other assays in which receptor activity in response to test compounds is measured may also be practiced with the cells provided herein (see, e.g., U.S. Patent No. 5,670,113).

Because T-type channels appear to be associated with a variety of key functions, cells that express T-channels and assays using such cells will be useful for identification of compounds for treatment of a variety of disorders, disease and conditions. Identified compounds will be candidates for use in the treatment of disorders and conditions associated with T-channel activity. Such activities include, but are not limited to, those involving role in muscle excitability, secretion and pacemaker activity, Ca²⁺ dependent burst firing, neuronal oscillations, and potentiation of synaptic signals, for improving arterial compliance in systolic hypertension, or improving vascular tone, such as by decreasing vascular welling, in peripheral circulatory disease, and others. Other disorders include, but are not limited to hypertension, cardiovascular disorders, including but not limited to: myocardial infarct, cardiac arrhythmia, heart failure and angina pectoris; neurological disorders, such as schizophrenia, epilepsy and depression, peripheral muscle disorders, respiratory disorders and endocrine disorders.

In particular, cells that express LVA channels, such as the α_{1H} subunits, are useful for identifying compounds that are candidates for treatment of disorders associated with conduction tissues, such as atrial pacemaker cells, Purkinje fibers, and also coronary smooth muscles. Such disorders include, but are not limited to, compounds useful for treatment of cardiovascular, such as angina, vascular, such as hypertension, and urologic, hepatic, reproductive, adjunctive therapies for reestablishing normal heart rate and cardiac output following traumatic injury, heart attack and other cardiac injuries; treatments of myocardial infarct (MI), post-MI and in an acute setting. Other compounds that interact with LVA, particularly T-type, calcium channels, may be effective for increasing cardiac contractile force, such as measured by left ventricular end diastolic pressure, and without changing blood pressure or heart rate. In an acute other compounds may be effective to decrease formation of scar tissue, such as that measured by collagen deposition or septal thickness, and without cardiodepressant effects. The assays may identify compounds useful in regulating vascular smooth muscle tone, either vasodilating or vasoconstricting in: (a) treatments for reestablishing blood pressure control, e.g., following traumatic injury, surgery or cardiopulmonary bypass, and in prophylactic treatments designed to minimize cardiovascular effects of anaesthetic drugs; (b) treatments for improving vascular reflexes and blood pressure control by the autonomic nervous system; for identifying compounds useful in treating urological disorders: (a) treating and restoring renal function following surgery, traumatic injury, uremia and adverse drug reactions; (b) treating bladder dysfunctions; and (c) uremic neuronal toxicity and hypotension in patients on hemodialysis; reproductive disorders, for identifying compounds useful in treating: (a) disorders of sexual function including impotence; (b) alcoholic impotence (under autonomic control that may be subject to T-channel controls); hepatic disorders for identifying compounds useful in treating and reducing neuronal toxicity and autonomic nervous system damage resulting from acute over-consumption of alcohol; neurologic disorders for identifying compounds useful in treating: (a) epilepsy and diencephalic epilepsy; (b) Parkinson's disease; (c) aberrant temperature control, such as, abnormalities of shivering and sweat gland secretion and peripheral vascular blood supply; (d) aberrant pituitary and hypothalamic functions including abnormal secretion of noradrenaline, dopamine and other hormones; for respiratory such as in treating abnormal respiration, e.g., post-surgical complications of anesthetics; and endocrine disorders, for identifying compounds useful in treating aberrant secretion of hormones including e.g., possible treatments for overproduction of insulin, thyroxin, adrenalin, and other hormonal imbalances.

Purified human α_{1H} calcium channel subunits and purified human calcium channels containing such subunits are provided. The subunits and channels can be isolated from a eukaryotic cell transfected with nucleic acid that encodes the subunit.

Immunoglobulins or antibodies obtained from the serum of an animal immunized with a substantially pure preparation of a human calcium channel, human calcium channel subunit or epitope-containing fragment of a human calcium subunit are provided. Monoclonal antibodies produced using a human calcium channel, human calcium channel subunit or epitope-containing fragment thereof as an immunogen are also provided. *E. coli* fusion proteins including a fragment of a human calcium channel subunit may also be used as immunogen. Such fusion proteins may contain a bacterial protein or portion thereof, such as the *E. coli* TrpE protein, fused to a calcium channel subunit peptide. The immunoglobulins that are produced using the calcium channel subunits or purified calcium channels as immunogens have, among other properties, the ability to specifically and preferentially bind to and/or cause the immunoprecipitation of a human calcium channel or a subunit thereof which may be present in a biological sample or a solution derived from such a biological sample. Such antibodies may also be used to selectively isolate cells that express calcium channels that contain the subunit for which the antibodies are specific.

Methods for modulating the activity of ion channels by contacting the calcium channels with an effective amount of the above-described antibodies are also provided.

Thus, assays for identifying compounds that modulate the activity of LVA calcium channels, particularly T-type channels are provided as well as compounds identified by the methods.

Also provided are methods for diagnosing LVA calcium channel-mediated, particularly T-type channel-mediated, disorders. Methods of diagnosis will involve detection of aberrant channel expression or function, such altered amino acid sequences, altered pharmacological profiles and altered electrophysiological profiles compared to normal or wild-type channels. Such methods typically can employ antibodies specific for the altered channel or nucleic acid probes to detect altered genes or transcripts.

### DESCRIPTION OF THE FIGURES

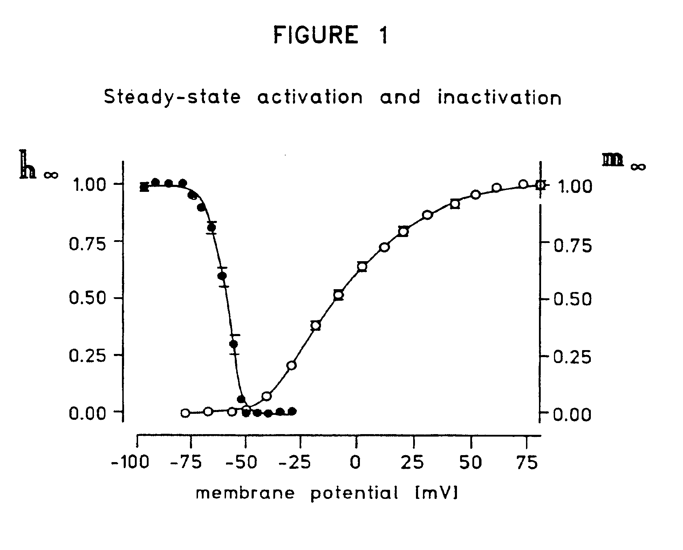

FIGURE 1 shows the voltage-dependence of activation (moo) and steady-state inactivation (h) of human α_{1H} calcium channels expressed transiently in HEK cells. Voltage-dependence of activation (moo) was determined from tail current analysis. Tail currents were normalized with respect to the maximum peak tail current obtained at + 60 mV and were plotted (open symbols, mean ± SEM; n = 11) vs. test potential. Data were fitted by the sum of two Boltzman function m∞ = FA*[1+exp (-(Vtest-V1/2,A)/KA)]1 +F_{B}*[1 +exp(-(Vₜₑₛₜ-V_{1/2.B})/k_{B})]⁻¹, F_{A}=0.67, V_{1/2.A}=-21.5mV, k_{A} = 7.5, F_{B} = 0.33, V_{1/2.B} = 25.5 mV, k_{B} = 14.7. Steady-state inactivation (h∞) was determined from a holding potential of -100 mV by a test pulse to -20 mV (p1), followed by a 20 second prepulse from -100 mV to -10 mV in 5 mV decrements (pHold) preceding a second test pulse to -20 mV (p2). Normalized current amplitudes were plotted (closed symbols, mean ± SEM; n = 9) vs. holding potential. Data were fitted by a Boltzman function h∞=[1 +exp((V_{hold}-V_{1/2})/k)]⁻¹, V_{1/2}=-63.9 mV, k=3. 9mV.

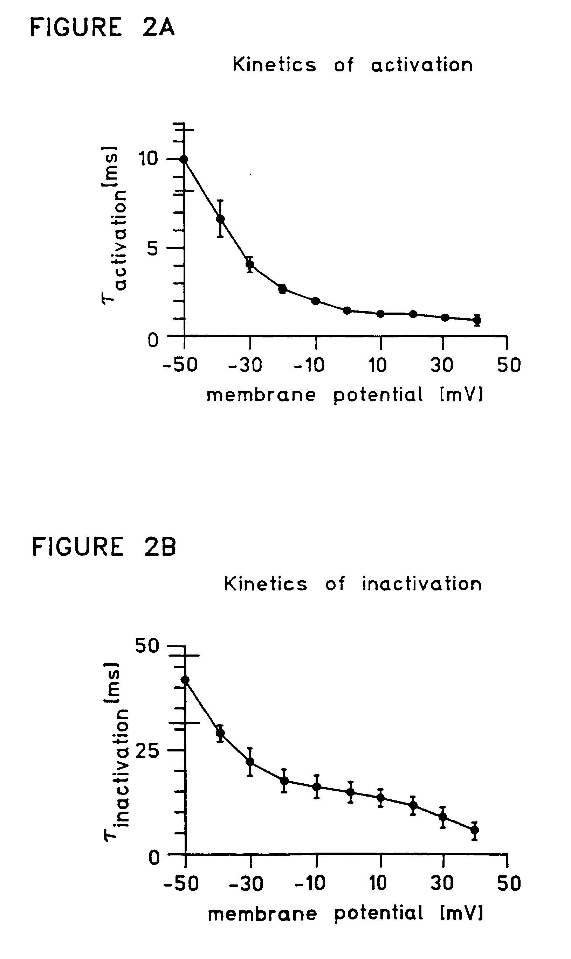

FIGURE 2 shows the kinetics of activation (FIGURE 2A) and inactivation (FIGURE 2B) of human α_{1H} (α_{1H-1}) calcium channels; kinetics of activation and inactivation were determined from current traces by fitting an exponential function to rising (FIG. 2A) or declining (FIG. 2B) phase of the current (the voltage-dependence for activation and inactivation follows approximately an exponential function).

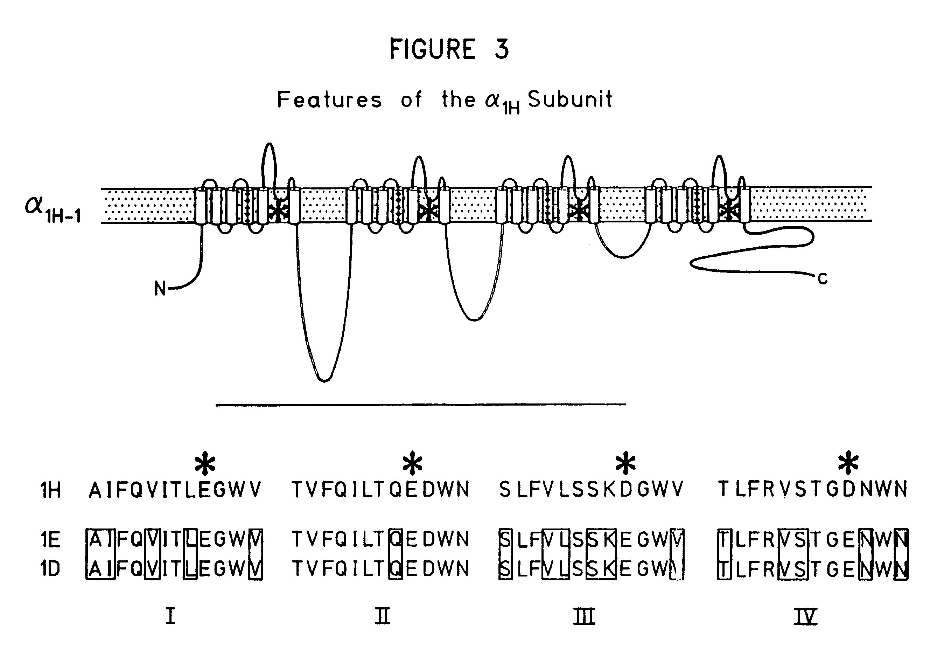

FIGURE 3 schematically depicts features of the α_{1H-1} subunit and shows amino acid sequence alignment of human α_{1H} with α_{1D} and α_{1E} in each of the four pore regions; *indicates residues involved in ion selectivity in each of the four pore regions; the unusually large loop in the LVA-associated α_{1H} subunits between transmembrane domains I and II.

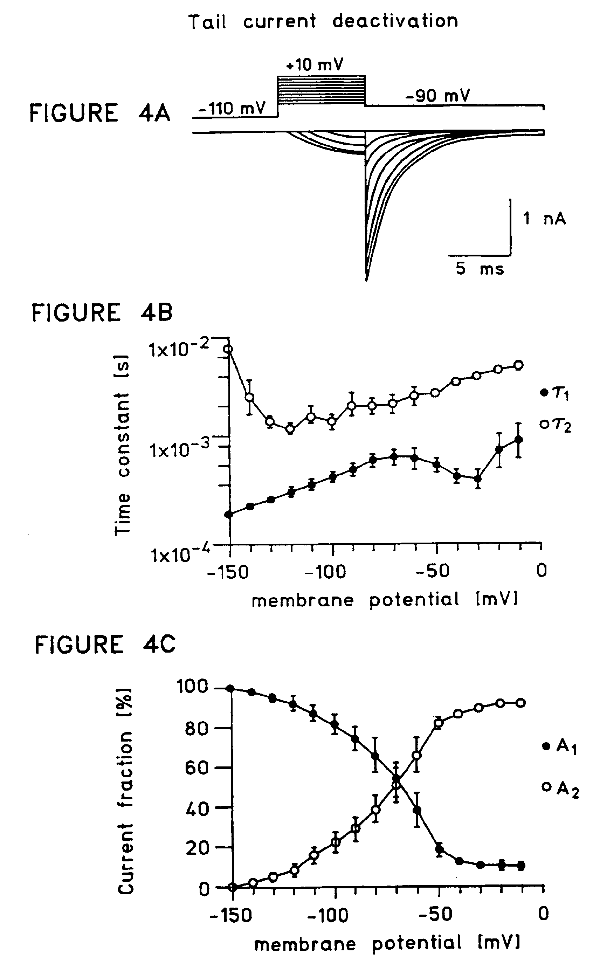

FIGURE 4A shows the tail currents elicited by repolarization to -90 mV following 10 ms step depolarizations between -80 and -10 mV, For tail current measurements the digitization/filter rates were 50/16 kHz. Tail current decay was fitted to a bi-exponential function of the form I=A₀ + A₁ exp(-t/τ₁) + A₂ exp(-t/τ₂). The bi-exponential decay profile of the tail current was observed in every cell examined (n= 12). FIGURES 4B and 4C show the voltage-dependence of the time constants τ₁ and τ₂ for current deactivation (FIGURE 4B) and the current fractions A₁ and A₂ (FIGURE 4C).

### DETAILED DESCRIPTION OF THE INVENTION

### Definitions:

Unless defined otherwise, all technical and scientific terms used herein have the same meaning as is commonly understood by one of skill in the art to which this invention belongs.

Reference to each of the calcium channel subunits includes the subunits that are specifically disclosed herein and human calcium channel subunits encoded by nucleic acid that can be isolated by using the nucleic acid disclosed as probes and screening an appropriate human cDNA or genomic library under at least low stringency, preferably high stringency. Such DNA also includes DNA that encodes proteins that have about 40% homology, typically at least about 90% sequence identity taking into account gaps) to any of the subunits proteins described herein or DNA or RNA that hybridizes under conditions of at least low stringency to the DNA provided herein and the protein encoded by such DNA exhibits additional identifying characteristics, such as function or molecular weight. In particular, reference to an α_{1H} subunit refers to subunits that can be isolated from nucleic acid libraries from any desired source using the nucleic acid disclosed herein as a probe. The encoded subunit is characterized by the presence of the notably long intracellular loop between transmembrane domains I and II, and/or properties ascribed to T-type or LVA type channels.

It is understood that subunits that are encoded by transcripts that represent splice variants of the disclosed subunits or other such subunits may exhibit less than 40% overall homology to any single subunit, but will include regions of such homology to one or more such subunits. It is also understood that 40% homology refers to proteins that share approximately 40% of their amino acids in common or that share somewhat less, but include conservative amino acid substitutions, whereby the activity of the protein is not substantially altered.

The subunits and DNA fragments encoding such subunits are provided herein or known to those of skill in the art (see, published International PCT application Nos. WO89/09834, WO93/04083, WO95/04822, U.S. Patent Nos. 5,792,846, 5,726,035, 5,407,820, 5,686,241, 5,618,720, 5,710,250, 5,429,921, 5,429,921 and 5,386,025) include any α₁, α₂, β or γ subunits of a human calcium channel.

Nucleic acid encoding LVA subunits, particularly α_{1H} subunits of human and other animal calcium channels, are provided herein. In particular, such DNA fragments include an isolated nucleic acid fragment that encodes a low-voltage activated α_{1H} subunit of an animal calcium channel wherein the sequence of nucleotides comprises the coding portion of the sequence of nucleotides set forth in any of SEQ ID Nos. 12-16.

In one embodiment is a nucleic acid which encodes an α_{1H-1} or α_{1H-2} subunit which comprises the coding portion of the Sequence of nucleotides set forth in any of SEQ ID Nos. 12, 15 or 16.

As used herein, the α₁ subunit types, encoded by different genes, are designated as type α_{1A}, α_{1B}, α_{1C}, α_{1D}, α_{1E} and α_{1H}. These types have also been referred to as VDCC IV for α_{1B}, VDCC II for α_{1C} and VDCC III for α_{1D}. Subunit subtypes, which are splice variants, are referred to, for example as α_{1H-1}, α_{1H-2}, α_{1B-1}, α_{1B-2}, α_{1C-1}, etc.

Thus, as used herein, nucleic acid (DNA or RNA) encoding the α₁ subunit refers to nucleic acid that hybridizes to the DNA provided herein under conditions of at least low stringency, typically high stringency, or encodes a subunit that has at least about 40% homology to protein encoded by DNA disclosed herein that encodes the specified α₁ subunit of a human calcium channel. In the case of LVA channels, nucleic acid that encodes a subunit that hybridizes under at least low stringency, preferably high stringency, to nucleic acid that encodes an α_{1H} subunit, and that encodes a subunit having the requisite LVA properties in assays for such activity, as those described herein. Splice variants will have varying percentages of overall homology (or identity), but will be derived from the same gene and will include regions of 100% identity.

In particular, a splice variant of any of the α₁ subunits (or any of the subunits particularly disclosed herein) will contain regions (at least one exon) of divergence and one or more regions (at least one exon, typically more than about 16 nucleotides, and generally substantially more) that have 100% homology with one or more of the α₁ subunit subtypes provided herein, and will also contain a region that has substantially less homology, since it is derived from a different exon. It is well within the skill of those in this art to identify exons and splice variants. Thus, for example, an α_{1H} subunit will be readily identifiable, because it will share at least about 40% protein homology with one of the α_{1H} subunits disclosed herein, and will include at least one region (one exon) that is 100% homologous. It will also have activity, as discussed below, that indicates that it is an LVA α₁ subunit.

It is noted herein, that identity and homology refer to the percentage of amino acids when proteins are compared or nucleotides when nucleic acids are compared that are shared. Numerous computer programs for determining identity are available. In all instances, intended gap penalties and other parameters are the defaults set by the manufacturer. Although not really needed when there is a high (90% or greater) degree of identity between sequences such programs include, but are not limited to commercially available sequence alignment programs, such as the DNAStar "MegAlign" program (Madison, WI) and the University of Wisconsin Genetics Computer Group (UWG) "Gap" program (Madison WI), to determine a percentage of sequence identity (see, also, von Heijne, entitled "Sequence Analysis in Molecular Biology: Treasure Trove of Trivial Pursuit" Academic Press (1987) Appendix 2 (citing to UWG and DNAStar among seven commercially available software programs)).

An α₁ subunit may be identified by its ability to form a calcium channel. Typically, α₁ subunits have molecular masses greater than at least about 120 kD. Also, hydropathy plots of deduced α₁ subunit amino acid sequences indicate that the α₁ subunits contain four internal repeats, each containing six transmembrane domains. An α_{1H}-subunit is identified by its pore-forming ability and also the low-voltage activation of the resulting channel.

The activity of a calcium channel may be assessed *in vitro* by methods known to those of skill in the art, including the electrophysiological and other methods described herein. Typically, α₁ subunits include regions with which one or more modulators of calcium channel activity, such as a 1 ,4-DHP or ω-CgTx, interact directly or indirectly. Types of α₁ subunits may be distinguished by any method known to those of skill in the art, including on the basis of binding specificity. For example, it has been found herein that α_{1B} subunits participate in the formation of channels that have previously been referred to as N-type channels, α_{1D} subunits participate in the formation of channels that had previously been referred to as L-type channels, α_{1A} subunits appear to participate in the formation of channels that exhibit characteristics typical of channels that had previously been designated P-type channels, and α_{1H} subunits appear to participate in channels that exhibit activities associated with T-type channels. Thus, for example, the activity of channels that contain the α_{1B} subunit are insensitive to 1,4-DHPs; whereas the activity of channels that contain the α_{1D} subunit are modulated or altered by a 1,4-DHP. It is presently preferable to refer to calcium channels based on pharmacological characteristics and current kinetics and to avoid historical designations. Types and subtypes of α₁ subunits may be characterized on the basis of the effects of such modulators on the subunit or a channel containing the subunit as well as differences in currents and current kinetics produced by calcium channels containing the subunit. The α_{1H} subunits may be further identified by the presence the notably long intracellular loop regions, such as between transmembrane domains I and II (e.g., nt 1506 to nt 2627 of SEQ ID No. 12), and also the loop in domain I.

In particular, nucleic acid that encodes an α_{1H} subunit as used herein, will hybridize under conditions of high stringency to the nucleic acid disclosed herein as SEQ ID Nos. 12, 15 and 16, and will form a channel in a mammalian cell, such as an HEK cell, that exhibits electrophysiological and/or pharmacological properties of a LVA or T-channel. The electrophysiological properties include one or more of the following electrophysiological properties a relative conductance of Ba²+ of about 5 pS (picoseconds) to about 9 pS, an activation time of about 2 to about 8 milliseconds, a kinetics of activation V_{1/2} value of about -60 millivolts to about 26 millivolts, an inactivation time of about 10 to about 30 milliseconds, a kinetics of inactivation V_{1/2}value of about -100 millivolts to about -500 millivolts, and a tail deactivation time of about 2 to about 12 milliseconds.

In addition, the resulting channel may have pharmacological properties, such as a relatively high degree of sensitivity to mibefradil, (IS,2S)-2-[2-[[3-(1H-benzimidazol-2-yl)propyl]methyl-amino]ethyl]-6-fluoro-1-isopropyl-1,2,3,4-tetrahydronaphthalen-2-yl methoxyacetate (Hoffman-LaRoche, Inc.) and/or a relatively high degree of resistance to the Conus snail toxins GVIA and MVIIC as well as the arachnid toxins AgaIIIA and AgalVA compared to HVA calcium channels.

As used herein, an α₂ subunit is encoded by nucleic acid (DNA or RNA) disclosed, for example, in U.S. Patent No. 5,407,820, U.S. Patent No. 5,792,846 and International PCT application No. WO95/04822 that encodes an α₂ subunit of a mammalian calcium channel or that hybridizes to DNA under conditions of low stringency, preferably high stringency, or encodes a protein that has at least about 40% homology, typically at least about 90% identity, taking into account gaps, with that disclosed therein. Such DNA encodes a protein that typically has a molecular mass greater than about 120 kD, but does not form a calcium channel in the absence of an α₁ subunit, and may alter the activity of a calcium channel that contains an α₁ subunit. Subtypes of the α₂ subunit that arise as splice variants are designated by lower case letter, such as α₂ₐ, ... α₂ₑ. In addition, the α₂ subunit and the large fragment produced when the protein is subjected to reducing conditions appear to be glycosylated with at least N-linked sugars and do not specifically bind to the 1,4-DHPs and phenylalkylamines that specifically bind to the α₁ subunit. The smaller fragment, the C-terminal fragment, is referred to as the δ subunit and includes amino acids from about 946 (as numbered in International PCT application No. WO95/04822, *e.g.*, SEQ ID No. 11 therein) through about the C-terminus. This fragment may dissociate from the remaining portion of α₂ when the α₂ subunit is exposed to reducing conditions. For purposes herein α₂ is also referred to as *α*₂*δ.* Thus, reference to *α*₂*δ* means the α₂ subunit, including the C-terminal *δ* portion.

As used herein, a β subunit is encoded by DNA disclosed, for example, in U.S. Patent No. 5,407,820, U.S. Patent No. 5,792,846 and International PCT application No. WO95/04822 or that hybridizes to the DNA provided therein under conditions of low stringency, preferably high stringency, or encodes a protein that has at least about 40% homology, typically about at least about 90% homology) with that disclosed therein and is a protein that typically has a molecular mass lower than the α subunits and on the order of about 50-80 kD, does not form a detectable calcium channel in the absence of an α₁ subunit, but may alter the activity of a calcium channel that contains an α₁ subunit or that contains an α₁ and α₂ subunit.

Types of the β subunit that are encoded by different genes are designated with subscripts, such as β₁, β₂, β₃ and β₄. Subtypes of β subunits that arise as splice variants of a particular type are designated with a numerical subscript referring to the type and to the variant. Such subtypes include, but are not limited to the β₁ splice variants, including β₁₋₁₋β₁₋₅ and β₂ variants, including β_{2C}-β_{2E}.

As used herein, a γ subunit is a subunit of calcium channel encoded by DNA disclosed for example in U.S. Patent Nos. 5,726,035 and 5,386,025; see, also Jay *et al.* (1990) Science 248:490-492 and Lett *et al.* (*1998) Nature Genetics 19:340-347) and may be isolated and identified using the nucleic disclosed therein as a probe by hybridization or other such method known to those of skill in the art, whereby full-length clones encoding a γ subunit may be isolated or constructed. A γ subunit will be encoded by nucleic acid that hybridizes to the DNA provided therein under conditions of low stringency, preferably high stringency, exhibits sufficient sequence homology to encode a protein that has at least about 40% homology with the γ subunit described herein.

Thus, one of skill in the art, in light of the disclosure herein, can identify DNA encoding α₁, *α*₂*, β, δ* and γ calcium channel subunits, including types encoded by different genes and subtypes that represent splice variants. For example, DNA or RNA probes based on the DNA disclosed herein may be used to screen an appropriate library, including a genomic or cDNA library, for hybridization to the probe and obtain DNA in one or more clones that includes an open reading fragment that encodes an entire protein. Subsequent to screening an appropriate library with the DNA disclosed herein, the isolated DNA can be examined for the presence of an open reading frame from which the sequence of the encoded protein may be deduced. Determination of the molecular weight and comparison with the sequences herein should reveal the identity of the subunit as an α₁, α₂ etc. subunit. Functional assays may, if necessary, be used to determine whether the subunit is an *α*₁*,* α₂ subunit or β subunit.

For example, DNA encoding an α_{1A} subunit may be isolated by screening an appropriate library with DNA, encoding all or a portion of the human α_{1A} subunit. Such DNA includes the DNA in the phage deposited under ATCC Accession No. 75293 that encodes a portion of an α₁ subunit. DNA encoding an α_{1A} subunit may be obtained from an appropriate library by screening with an oligonucleotide having all or a portion of the sequence of an α_{1A} subunit (see, *e.g.,* published International PCT application No. WO95/04822, particularly SEQ ID Nos. 21, 22 and/or 23 or with the DNA in the deposited phage therein). Alternatively, such DNA may have the coding sequence that encodes an α_{1A} subunit. Any method known to those of skill in the art for isolation and identification of DNA and preparation of full-length genomic or cDNA clones, including methods exemplified herein, may be used.

DNA encoding α_{1H} can be isolated by screening a human medullary thyroid carcinoma cell line (TT cells) or other suitable library human cDNA library with DNA probes prepared from nucleic acid provided herein. Full-length clones are constructed and expressed as described and exemplified herein and the resulting channels tested to verify that the encoding nucleic acid encodes a LVA channel.

The subunit encoded by isolated DNA may be identified by comparison with the DNA and amino acid sequences of the subunits provided herein. Splice variants share extensive regions of homology, but include non-homologous regions, subunits encoded by different genes share a uniform distribution of non-homologous sequences.

As used herein, a splice variant refers to a variant produced by differential processing of a primary transcript of genomic DNA that results in more than one type of mRNA. Splice variants may occur within a single tissue type or among tissues (tissue-specific variants). Thus, cDNA clones that encode calcium channel subunit subtypes that have regions of identical amino acids and regions of different amino acid sequences are referred to herein as "splice variants".

As used herein, a "calcium channel-selective ion" is an ion that is capable of flowing through, or being blocked from flowing through, a calcium channel which spans a cellular membrane under conditions which would substantially similarly permit or block the flow of Ca²⁺. Ba²⁺ is an example of an ion which is a calcium channel-selective ion.

As used herein, a compound that modulates calcium channel activity is one that affects the ability of the calcium channel to pass calcium channel-selective ions or affects other detectable calcium channel features, such as current kinetics. Such compounds include calcium channel antagonists and agonists and compounds that exert their effect on the activity of the calcium channel directly or indirectly.

As used herein, a "substantially pure" subunit or protein is a subunit or protein that is sufficiently free of other polypeptide contaminants to appear homogeneous by SDS-PAGE or to be unambiguously sequenced.

As used herein, selectively hybridize means that a DNA fragment hybridizes to a second fragment with sufficient specificity to permit the second fragment to be identified or isolated from among a plurality of fragments. In general, selective hybridization occurs at conditions of high stringency.

As used herein, heterologous or foreign DNA and RNA are used interchangeably and refer to DNA or RNA that does not occur naturally as part of the genome in which it is present or which is found in a location or locations in the genome that differ from that in which it occurs in nature. It is DNA or RNA that is not endogenous to the cell and has been artificially introduced into the cell. Examples of heterologous DNA include, but are not limited to, DNA that encodes a calcium channel subunit and DNA that encodes RNA or proteins that mediate or alter expression of endogenous DNA by affecting transcription, translation, or other regulatable biochemical processes. The cell that expresses the heterologous DNA, such as DNA encoding a calcium channel subunit, may contain DNA encoding the same or different calcium channel subunits. The heterologous DNA need not be expressed and may be introduced in a manner such that it is integrated into the host cell genome or is maintained episomafly.

As used herein, operative linkage of heterologous DNA to regulatory and effector sequences of nucleotides, such as promoters, enhancers, transcriptional and translational stop sites, and other signal sequences, refers to the functional relationship between such DNA and such sequences of nucleotides. For example, operative linkage of heterologous DNA to a promoter refers to the physical and functional relationship between the DNA and the promoter such that the transcription of such DNA is initiated from the promoter by an RNA polymerase that specifically recognizes, binds to and transcribes the DNA in reading frame.

As used herein, isolated, substantially pure DNA refers to DNA fragments purified according to standard techniques employed by those skilled in the art (see, *e.g.,* Maniatis *et al.* (1982) *Molecular Cloning: A Laboratory Manual,* Cold Spring Harbor Laboratory Press, Cold Spring Harbor, NY).

As used herein, expression refers to the process by which nucleic acid is transcribed into mRNA and translated into peptides, polypeptides, or proteins. If the nucleic acid is derived from genomic DNA, expression may, if an appropriate eukaryotic host cell or organism is selected, include splicing of the mRNA.

As used herein, vector or plasmid refers to discrete elements that are used to introduce heterologous DNA into cells for either expression of the heterologous DNA or for replication of the cloned heterologous DNA. Selection and use of such vectors and plasmids are well within the level of skill of the art.

As used herein, expression vector includes vectors capable of expressing DNA fragments that are in operative linkage with regulatory sequences, such as promoter regions, that are capable of effecting expression of such DNA fragments. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or may integrate into the host cell genome.

As used herein, a promoter region refers to the portion of DNA of a gene that controls transcription of the DNA to which it is operatively linked. The promoter region includes specific sequences of DNA that are sufficient for RNA polymerase recognition, binding and transcription initiation. This portion of the promoter region is referred to as the promoter. In addition, the promoter region includes sequences that modulate this recognition, binding and transcription initiation activity of the RNA polymerase. These sequences may be *cis* acting or may be responsive to *trans* acting factors. Promoters, depending upon the nature of the regulation, may be constitutive or regulated.

As used herein, a recombinant eukaryotic cell is a eukaryotic cell that contains heterologous DNA or RNA.

As used herein, a recombinant or heterologous calcium channel refers to a calcium channel that contains one or more subunits that are encoded by heterologous DNA that has been introduced into and expressed in a eukaryotic cell that expresses the recombinant calcium channel. A recombinant calcium channel may also include subunits that are produced by DNA endogenous to the cell. In certain embodiments, the recombinant or heterologous calcium channel may contain only subunits that are encoded by heterologous DNA.

As used herein, "functional" with respect to a recombinant or heterologous calcium channel means that the channel is able to provide for and regulate entry of calcium channel-selective ions, including, but not limited to, Ca²⁺ or Ba²⁺, in response to a stimulus and/or bind ligands with affinity for the channel. Preferably such calcium channel activity is distinguishable, such as by electrophysiological, pharmacological and other means known to those of skill in the art, from any endogenous calcium channel activity that is in the host cell.

As used herein, a T-type channel or LVA type channel typically refers to a calcium channel that exhibits a low-threshold calcium current that is activated and inactivated at low voltages compared to calcium channels (such as those that include an α_{1D} subunit) referred to as high voltage activated (HVA) channels. In addition or alternatively, a T-type channel may be characterized by distinct biophysical features, such as slow deactivation rates, very low conductances (5-9 pS) and voltage-dependent inactivation. T channels may exhibit a relatively high degree of sensitivity to mibefradil (Hoffman-LaRoche, Inc.) and/or a relatively high degree of resistance to the Conus snail toxins GVIA and MVIIC as well as the arachnid toxins AgaIIIA and AgaIVA compared to HVA calcium channels. These channels also typically exhibit reduced affinity for cadmium. T-type channels or LVA type channels may also be characterized at the nucleic acid level by the presence of one or more extended intracellular loops (see, e.g., SEQ ID NO. 12, 15 and 16) between transmembrane domains, such as between transmembrane domains I and II.

As used herein, a polypeptide having an amino acid sequence substantially as set forth in a particular SEQ ID No. includes protein that may have the same function but may include minor variations in sequence, such as conservative amino acid changes or minor deletions or insertions that do not alter the activity of the protein. The activity of a calcium channel receptor subunit protein, particularly a LVA or T-type channel, refers to its ability to form a functional calcium channel alone or with other subunits. A T-type channel will have the distinguishing properties defined herein.

As used herein, a physiological concentration of a compound is that which is necessary and sufficient for a biological process to occur. For example, a physiological concentration of a calcium channel-selective ion is a concentration of the calcium channel-selective ion necessary and sufficient to provide an inward current when the channels open.

As used herein, activity of a calcium channel refers to the movement of a calcium channel-selective ion through a calcium channel. Such activity may be measured by any method known to those of skill in the art, including, but not limited to, measurement of the amount of current which flows through the recombinant channel in response to a stimulus.

As used herein, a "functional assay" refers to an assay that identifies functional calcium channels. A functional assay, thus, is an assay to assess function.

As understood by those skilled in the art, assay methods for identifying compounds, such as antagonists and agonists, that modulate calcium channel activity, generally require comparison to a control. One type of a "control" cell or "control" culture is a cell or culture that is treated substantially the same as the cell or culture exposed to the test compound except that the control culture is not exposed to the test compound. Another type of a "control" cell or "control" culture may be a cell or a culture of cells which are identical to the transfected cells except the cells employed for the control culture do not express functional calcium channels. In this situation, the response of test cell to the test compound is compared to the response (or lack of response) of the calcium channel-negative cell to the test compound, when cells or cultures of each type of cell are exposed to substantially the same reaction conditions in the presence of the compound being assayed. For example, in methods that use patch clamp electrophysiological procedures, the same cell can be tested in the presence and absence of the test compound, by changing the external solution bathing the cell as known in the art.

It is also understood that each of the subunits disclosed herein may be modified by making conservative amino acid substitutions and the resulting modified subunits are contemplated herein. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (see, e.g., Watson et al. *Molecular Biology of the Gene,* 4th Edition, 1987, The Benjamin/Cummings Pub. Co., p.224). Such substitutions are preferably, although not exclusively, made in accordance with those set forth in TABLE 1 as follows:

**TABLE 1**

| Original residue | Conservative substitution |
|---|---|
| Ala (A) | Gly; Ser |
| Arg (R) | Lys |
| Asn (N) | Gln; His |
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile; Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions are also permissible and may be determined empirically or in accord with known conservative substitutions. Any such modification of the polypeptide may be effected by any means known to those of skill in this art. Mutation may be effected by any method known to those of skill in the art, including site-specific or site-directed mutagenesis of DNA encoding the protein and the use of DNA amplification methods using primers to introduce and amplify alterations in the DNA template.

As used herein, treatment means any manner in which the symptoms of a conditions, disorder or disease are ameliorated or otherwise beneficially altered. Treatment also encompasses any pharmaceutical use of the compositions herein, such as use as contraceptive agents.

As used herein, a LVA-activated calcium channel-mediated disorder refers to disorders that are associated with LVA channel activities. A T-type calcium channel-mediated disorders LVA-activated channel-mediated disorders that are associated with T-type channels. Such disorders include, but are not limited to: cardiovascular, hepatic, endocrine, urologic, reproductive, muscular, neurological and other disorders in which LVA channels, particular T-type channels, play a role either in mediating the disorder in some manner contributing to it.

As used herein, amelioration of the symptoms of a particular disorder by administration of a particular pharmaceutical composition refers to any lessening, whether permanent or temporary, lasting or transient that can be attributed to or associated with administration of the composition.

As used herein, substantially pure means sufficiently homogeneous to appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound.

As used herein, biological activity refers to the in vivo activities of a compound or physiological responses that result upon in vivo administration of a compound, composition or other mixture. Biological activity, thus, encompasses therapeutic effects and pharmaceutical activity of such compounds, compositions and mixtures.

### Identification and isolation of DNA encoding human calcium channel subunits

Methods for identifying and isolating nucleic acid (DNA and RNA) encoding α₁, α₂, β and γ, particularly nucleic acid encoding LVA α_{1H} subunits of human calcium channels are provided.

Identification and isolation of such nucleic acid may be accomplished by hybridizing, under appropriate conditions, at least low stringency, preferably high stringency,to restriction enzyme-digested human DNA with a labeled probe having at least 16 or more nucleotides (25, 30 or longer) and derived from any contiguous portion of DNA having a sequence of nucleotides set forth herein by sequence identification number. Once a hybridizing fragment is identified in the hybridization reaction, it can be cloned employing standard cloning techniques known to those of skill in the art. Full-length clones may be identified by the presence of a complete open reading frame and the identity of the encoded protein verified by sequence comparison with the subunits provided herein and by functional assays to assess calcium channel- forming ability or other function. This method can be used to identify genomic DNA encoding the subunit or cDNA encoding splice variants of human calcium channel subunits generated by alternative splicing of the primary transcript of genomic subunit DNA. For instance, DNA, cDNA or genomic DNA, encoding a calcium channel subunit may be identified by hybridization to a DNA probe and characterized by methods known to those of skill in the art, such as restriction mapping and DNA sequencing, and compared to the DNA provided herein in order to identify heterogeneity or divergence in the sequences of the DNA. Such sequence differences may indicate that the transcripts from which the cDNA was produced result from alternative splicing of a primary transcript, if the non-homologous and homologous regions are clustered, or from a different gene if the non-homologous regions are distributed throughout the cloned DNA. Splice variants share regions of 100% homology. As noted herein, the resulting nucleic acid may be expressed in cells and the resulting cells tested to verify or ascertain that expressed calcium channels exhibit pharmacological and/or electrophysiological properties of LVA or T-channels.

Any suitable method for isolating genes using the DNA provided herein may be used. For example, oligonucleotides corresponding to regions of sequence differences have been used to isolate, by hybridization, DNA encoding the full-length splice variant and can be used to isolate genomic clones. A probe, based on a nucleotide sequence disclosed herein, which encodes at least a portion of a subunit of a human calcium channel, such as a tissue-specific exon, may be used as a probe to clone related DNA, to clone a full-length cDNA clone or genomic clone encoding the human calcium channel subunit.

Labeled, including, but not limited to, radioactively or enzymatically labeled, RNA or single-stranded DNA of at least 16 contiguous bases, generally at least 30 contiguous bases of a nucleic acid which encodes at least a portion of a human calcium channel subunit, the sequence of which nucleic acid corresponds to a segment of a nucleic acid sequence disclosed herein by reference to a SEQ ID No. are disclosed. Such nucleic acid segments may be used as probes in the methods provided herein for cloning DNA encoding calcium channel subunits. See, generally, Sambrook *et al.* (1989) *Molecular Cloning: A Laboratory Manual,* 2nd Edition, Cold Spring Harbor Laboratory Press.

In addition, nucleic acid amplification techniques, which are well known in the art, can be used to locate splice variants of calcium channel subunits by employing oligonucleotides based on DNA sequences surrounding the divergent sequence primers for amplifying human RNA or genomic DNA. Size and sequence determinations of the amplification products can reveal splice variants. Furthermore, isolation of human genomic DNA sequences by hybridization can yield DNA containing multiple exons, separated by introns, that correspond to different splice variants of transcripts encoding human calcium channel subunits.

DNA encoding types and subtypes of each of the α₁, α₂, β and γ subunits of voltage-dependent human calcium channels has been cloned by nucleic acid amplification of cDNA from selected tissues or by screening human cDNA libraries prepared from isolated poly A + mRNA from cell lines or tissue of human origin having such calcium channels. Among the sources of such cells or tissue for obtaining mRNA are human brain tissue or a human cell line of neural origin, such as a neuroblastoma cell line, human skeletal muscle or smooth muscle cells, and the like. Methods of preparing cDNA libraries are well known in the art (see generally Ausubel *et al.* (1987) *Current Protocols in Molecular Biology,* Wiley-Interscience, New York; and Davis *et al.* (1986) *Basic Methods in Molecular Biology,* Elsevier Science Publishing Co., New York).

Preferred regions from which to construct probes include 5' and/or 3' coding sequences, sequences predicted to encode transmembrane domains, sequences predicted to encode cytoplasmic loops, signal sequences, ligand-binding sites, and other functionally significant sequences (see Table, below). Either the full-length subunit-encoding DNA or fragments thereof can be used as probes, preferably labeled with suitable label means for ready detection. When fragments are used as probes, preferably the DNA sequences will be typically from the carboxyl-end-encoding portion of the DNA, and most preferably will include predicted transmembrane domain-encoding portions based on hydropathy analysis of the deduced amino acid sequence (see, e.g., Kyte and Doolittle ((1982) *J. Mol. Biol.* 167:105).

Riboprobes that are specific for human calcium channel subunit types or subtypes have been prepared. These probes are useful for identifying expression of particular subunits in selected tissues and cells. The regions from which the probes were prepared were identified by comparing the DNA and amino acid sequences of all known α or β subunit subtypes. Regions of least homology, preferably human-derived sequences, and generally about 250 to about 600 nucleotides were selected. Numerous riboprobes for α and β subunits have been prepared (see, *e.g.*, Table 2 in International PCT application No. WO95/04822), which is repeated in part in the following Table.

**TABLE 2**

| **SUMMARY OF RNA PROBES** | | | | |
|---|---|---|---|---|
| SUBUNIT SPECIFICITY | NUCLEOTIDE POSITION | PROBE NAME | PROBE TYPE | ORIENTATION |
| α1A generic | 3357-3840 | pGEM7Zα1A* | riboprobe | n/a |
| | 761-790 | SE700 | oligo | antisense |
| | 3440-3464 | SE718 | oligo | antisense |
| | 3542-3565 | SE724 | oligo | sense |
| α1B generic | 3091-3463 | pGEM7Zα1B_{cyt} | riboprobe | n/a |
| | 6635-6858 | pGEM7Zα1B_{cooh} | riboprobe | n/a |
| α1B-1 specific | 6490-6676 | pCRII α1B-1/187 | riboprobe | n/a |
| α1E generic | 3114-3462 | pGEM7Zα1E | riboprobe | n/a |

| | | | | |
|---|---|---|---|---|
| * The pGEM series are available from Promega, Madison WI; see also, U.S. Patent No. 4,766,072. | | | | |

For the α_{1H}-specific probes (and also antibodies), regions unique to the α_{1H} subunits, such as the extended intracellular loops present in these channels may be used. For α_{1H-1} specific antibodies the region present in α_{1H-1} and absent from α_{1H-2} may be useful for preparation of subunit specific probes, purpose.

The DNA clones and fragments thereof provided herein thus can be used to isolate genomic clones encoding each subunit and to isolate any splice variants by hybridization screening of libraries prepared from different human tissues. Nucleic acid amplification techniques, which are well known in the art, can also be used to locate DNA encoding splice variants of human calcium channel subunits. This is accomplished by employing oligonucleotides based on DNA sequences surrounding divergent sequence(s) as primers for amplifying human RNA or genomic DNA. Size and sequence determinations of the amplification products can reveal the existence of splice variants. Furthermore, isolation of human genomic DNA sequences by hybridization can yield DNA containing multiple exons, separated by introns, that correspond to different splice variants of transcripts encoding human calcium channel subunits.

Once DNA encoding a calcium channel subunit is isolated, ribonuclease (RNase) protection assays can be employed to determine which tissues express mRNA encoding a particular calcium channel subunit or variant. These assays provide a sensitive means for detecting and quantitating an RNA species in a complex mixture of total cellular RNA. The subunit DNA is labeled and hybridized with cellular RNA. If complementary mRNA is present in the cellular RNA, a DNA-RNA hybrid results. The RNA sample is then treated with RNase, which degrades single-stranded RNA. Any RNA-DNA hybrids are protected from RNase degradation and can be visualized by gel electrophoresis and autoradiography. *In situ* hybridization techniques can also be used to determine which tissues express mRNA encoding a particular calcium channel subunit. The labeled subunit-encoding DNA clones are hybridized to different tissue slices to visualize subunit mRNA expression.

With respect to each of the respective subunits (α₁, α₂, β or γ) of human calcium channels, once the DNA encoding the channel subunit was identified by a nucleic acid screening method, the isolated clone was used for further screening to identify overlapping clones. Some of the cloned DNA fragments can and have been subcloned into an appropriate vector such as pIB124/25 (IBI, New Haven, CT), M13mp18/19, pGEM4, pGEM3, pGEM7Z, pSP72 and other such vectors known to those of skill in this art, and characterized by DNA sequencing and restriction enzyme mapping. A sequential series of overlapping clones may thus be generated for each of the subunits until a full-length clone can be prepared by methods, known to those of skill in the art, that include identification of translation initiation (start) and translation termination (stop) codons. For expression of the cloned DNA, the 5' noncoding region and other transcriptional and translational control regions of such a clone may be replaced with an efficient ribosome binding site and other regulatory regions as known in the art. Other modifications of the 5' end, known to those of skill in the art, that may be required to optimize translation and/or transcription efficiency may also be effected, if deemed necessary.

Examples 1-3 below, describe in detail the cloning DNA encoding α_{1H} splice variants and electrophyiological and pharmacological properties thereof. Except where noted, the methods of expression and other data is described with reference to the α_{1H-1} encoding nucleic acid. It is understood that the exemplified methods may be used to isolate additional splice variants and related subunits from humans and other mammals and animals and may also be used to express such nucleic acid to produce cells for use in screening assays to identify compounds that modulate the activity of LVA activated channels, particularly T-type channels. The nucleic acid may also be used in diagnostic assays to identify mutations and to produce proteins and then antibodies for use as reagents in diagnostic assays for disorders associated with T-type calcium channel activities.

### α₁ subunits of LVA channels

Nucleic acid encoding α₁ subunits that form LVA channels is provided herein. The nucleic acid provided herein may also be used to isolate related channels from other tissues, and other mammals and animals.

### Identification and isolation of DNA encoding the α_{1H} human calcium channel subunits

Calcium channels that contain α_{1H} should exhibit properties that differ from known HVA channels, formed from the α_{1A} - α_{1E} calcium channel subunits. Such differences may include low voltage activation, voltage-dependent inactivation, relatively high sensitivity to mibefradil and relatively high resistance to snail and arachnid toxins that inhibit most HVA channels (e.g., spider venom toxins ω-AgaIIIA and ω-AgaIVA and the Conus snail toxin GVIA). In addition α_{1H}-subunits may be identified by homology with other α₁-subunits and additionally by presence of an extended intracellular loop in the encoded subunit (see, e.g., SEQ No. 12. nucleotides 1506-2627) located between transmembrane domains I and II. This region in α_{1H} is extended compared to other calcium channel α₁ subunits, such as α_{1A}-α_{1E}.

DNA encoding an α_{1H}-subunit may be isolated using the DNA provided herein. In particular, probes of at least about 16 nucleotides or 30 nucleotides or other suitable length, such 30, 100 etc. bases, may be used to screen selected libraries, including mammalian DNA libraries. The selected libraries are preferably prepared from mammalian tissue or cell sources known to express T-type channels. The sequence of the probe is preferably based on the sequence of the intracellular loop located between transmembrane domains I and II (see, e.g., SEQ ID Nos. 12 and 15).

DNA encoding the α_{1H} subunit was isolated by amplifying a region of genes encoding an α₁ subunit expressed in a human thyroid carcinoma cell line (TT cells) using degenerate oligonucleotide primers.
The TT cell line is derived from a human medullary thyroid carcinoma and has been used to study calcitonin secretion and gene expression (debustros et al. (1986) J. Biol. Chem. 261:8036-8041; deBustros et al. 1990 Mol. Cell. Biol. 10:1773-1778). Whole-cell recordings from these cells reveal that the only voltage gated calcium channels expressed by these cells are low-voltage activated, rapidly inactivating and slowly deactivating, which are biophysical properties consistent with a T-type channel.

A portion of one of the positive clones was used to further screen a human thyroid carcinoma cDNA library to identify overlapping clones that span the entire length of the nucleotide sequence encoding the human α_{1H} subunit. A full-length α_{1H} DNA clone can be constructed by ligating portions of the partial cDNA clones as described in Example 1. SEQ ID No. 15 sets forth the nucleotide sequence of a clone encoding an α_{1H-1} subunit as well as the deduced amino acid sequence.

Two splice variants, α_{1H-1} and α_{1H-2}, were detected by RT-PCR (reverse transcriptase-amplification) using RNA from multiple tissues. The α_{1H-2} isoform (SEQ ID No. 16) contains a 957 nucleotide deletion, relative to α_{1H-1} (SEQ ID Nos. 12 and 15) in the I-II intracellular loop, i.e,. (e.g., nt 1506 to nt 2627 of SEQ ID No. 12).

The α_{1H-1} subunit exhibits marked sequence differences, as well as certain structural similarities to previously cloned α₁ subunits. Notably, the deduced amino acid sequence of α_{1H-1} shares less than 30% overall sequence identity with human α_{1A}-α_{1E}-encoding nucleic acids, which encode high-voltage activated calcium channels. Northern blot analysis indicates that mRNA transcripts for α_{1H} are expressed in the brain, primarily in the amygdala, caudate nucleus and putamen, and in peripheral tissues, primarily in the liver, kidney and heart.

Specifically, a comparison of the nucleic acid and deduced amino acid sequences of this α_{1H} calcium channel subunit with other human α₁ subunits reveals several distinct features. There are notable differences between α_{1H} and the HVA α₁ sequences. First, the intracellular loop between transmembrane Domains I and II is notably long. As exemplified in SEQ ID No. 12, the intracellular loop of human α_{1H} subunit is 1,122 nt in length whereas the corresponding intracellular loops in the other human α₁ subunits described herein range from 351 to 381 nt in length. Thus, the intracellular loop of human α_{1H} is nearly 250 amino acids longer than human α₁ subunits found in HVA calcium channels. The deduced amino acid sequence of this region (aa 420 to aa 794 of SEQ ID No. 12) contains a large number of proline residues and includes a poly-HIS region of 9 contiguous histidine residues (aa 52 to aa 528 of SEQ ID No. 12) and a region where 8 of 10 residues are alanine. The large intracellular loop located between transmembrane Domains I and II resembles the large intracellular loops found in a corresponding location in sodium channel α subunits some of which may function as homomers. It has been proposed that T-type channels have an activity that is a hybrid between HVA calcium channels and sodium channel. The α_{1H} subunits provided herein may also function as sodium channels.

Second, the isolated human α_{1H} subunit lacks amino acid residues that are generally known to be critical (e.g., see De Waard *et al.* (1996) FEBS Letters 380:272-276; Pragnell *et al.* (1994) Nature 368:67-70) for the interaction between α₁ subunits and the β subunits. There are at least thirteen residues located in this intracellular loop between transmembrane Domains I and II that form a motif that is highly conserved among α₁ subunits, such as α_{1A}-α_{1E} described herein (see, also Pragnell *et al.* (1994) Nature 368:67-70). In particular, this loop lacks the α₁ interaction domain (AID) involved in binding the β subunit. Also absent from this region is the G*βγ* binding motif, GlnXXGluArg, originally identified in adenylyl cyclase 2 and found in the non-L-type, HVA α₁ subunits. An identical sequence occurs, however, within the II-III intracellular loop of the α_{1H} sequence, suggesting a possible interaction of G*βγ* in this region. The α_{1H} subunit also contains differences in the determinants of ion selectivity found in the S5-S6 linkers of HVA channels. In the S5-S6 pore loops of domain III and IV, the glutamate residues that play a critical role in Ca²⁺ selectivity and ion permeation are replaced by aspartate residues.

Third, the human α_{1H} subunit has another notably long extracellular loop in Domain I located between IS5 and IS6. This extracellular loop ranges from 249 to 270 nucleotide residues in other human α₁ subunits whereas the human α_{1H} subunit has 426 nucleotide residues. Other distinguishing features may be ascertained and have been ascertained by expressing the subunit in cells as described herein.

The nucleic acid encoding an α_{1H} subunit can be used to screen appropriate libraries, particularly mammalian libraries, and more particularly mammalian libraries from tissues or cells that exhibit T-type channel activity. The encoded subunit can be identified by the above-noted distinguishing properties. Nucleic acid probes from the α_{1H-1-}encoding clone was used to identify and isolate clones encoding a second variant, designated α_{1H-2}, which has a 957 bp deletion relative to α_{1H-1}.

The α_{1H} subunit forms a functional channel in two different expression systems without the addition of exogenous *α*₂*δ* and β subunits. The absence of a β subunit interaction site within the I-II loop of the α_{1H} sequence is consistent with the report that β subunit depletion with antisense oligonucleotides in nodosus ganglia has no effect on T-type currents in that region. In addition, none of the known β subunits in HEK293 cells were detected by western analysis using β subunit-specific antisera, indicating that the previously cloned β subunits may not play a role in the formation of LVA Ca²⁺ channels containing α₁H. Oöcytes and HEK293 cells express an endogenous *α*₂*δ* subunit and that TT cells, the source of the α_{1H} subunits described here, express relatively high amounts of *α*₂*δ* protein. Consequently, it is possible that α_{1H}-containing channels expressed, contain α₂δ subunit, and that the α₂δ subunit is a component of native α_{1H}-containing channels.

### Distribution of α_{1H} transcripts

Northern blots containing human mRNA from several neuronal and nonneuronal tissues were probed with labeled fragments generated from the full-length α_{1H} cDNA. A single transcript of ~8.5 kb is present in all tissues examined, which included heart, brain, placenta, lung, liver, skeletal muscle, kidney, pancreas. Neuronal tissues included, cerebellum, cerebral cortex, medulla, spinal cord, occipital lope, frontal lobe, temporal lobe, putamen, amygdala, caudate nucleus, corpus callosum, hippocampus, substantia nigra, subthalamic nucleus and thalamus. In nonneuronal tissues, the highest expression levels are found in the kidney, liver, and heart. In the brain, the α_{1H} transcript is most abundant in the amygdala, caudate nucleus, and putamen.

### Identification and isolation of DNA encoding other α₁ human calcium channel subunit types and subtypes

DNA encoding additional α₁ subunits can be isolated and identified using the DNA provided herein as described for the α_{1A}, α_{1B}, α_{1C}, α_{1D}, α_{1E} and α_{1H} subunits or using other methods known to those of skill in the art. In particular, the DNA provided herein may be used to screen appropriate libraries to isolate related DNA. Full-length clones can be constructed using methods, such as those described herein, and the resulting subunits characterized by comparison of their sequences and electrophysiological and pharmacological properties with the subunits exemplified herein.

A number of voltage-dependent calcium channel α₁ subunit genes, which are expressed in the human CNS and in other tissues, have been identified and have been designated as α_{1A}, α_{1B} (or VDCC IV), α_{1C} (or VDCC II), α_{1D} (or VDCC III), α_{1E} and α_{1H}. DNA, isolated from a human DNA libraries that encodes each of the subunit types has been isolated. DNA encoding subtypes of each of the types, which arise as splice variants are also provided. Subtypes are herein designated, for example, as α_{1B-1}, α_{1B-2}. The α_{1H} subunit is of particular interest herein

The α₁ subunit types A, B, C, D, E and F of voltage-dependent calcium channels, and subtypes thereof, differ with respect to sensitivity to known classes of calcium channel agonists and antagonists, such as DHPs, phenylalkylamines, omega conotoxins (ω-CgTx), the funnel web spider toxin ω-Aga-IV, pyrazonoylguanidines and or in other physical and structural properties. These subunit types also appear to differ in the holding potential and in the kinetics of currents produced upon depolarization of cell membranes containing calcium channels that include different types of α₁ subunits.

DNA that encodes an α₁ subunit that binds to at least one compound selected from among dihydropyridines, phenylalkylamines, ω-CgTx, components of funnel web spider toxin, and pyrazonoylguanidines is provided. For example, the α_{1B} subunit provided herein appears to specifically interact with w-CgTx in N-type channels, and the α_{1D} subunit provided herein specifically interacts with DHPs in L-type channels.

### Antibodies

Antibodies, monoclonal or polyclonal, specific for calcium channel subunit subtypes or for calcium channel types can be prepared employing standard techniques, known to those of skill in the art, using the subunit proteins or portions thereof as antigens. Anti-peptide and anti-fusion protein antibodies can be used (see, for example, Bahouth et al. (1991) *Trends Pharmacol. Sci.* 12:338-343; *Current Protocols in Molecular Biology* (Ausubel et al., eds.) John Wiley and Sons, New York (1984)) Factors to consider in selecting portions of the calcium channel subunits for use as immunogens (as either a synthetic peptide or a recombinantly produced bacterial fusion protein) include antigenicity accessibility (i.e., extracellular and cytoplasmic domains), uniqueness to the particular subunit, and other factors known to those of skill in this art. Antibodies have therapeutic uses and also use in diagnostic assays.

The availability of subunit-specific antibodies makes possible the application of the technique of immunohistochemistry to monitor the distribution and expression density of various subunits (e.g., in normal vs diseased brain tissue). Such antibodies could also be employed in diagnostic, such as LES diagnosis, and therapeutic applications, such as using antibodies that modulate activities of calcium channels.

The antibodies can be administered to a subject employing standard methods, such as, for example, by intraperitoneal, intramuscular, intravenous, or subcutaneous injection, implant or transdermal modes of administration. One of skill in the art can empirically determine dosage forms, treatment regiments, and other parameters, depending on the mode of administration employed.

Subunit-specific monoclonal antibodies and polyclonal antisera have been prepared. The regions from which the antigens were derived were identified by comparing the DNA and amino acid sequences of all known α or β subunit subtypes. Regions of least homology, preferably human-derived sequences were selected. The selected regions or fusion proteins containing the selected regions are used as immunogens. Hydrophobicity analyses of residues in selected protein regions and fusion proteins are also performed; regions of high hydrophobicity are avoided. Also, and more importantly, when preparing fusion proteins in bacterial hosts, rare codons are avoided. In particular, inclusion of 3 or more successive rare codons in a selected host is avoided. Numerous antibodies, polyclonal and monoclonal, specific for α or β subunit types or subtypes have been prepared; some of these are listed in the following Table. Exemplary antibodies and peptide antigens that have been used to prepare the antibodies are set forth Table 3:

**TABLE 3**

| SPECIFICITY | AMINO ACID NUMBER | ANTIGEN NAME | ANTIBODY TYPE |
|---|---|---|---|
| α1 generic | 112-140 | peptide 1A#1 | polyclonal |
| α1 generic | 1420-1447 | peptide 1A#2 | polyclonal |
| α1A generic | 1048-1208 | α1A#2(b) GST fusion* | polyclonal |
| | | | monoclonal |
| α1B generic | 983-1106 | α1B#2(b) GST fusion | polyclonal |
| | | | monoclonal |
| α1B-1 | 2164-2339 | α1B-1#3 GST fusion | polyclonal |
| α1B-2 | 2164-2237 | α1B-2#4 GST fusion | polyclonal |
| α1E generic | 985-1004 (α1E-3) | α1E#2(a) GST fusion | polyclonal |

| | | | |
|---|---|---|---|
| * GST gene fusion system is available from Pharmacia; see also, Smith et al. (1988) Gene 67:31. The system provides pGEX plasmids that are designed for inducible, high-level expression of genes or gene fragments as fusions with Schistosoma japonicum GST. Upon expression in a bacterial host, the resulting fusion proteins are purified from bacterial lysates by affinity chromatography. | | | |

The GST fusion proteins are each specific for the cytoplasmic loop region IIS6-IIS1, which is a region of low subtype homology for all subtypes, including α_{1C} and α_{1D}, for which similar fusions and antisera can be prepared.

Using similar methods, antibodies specific for LVA subunits, particularly the α_{1H} subunits provided herein, using, for example, the extended intracellular loops, can be prepared. Such antibodies will have use in diagnostic assays for disorders in which LVA calcium channels are implicated.

### Preparation of recombinant eukaryotic cells containing DNA encoding heterologous calcium channel subunits

DNA encoding one or more of the calcium channel subunits or a portion of a calcium channel subunit may be introduced into a host cell for expression or replication of the DNA. Such DNA may be introduced using methods described in the following examples or using other procedures well known to those skilled in the art. Incorporation of cloned DNA into a suitable expression vector, transfection of eukaryotic cells with a plasmid vector or a combination of plasmid vectors, each encoding one or more distinct genes or with linear DNA, and selection of transfected cells are also well known in the art (see, *e.g.,* Sambrook *et al.* (1989) *Molecular Cloning: A Laboratory Manual, Second Edition,* Cold Spring Harbor Laboratory Press).

Cloned full-length nucleic acid encoding any of the subunits of a calcium channel may be introduced into a plasmid vector for expression in a eukaryotic cell. Such nucleic acid may be genomic DNA or cDNA or RNA. Presently preferred cells are those containing heterologous DNA encoding an α_{1H} subunit. Host cells may be transfected with one or a combination of the plasmids, each of which encodes at least one calcium channel subunit. Alternatively, host cells may be transfected with linear DNA using methods well known to those of skill in the art.

While the DNA provided herein may be expressed in any eukaryotic cell, including yeast cells such as *P. pastoris* (see, *e.g.,* Cregg *et al.* (1987) *Bio*/*Technology 5*:479), mammalian expression systems for expression of the DNA encoding the human calcium channel subunits provided herein are preferred.

The heterologous DNA may be introduced by any method known to those of skill in the art, such as transfection with a vector encoding the heterologous DNA. Particularly preferred vectors for transfection of mammalian cells are the pSV2dhfr expression vectors, which contain the SV40 early promoter, mouse dhfr gene, SV40 polyadenylation and splice sites and sequences necessary for maintaining the vector in bacteria, cytomegalovirus (CMV) promoter-based vectors such as pCDNA1, or pcDNA-amp and MMTV promoter-based vectors. The vector pcDNA1 is a eukaryotic expression vector containing a cytomegalovirus (CMV) promoter which is a constitutive promoter recognized by mammalian host cell RNA polymerase II.DNA encoding the human calcium channel subunits has been inserted in the vector pCDNA 1 at a position immediately following the CMV promoter. The vector pCDNA1 is presently preferred and has been used to express the α_{1H} subunits in mammalian cells.

Stably or transiently transfected mammalian cells may be prepared by methods known in the art by transfecting cells with an expression vector having a selectable marker gene such as the gene for thymidine kinase, dihydrofolate reductase, neomycin resistance or the like, and, for transient transfection, growing the transfected cells under conditions selective for cells expressing the marker gene. Functional voltage-dependent calcium channels have been produced in HEK 293 cells transfected with a derivative of the vector pCDNA1 that contains DNA encoding a human calcium channel subunit.

The heterologous DNA may be maintained in the cell as an episomal element or may be integrated into chromosomal DNA of the cell. The resulting recombinant cells may then be cultured or subcultured (or passaged, in the case of mammalian cells) from such a culture or a subculture thereof. Methods for transfection, injection and culturing recombinant cells are known to the skilled artisan. Eukaryotic cells in which DNA or RNA may be introduced, include any cells that are transfectable by such DNA or RNA or into which such DNA may be injected. Virtually any eukaryotic cell can serve as a vehicle for heterologous DNA. Preferred cells are those that can also express the DNA and RNA and most preferred cells are those that can form recombinant or heterologous calcium channels that include one or more subunits encoded by the heterologous DNA. Such cells may be identified empirically or selected from among those known to be readily transfected or injected. Preferred cells for introducing DNA include those that can be transiently or stably transfected and include, but are not limited to, cells of mammalian origin, such as COS cells, mouse L cells, CHO cells, human embryonic kidney cells, African green monkey cells and other such cells known to those of skill in the art, amphibian cells, such as *Xenopus laevis* oöcytes, or those of yeast such as *Saccharomyces cerevisiae* or *Pichia pastoris.* Preferred cells for expressing injected RNA transcripts or cDNA include *Xenopus laevis* oöcytes. Cells that are preferred for transfection of DNA are those that can be readily and efficiently transfected. Such cells are known to those of skill in the art or may be empirically identified. Preferred cells include DG44 cells and HEK 293 cells, particularly HEK 293 cells that can be frozen in liquid nitrogen and then thawed and regrown. Such HEK 293 cells are described, for example in U.S. Patent No. 5,024,939 to Gorman (see, also Stillman *et al.* (1985) *Mol. Cell.Biol. 5*:2051-2060).

The cells may be used as vehicles for replicating heterologous DNA introduced therein or for expressing the heterologous DNA introduced therein. In certain embodiments, the cells are used as vehicles for expressing the heterologous DNA as a means to produce substantially pure human calcium channel subunits or heterologous calcium channels. Host cells containing the heterologous DNA may be cultured under conditions whereby the calcium channels are expressed. The calcium channel subunits may be purified using protein purification methods known to those of skill in the art. For example, antibodies, such as those provided herein, that specifically bind to one or more of the subunits may be used for affinity purification of the subunit or calcium channels containing the subunits.

Substantially pure subunits of a human calcium channel α_{1H} subunits of a human calcium channel, α₂ subunits of a human calcium channel, β subunits of a human calcium channel and γ subunits of a human calcium channel are provided. Substantially pure isolated calcium channels that contain at least one of the human calcium channel subunits are also provided. Substantially pure calcium channels that contain a mixture of one or more subunits encoded by the host cell and one or more subunits encoded by heterologous DNA or RNA that has been introduced into the cell are also provided. Substantially pure subtype- or tissue-type specific calcium channels are also provided.

In one embodiment, eukaryotic cells that contain heterologous DNA encoding at least one of α_{1H} subunit of a calcium channel that express the α_{1H} subunit and form functional homomeric human α_{1H}-containing calcium channels are provided. These cells may be used to screen for compounds that modulate the activity of T-type channels and LVA type calcium channels.

In other embodiments, eukaryotic cells that contain heterologous DNA encoding at least one of an α_{1H}subunit of a human calcium channel, an α₂ subunit of a human calcium channel, a *β* subunit of a human calcium channel and a γ subunit of a human calcium channel are provided. In accordance with one preferred embodiment, the heterologous DNA is expressed in the eukaryotic cell and preferably encodes a human calcium channel α_{1H} subunit.

### Expression of heterologous calcium channels: electrophysiology and pharmacology

The α_{1H-1}subunit-encoding DNA was transiently expressed in HEK203 cells and associated with expression of an α_{1H-1} protein of approximately 260kDa α_{1H-1}, as identified by SDS-PAGE/Western blot analysis.

Ba²⁺ or Ca²⁺ currents recorded from HEK293 cells transiently expressing α_{1H-1} channels, and found to exhibit biophysical and pharmacological properties characteristic of low-voltage activated, i.e., T-type, calcium channel currents. Similar results were obtained in *Xenopus* oocytes expressing α_{1H-1}.

Electrophysiological methods for measuring calcium channel activity are known to those of skill in the art and are exemplified herein. Any such methods may be used in order to detect the formation of functional calcium channels and to characterize the kinetics and other characteristics of the resulting currents. Pharmacological studies may be combined with the electrophysiological measurements in order to further characterize the calcium channels.

With respect to measurement of the activity of functional heterologous calcium channels, preferably, endogenous ion channel activity and, if desired, heterologous channel activity of channels that do not contain the desired subunits, of a host cell can be inhibited to a significant extent by chemical, pharmacological and electrophysiological means, including the use of differential holding potential, to increase the S/N ratio of the measured heterologous calcium channel activity.

Thus, various combinations of subunits encoded by the DNA provided herein are introduced into eukaryotic cells. The resulting cells can be examined to ascertain whether functional channels are expressed and to determine the properties of the channels. In particularly preferred aspects, the eukaryotic cell which contains the heterologous DNA expresses it and forms a recombinant functional calcium channel activity. In more preferred aspects, the recombinant calcium channel activity is readily detectable because it is a type that is absent from the untransfected host cell or is of a magnitude and/or pharmacological properties or exhibits biophysical properties not exhibited in the untransfected cell.

The eukaryotic cells can be transfected with various combinations of the subunit subtypes provided herein. The resulting cells will provide a uniform population of calcium channels for study of calcium channel activity and for use in the drug screening assays provided herein.
Experiments that have been performed have demonstrated the inadequacy of prior classification schemes.

Preferred among transfected cells is a recombinant eukaryotic cell with a functional heterologous calcium channel. The recombinant cell can be produced by introduction of and expression of heterologous DNA or RNA transcripts encoding an α_{1H}subunit of a human calcium channel as a homomer, more preferably also expressing, a heterologous DNA encoding a β subunit of a human calcium channel and/or heterologous DNA encoding an α₂ subunit of a human calcium channel. Especially-preferred is the expression in such a recombinant cell of each of the α_{1H}, β and α₂ subunits encoded by such heterologous DNA or RNA transcripts, and optionally expression of heterologous DNA or an RNA transcript encoding a γ subunit of a human calcium channel. The functional calcium channels may preferably include at least an α_{1H}subunit and a β subunit of a human calcium channel. Eukaryotic cells expressing these two subunits and also cells expressing additional subunits, have been prepared by transfection of DNA and by injection of RNA transcripts. Such cells have exhibited voltage-dependent calcium channel activity attributable to calcium channels that contain one or more of the heterologous human calcium channel subunits. For example, eukaryotic cells expressing heterologous calcium channels containing an α₂ subunit in addition to the α₁ subunit and a β subunit have been shown to exhibit increased calcium selective ion flow across the cellular membrane in response to depolarization, indicating that the α₂ subunit may potentiate calcium channel function. Cells that have been co-transfected with increasing ratios of α₂ to α₁ and the activity of the resulting calcium channels has been measured. The results indicate that increasing the amount of α₂₋encoding DNA relative to the other transfected subunits increases calcium channel activity.

Eukaryotic cells that express heterologous calcium channels containing a human α_{1H} subunit as a homomer or at least a human α_{1H} subunit and optionally an α₂δ subunit and/or a human β subunit are preferred. Eukaryotic cells transformed with a composition containing DNA or an RNA transcript that encodes an α_{1H} subunit alone or in combination with a β and/or an α₂ subunit may be used to produce cells that express functional calcium channels. Since recombinant cells expressing human calcium channels containing all of the human subunits encoded by the heterologous DNA or RNA are especially preferred, it is desirable to inject or transfect such host cells with a sufficient concentration of the subunit-encoding nucleic acids to form calcium channels that contain the human subunits encoded by heterologous DNA or RNA. The precise amounts and ratios of DNA or RNA encoding the subunits may be empirically determined and optimized for a particular combination of subunits, cells and assay conditions.

In particular, mammalian cells have been transiently and stably tranfected with DNA encoding one or more human calcium channel subunits. Such cells express heterologous calcium channels that exhibit pharmacological and electrophysiological properties that can be ascribed to human calcium channels. Such cells, however, represent homogeneous populations and the pharmacological and electrophysiological data provides insights into human calcium channel activity heretofore unattainable. For example, HEK cells that have been transiently transfected with DNA encoding the α_{1E-1}, α_{2b}, and β₁₋₃ subunits. The resulting cells transiently express these subunits, which form calcium channels that have properties that appear to be a pharmacologically distinct class of voltage-activated calcium channels distinct from those of L-, N-, T- and P-type channels. The observed α_{1E} currents were insensitive to drugs and toxins previously used to define other classes of voltage-activated calcium channels.

HEK cells that have been transiently transfected with DNA encoding α_{1B-1}, α_{2b}, and β₁₋₂ express heterologous calcium channels that exhibit sensitivity to w-conotoxin and currents typical of N-type channels. It has been found that alteration of the molar ratios of α_{1B-1}, α_{2b} and β₁₋₂ introduced into the cells to achieve equivalent mRNA levels significantly increased the number of receptors per cell, the current density, and affected the K_{d} for ω-conotoxin.

The electrophysiological properties of these channels produced from α_{1B-1}, α_{2b}, and β₁₋₂ was compared with those of channels produced by transiently transfecting HEK cells with DNA encoding α_{1B-1}, α_{2b} and β₁₋₃. The channels exhibited similar voltage dependence of activation, substantially identical voltage dependence, similar kinetics of activation and tail currents that could be fit by a single exponential. The voltage dependence of the kinetics of inactivation was significantly different at all voltages examined.

In certain embodiments, the eukaryotic cell with a heterologous calcium channel is produced by introducing into the cell a first composition, which contains at least one RNA transcript that is translated in the cell into a subunit of a human calcium channel. In preferred embodiments, the subunits that are translated include an α_{1H} subunit of a human calcium channel. More preferably, the composition that is introduced contains an RNA transcript which encodes an α_{1H} subunit of a human calcium channel and also contains (1) an RNA transcript which encodes a β subunit of a human calcium channel and/or (2) an RNA transcript which encodes an α₂ subunit of a human calcium channel. Especially preferred is the introduction of RNA encoding an α_{1H} a β and an α₂ human calcium channel subunit, and, optionally, a γ subunit of a human calcium channel. Methods for *in vitro* transcription of a cloned DNA and injection of the resulting RNA into eukaryotic cells are well known in the art. Transcripts of any of the full-length DNA encoding any of the subunits of a human calcium channel may be injected alone or in combination with other transcripts into eukaryotic cells for expression in the cells. Amphibian oöcytes are particularly preferred for expression of *in vitro* transcripts of the human calcium channel subunit cDNA clones provided herein. Amphibian oocytes that express functional heterologous calcium channels have been produced by this method.

### Pharmacological and electrophysiological properties

As described in the examples, nucleic acid encoding α_{1H-1} and nucleic acid encoding α_{1H-2} has been expressed in mammalian cells and in amphibian oöcytes. Electrophyisological and pharmacological properties have been studied.

The biophysical properties of recombinant human α_{1H}²⁺ channels expressed in HEK293 cells and *Xenopus* oocytes are in good agreement, indicating that the biophysical properties of recombinant human α_{1H} channels are independent of the expression system. Several biophysical characteristics support the conclusion that the human α_{1H} subunit is the pore-forming α₁ subunit of a T-type channel. The rates of activation, inactivation, and deactivation and the single-channel conductance of α_{1H}-containing channels are within the ranges described for T-type channels. The conductance value of 9 pS measured in this study is near the value determined for rat α_{1G} -containing channels and is significantly lower than those determined for recombinant HVA channels. In addition, α_{1H}-containing channels conduct Ba2 + and Ca²⁺ equally well, consistent with the finding that the conductance of T-type channels for Ba2 + and Ca²⁺ is nearly equivalent in most cell types.

α_{1H}-Containing Ca²⁺ channels display a pharmacological profile differing from those of HVA channels. α_{1H}-mediated currents are inhibited by Ni²⁺, amiloride, and mibefradil (Ro 40-5967), agents shown to reduce LVA currents in a number of cell types. In contrast, ethosuximide, an antiepileptic agent that inhibits LVA currents in some cell types, had no effect on α_{1H}-mediated currents. Although the L-type Ca²⁺-channel modulators nimodipine and (-)-Bay K 8644 had little effect at a concentration of 1*µM* on α_{1H}-containing channels, both compounds produced a marked inhibition at a concentration of 10 *µM*, consistent with their effects on T-type channels in rat hypothalamic neurons (Akaike et al., 1989). In summary, the pharmacological properties of α_{1H}-containing channels described here have many similarities to native T-type channels studied in a variety of cell types. The pharmacological profiles of T-type channels vary considerably between cell types, and no hallmark pharmacological feature of T-type channels has been identified. These results are consistent with the finding herein that multiple α₁ subunits are responsible for the pharmacological profiles of a family of LVA, or T-type, channels.

### Assays and Clinical uses of the cells and calcium channels

### Assays

### Assays for identifying compounds that modulate calcium channel activity

Among the uses for eukaryotic cells which recombinantly express one or more subunits are assays for determining whether a test compound has calcium channel agonist or antagonist activity. These eukaryotic cells may also be used to select from among known calcium channel agonists and antagonists those exhibiting a particular calcium channel subtype specificity and to thereby select compounds that have potential as disease- or tissue-specific therapeutic agents.

*In vitro* methods for identifying compounds, such as calcium channel agonist and antagonists, that modulate the activity of calcium channels using eukaryotic cells that express heterologous human calcium channels are provided.

In particular, the assays use eukaryotic cells that express homomeric or heteromeric human calcium channel subunits encoded by heterologous DNA provided herein, for screening potential calcium channel agonists and antagonists which are specific for human calcium channels and particularly for screening for compounds that are specific for particular human calcium channel subtypes. Such assays may be used in conjunction with methods of rational drug design to select among agonists and antagonists, which differ slightly in structure, those particularly useful for modulating the activity of human calcium channels, and to design or select compounds that exhibit subtype- or tissue-specific calcium channel antagonist and agonist activities. These assays should accurately predict the relative therapeutic efficacy of a compound for the treatment of certain disorders in humans. In addition, since subtype-and tissue-specific calcium channel subunits are provided, cells with tissue- specific or subtype-specific recombinant calcium channels may be prepared and used in assays for identification of human calcium channel tissue- or subtype-specific drugs.

Desirably, the host cell for the expression of calcium channel subunits does not produce endogenous calcium channel subunits of the type or in an amount that substantially interferes with the detection of heterologous calcium channel subunits in ligand binding assays or detection of heterologous calcium channel function, such as generation of calcium current, in functional assays. Also, the host cells preferably should not produce endogenous calcium channels which detectably interact with compounds having, at physiological concentrations (generally nanomolar or picomolar concentrations), affinity for calcium channels that contain one or all of the human calcium channel subunits provided herein.

With respect to ligand binding assays for identifying a compound which has affinity for calcium channels, cells are employed which express, preferably, at least a heterologous α_{1H} subunit. Transfected eukaryotic cells which express at least an α_{1H} subunit may be used to determine the ability of a test compound to specifically bind to heterologous calcium channels by, for example, evaluating the ability of the test compound to inhibit the interaction of a labeled compound known to specifically interact with calcium channels. Such ligand binding assays may be performed on intact transfected cells or membranes prepared therefrom.

The capacity of a test compound to bind to or otherwise interact with membranes that contain α_{1H} subunit-containing heterologous calcium channels may be determined by using any appropriate method, such as competitive binding analysis, such as Scatchard plots, in which the binding capacity of such membranes is determined in the presence and absence of one or more concentrations of a compound having known affinity for the calcium channel. Where necessary, the results may be compared to a control experiment designed in accordance with methods known to those of skill in the art. For example, as a negative control, the results may be compared to those of assays of an identically treated membrane preparation from host cells which have not been transfected with one or more subunit-encoding nucleic acids.

The assays involve contacting the cell membrane of a recombinant eukaryotic cell which expresses at least an α_{1H} subunit of a human calcium channel, with a test compound and measuring the ability of the test compound to specifically bind to the membrane or alter or modulate the activity of a heterologous calcium channel on the membrane.

In preferred embodiments, the assay uses a recombinant cell that has a calcium channel containing an α_{1H} subunit of a human calcium channel. In other preferred embodiments, the assay uses a recombinant cell that has a calcium channel containing an α_{1H}subunit of a human calcium channel in combination with a β subunit of a human calcium channel and/or an α₂ subunit of a human calcium channel. Recombinant cells expressing heterologous calcium channels containing each of the α_{1H} and optionally a β and/or α₂ human subunits, and, optionally, a γ subunit of a human calcium channel are especially preferred for use in such assays.

In certain embodiments, the assays for identifying compounds that modulate calcium channel activity are practiced by measuring the calcium channel activity of a eukaryotic cell having a heterologous, functional calcium channel when such cell is exposed to a solution containing the test compound and a calcium channel-selective ion and comparing the measured calcium channel activity to the calcium channel activity of the same cell or a substantially identical control cell in a solution not containing the test compound. The cell is maintained in a solution having a concentration of calcium channel-selective ions sufficient to provide an inward current when the channels open. Recombinant cells expressing calcium channels that include each of the α_{1H}, β and α₂ human subunits, and, optionally, a γ subunit of a human calcium channel, are especially preferred for use in such assays. Methods for practicing such assays are known to those of skill in the art. For example, for similar methods applied with *Xenopus laevis* oöcytes and acetylcholine receptors, see, Mishina *et al.* ((1985) *Nature 313*:364) and, with such oöcytes and sodium channels (see, Noda *et al.* (1986) *Nature 322:*826-828). For similar studies which have been carried out with the acetylcholine receptor, see, *e.g.,* Claudio *et al.* ((1987) *Science 238*:1688-1694). Transcription based assays are also contemplated herein.

Functional recombinant or heterologous calcium channels may be identified by any method known to those of skill in the art. For example, electrophysiological procedures for measuring the current across an ion-selective membrane of a cell, which are well known, may be used. The amount and duration of the flow of calcium-selective ions through heterologous calcium channels of a recombinant cell containing DNA encoding one or more of the subunits provided herein has been measured using electrophysiological recordings using a two electrode and the whole-cell patch clamp techniques. In order to improve the sensitivity of the assays, known methods can be used to eliminate or reduce non-calcium currents and calcium currents resulting from endogenous calcium channels, when measuring calcium currents through recombinant channels. For example, the DHP Bay K 8644 specifically enhances L-type calcium channel function by increasing the duration of the open state of the channels (see, *e.g.,* Hess, J.B., *et al.* (1984) *Nature 311*:538-544). Prolonged opening of the channels results in calcium currents of increased magnitude and duration. Tail currents can be observed upon repolarization of the cell membrane after activation of ion channels by a depolarizing voltage command. The opened channels require a finite time to close or "deactivate" upon repolarization, and the current that flows through the channels during this period is referred to as a tail current. Because Bay K 8644 prolongs opening events in calcium channels, it tends to prolong these tail currents and make them more pronounced.

In practicing these assays, stably or transiently transfected cells or injected cells that express voltage-dependent human calcium channels containing one or more of the subunits of a human calcium channel desirably may be used in assays to identify agents, such as calcium channel agonists and antagonists, that modulate calcium channel activity. Functionally testing the activity of test compounds, including compounds having unknown activity, for calcium channel agonist or antagonist activity to determine if the test compound potentiates, inhibits or otherwise alters the flow of calcium ions or other ions through a human calcium channel can be accomplished by (a) maintaining a eukaryotic cell which is transfected or injected to express a heterologous functional calcium channel capable of regulating the flow of calcium channel-selective ions into the cell in a medium containing calcium channel-selective ions (i) in the presence of and (ii) in the absence of a test compound; (b) maintaining the cell under conditions such that the heterologous calcium channels are substantially closed and endogenous calcium channels of the cell are substantially inhibited (c) depolarizing the membrane of the cell maintained in step (b) to an extent and for an amount of time sufficient to cause (preferably, substantially only) the heterologous calcium channels to become permeable to the calcium channel-selective ions; and (d) comparing the amount and duration of current flow into the cell in the presence of the test compound to that of the current flow into the cell, or a substantially similar cell, in the absence of the test compound.

The assays thus use cells, provided herein, that express heterologous functional calcium channels and measure functionally, such as electrophysiologically, the ability of a test compound to potentiate, antagonize or otherwise modulate the magnitude and duration of the flow of calcium channel-selective ions, such as Ca²⁺ or Ba²⁺, through the heterologous functional channel. The amount of current which flows through the recombinant calcium channels of a cell may be determined directly, such as electrophysiologically, or by monitoring an independent reaction which occurs intracellularly and which is directly influenced in a calcium (or other) ion dependent manner. Any method for assessing the activity of a calcium channel may be used in conjunction with the cells and assays provided herein. For example, in one embodiment of the method for testing a compound for its ability to modulate calcium channel activity, the amount of current is measured by its modulation of a reaction which is sensitive to calcium channel-selective ions and uses a eukaryotic cell which expresses a heterologous calcium channel and also contains a transcriptional control element operatively linked for expression to a structural gene that encodes an indicator protein. The transcriptional control element used for transcription of the indicator gene is responsive in the cell to a calcium channel-selective ion, such as Ca²⁺ and Ba²⁺. The details of such transcriptional based assays are described in commonly owned PCT International Patent Application No. PCT/US91/5625, filed August 7, 1991, which claims priority to copending commonly owned allowed U.S. Application Serial No. 07/563,751, filed August 7, 1990; see also, commonly owned published PCT International Patent Application PCT US92/11090, which corresponds to co-pending U.S. Applications Serial Nos. 08/229,150 and 08/244,985. The contents of these applications are herein incorporated by reference thereto.

### Biophysical and pharmacological properties of α_{1H} subunits

HEK cells were transfected with DNA and oöcytes injected wiht nucleic acid provided herein. The cell expressed calcium channels, which were then characterized electrophysiologically and pharmacologically. These results are described in the examples. Both splice variants formed calcium channels that exhibit properties associated with T-type channels. Variant-specific properties were observed.

These observed differences in the amino acid sequences of α_{1H-1} and α_{1H-2} will result in marked differences in susceptibility of these receptors to cellular regulation, particularly since the observed region of sequence divergence resides in the cytosolic linker region between domains I and II and the analogous sequence region in high-voltage activated calcium channels has been implicated in binding of cytosolic regulatory proteins. Observed differences in biophysical properties of α_{1H-1} and α_{1H-2} are also likely indicative of differences in the sensitivity of these two different channel subunits to pharmaceutical compounds. Thus, it seems likely that low-voltage activated calcium channels containing either the α_{1H-1} or the α_{1H-2} subunit will be subject to different regulatory controls, and different profiles of susceptibility to pharmaceutical compounds. For example, amiloride blocks the T-type current in neuroblastoma cells with an IC₅₀ of ~50 µM, whereas in hippocampal neurons 300 µM amiloride reduces the T-type current by only 40%.

In this respect, each a different α_{1H} channel is a separate screening target for development of pharmaceutical drug compounds. Differential effects of drugs on different neural cells and in different neural tissues can be understood based on different patterns of expression of α_{1H-1} and/or α_{1H-2} *in vivo* and will provide a means to identify drugs specific for each subtype and associated disorders or conditions. The observed sequence variation in α_{1H} subunits explains observed pharmacological variability of T-type calcium channels in different native tissues, providing a useful tool to identify where the respective α_{1H-1} and α_{1H-2} subunit is expressed to use screening assays to identify targeted therapeutic drug candidates.

Differences in α_{1H-1} and α_{1H-2} functionality and expression in different tissues provides basis for using recombinant cells expressing calcium channels having either the α_{1H-1} or α_{1H-2} subunit. Agonists and antagonists capable of differentially affecting calcium channels containing these two different subunits should be useful for targeting therapeutic intervention into selected neural locations, e.g., to cardiovascular neurons an cardiac pacemaker neurons expressing α_{1H-2}. Calcium channels formed from α_{1H} subunits open at small changes in membrane potential, but only allow moderate Ca²⁺ influx before closing. By allowing moderate influx of divalent ions the α_{1H} containing channels are likely to:
(i) participate in pathways triggering changes in gene expression in response to subtle change sin membrane potential difference, i.e., in neuronal and non-neuronal cell types (e.g., in activation of immune cells such as T-cells, in activation of kidney and liver cells in response to metabolic changes;
(ii) exert subtle controls over the overall excitability or accessibility of neurons to synaptic transmission, such as in determining which neurons will respond to stimulae, and to what extent, such as in peripheral neurons and ganglia;
(iii) determine the extent of neural responses to stimulae such as chronic pain;
(iv) regulate the sensitivity of neurons in critical neural centers so that neuronal cells in these centers are protected from the adverse effects associated with excessive bursts of firing (e.g., in the cardiac pacemaker);
(v) act to set the steady state pattern of inactivation of neurons in different regions of the brain, (e.g., in response to sleep, sex, emotion, depression, fatigue and the other stimulae or conditions).

### Electrophysiology of cells that express channels containing the α_{1H-1} subunit

### Expression of recombinant α_{1H-1} channels

Following transient transfection of HEK293 cells with a DNA encoding the α_{1H} subunit, Ba²⁺ currents that were rapidly activating and inactivating were observed. Ba²⁺ currents (15 m*M*) elicited by step depolarizations to various test potentials from a holding potential of -90-mV were measured. Currents were activated at a test potential of -50 mV, peaked between -20 and -10 mV, and reversed at a membrane potential more positive that + 60 mV. Similar results were obtained with Ca²⁺ (15 m*M*) as the charge carrier.

One hallmark of LVA channels is their slow rate of deactivation, which is reflected in a show decay of tail currents. The time constant of this decay is ~10-fold slower for LVA channels (2-12 ms) than for HVA channels < 300 µs. A slow decay of α_{1H-1} mediated tail currents over a period of ~15 ms was observed. In contrast to the monoexponential decay of the tail currents reported for many native T-type Ca²⁺ channels, tail currents from α_{1H-1} channels showed a biexponential decay. At a test potential of -20 mV, the decay rate of the slow component, comprising 88.1 ± 33.8% of the total current, was 2.1 ± 1.06 ms (n = 6), which is similar to those observed in native T-type Ca²⁺ channels. The decay rate of the faster component was 0.64 ± 0.21 ms (n = 6).

Whole-cell patch clamp recordings were performed on HEK293 cells transiently expressing the human α_{1H-1} subunit. Step-depolarizations elicited inward Ba²⁺ currents that activate slowly and inactivate rapidly (2.8 ± 0.6 and 16.9 ± 5.3 ms, at -20 mV). The activation curve of α_{1H-1} is shifted to the left (V1/2:-29.5 mV) compared to HVA ca²⁺ channels. The tails currents of α_{1H-1}-containing channels decay slowly (τ1, τ2 ± 1.0, 0.6, ± 0.2 ms). The permeability for Ba²⁺ and Ca²⁺ was virtually identical. The single channel conductance, determined with 110 mM ba²⁺ as charge carrier, is 9pS.

The voltage dependence of activation of α_{1H-1} containing Ca²⁺ channels was determined from tail-current analysis. Normalized tail-current amplitudes were plotted as a function of test potential and revealed a biphasic activation curve that was well fitted by the sum of two Boltzmann functions (Figure 1). The potentials for half-maximal activation of the individual Boltzmann terms were as follows: V_{½,A}: -25.1 ± 3 3.0 mV; and V_{½,B}: + 25.5 ± 3 9.9 mV (n = 11). A value similar to V_{½,A} has been reported previously for voltage dependence of activation of T-type CA²⁺ channels in the human TT cell line (-27 mV). The value of the second Boltzmann term V_{½,B} is somewhat similar to that reported for HVA Ca²⁺ channels. Using a similar protocol, tail currents of HVA Ca²⁺ channels decay with time constants of < 300 *µs*, whereas with α_{1H} the most prominent at test potentials close to V_{½,B}. The availability of α_{1H} containing Ca²⁺ channels for opening was dependent on the membrane for potential as shown in Fig. 1. The potential for half-maximal steady-state inactivation (V_{½}) was - 63.2 ± 2.0 mV (n = 9).

The rapid inactivation of α_{1H} Ca²⁺ channels was strongly voltage-dependent. The current decay was best described with an exponential function with time constants ranging from 42.2 ± 7.8 to 8.8 ± 3.8 ms at membrane potentials between -50 and + 30 mV (n = 6; data not show). Activation kinetics of α_{1H} Ca²⁺ channels were also voltage-dependent with time constants ranging from 9.9 ± 4.7 to 0.9 ± 0.3 ms for membrane potentials between -50 and + 30 mV (n = 8; data not shown). α_{1H} Ca²⁺ channels inactivated completely during the 150-ms depolarization. Recovery from inactivation occurred within a period of ~ 3 s with a fast component (τ = 37 ± 9 ms; 16.5 ± 4.6% of all channels) and a slow component (τ = 37 ± 61 ms; 78 ± 8.5% of all channels; n = 3; data not shown). To confirm the biophysical properties of recombinant α_{1H} channels observed in whole-cell recordings from HEK293 cells, the functional expression of α_{1H} in *Xenopus* oöcytes was tested. Substantial currents (< 1 µA) after injection of α_{1H} transcripts alone was observed. The activation and inactivation kinetics, as well as the steady-state inactivation properties, were similar to those obtained in HEK293 cells (see EXAMPLES).

Single-channel properties of α_{1H}Ca²⁺ channels in HEK293 cells were determined in cell-attached recordings with 110 m*M* Ba²⁺ as the charge carrier. Single-channel recordings at a test potential of -30 mV from a patch that contains at least three α_{1H} showed that channel openings occurred in bursts and were clustered mainly in the first third of the 100-ms depolarizing pulse, especially with stronger depolarizations. Occasionally, channel activity was spread throughout the entire sweep. The time course of the ensemble-averaged current recorded at -30mV in 110 mM Ba²⁺ was similar to the α_{1H} whole-cell Ba²⁺ current recorded at -40 mV in 15 mM Ba²⁺. The currents were compared at different potentials to compensate for the shift in the activation curve to more positive potentials due to the increase in divalent concentration. The unitary current-voltage relationship yielded a unitary slope conductance of 9.06 ± 0.22 pS (n = 4).

### Summary of Electrophysiologic Characteristics

The biophysical properties of calcium channels containing the human α_{1H} subunit were evaluated. Whole cell recordings from transiently transfected HEK293 cells indicate that the current-voltage relationship, permeability to Ca²⁺ and Ba²⁺, kinetics of activation, and single channel conductance of calcium channels containing α_{1H} subunits were similar to those of native T-type calcium channels in tissues. Tail currents from A_{1H} channels showed a bi-exponential decay, exhibiting a fast and a slower component. At very negative membrane potentials (-150 to -100 mV) the fast component (r: 200-450 *µ*s) dominated the inactivation process, while at depolarizing potentials > -50 mV the slower component (2-3 ms) dominated. At the resting membrane potential, i.e., ≤-80 mV, both components contribute equally.

### Pharmacological properties

The pharmacological properties of α_{1H}-containing calcium channels were also consistent with those observed for native T-type calcium channels. Interestingly, the sensitivity of α_{1H-1}-containing calcium channels to Cd²⁺ or Amiloride was about 10-fold lower when expressed in HEK293 cells than when expressed in *Xenopus* oöcytes.

The data indicate that human α_{1H} calcium channel subunits have properties consistent with that of native T-type calcium channels and, as such, α_{1H} represent a member in the rapidly growing family of low-voltage activated calcium channels.

### Assays for diagnosis of LVA-calcium channel mediated disorders and clinical applications

### Clinical applications

In relation to therapeutic treatment of various disease states, the availability of DNA encoding human calcium channel subunits permits identification of any alterations in such genes (e.g., mutations) which may correlate with the occurrence of certain disease states. In addition, the creation of animal models of such disease states becomes possible, by specifically introducing such mutations into synthetic DNA fragments that can then be introduced into laboratory animals or *in vitro* assay systems to determine the effects thereof.

Also, genetic screening can be carried out using the nucleotide sequences as probes. Thus, nucleic acid samples from subjects having pathological conditions suspected of involving alteration/modification of any one or more of the calcium channel subunits can be screened with appropriate probes to determine if any abnormalities exist with respect to any of the endogenous calcium channels. Similarly, subjects having a family history of disease states related to calcium channel dysfunction can be screened to determine if they are also predisposed to such disease states.

Disorders and for which screening assays can be developed and also for which candidate compounds for treatment of the disorders include, but are not limited to: cardiac treatments, such as myocardial infarct, cardiac arrhythmia, heart failure, and angina pectoris. Identified compounds will be useful in: (a) adjunctive therapies for reestablishing normal heart rate and cardiac output following traumatic injury, heart attack and other heart injuries; (b) treatments of myocardial infarct (MI), post-MI and in an acute setting. The compounds may be effective to increase cardiac contractile force, such as that measured by left ventricular enddiastolic pressure, and without changing blood pressure or heart rate. In an acute setting the compounds may be effective to decrease formation of scar tissue, such as that measured by collagen deposition or septal thickness, and without cardiodepressant effects. The identified compounds will be useful for and assays for diagnosis and compound screening will be useful in connection with vascular treatments and hypertension, for identifying compounds useful in regulating vascular smooth muscle tone, including vasodilating or vasoconstricting. Such compounds can be used in (a) treatments for reestablishing blood pressure control, e.g., following traumatic injury, surgery or cardiopulmonary bypass, and in prophylactic treatments designed to minimizing cardiovascular effects of anaesthetic drugs; (b) treatments for improving vascular reflexes and blood pressure control by the autonomic nervous system. Other conditions include urologic, for identifying compounds useful in: (a) treating and restoring renal function following surgery, traumatic injury, uremia and adverse drug reactions; (b) treating bladder dysfunctions; and (c) uremic neuronal toxicity and hypotension in patients on hemodialysis; reproductive conditions, for identifying compounds useful in treating: (a) disorders of sexual function including impotence; and (b) alcoholic impotence (under autonomic control that may be subject to T-channel controls); hepatic, for identifying compounds useful in treating and reducing neuronal toxicity and autonomic nervous system damage resulting from acute over-consumption of alcohol; neurological conditions for identifying compounds useful in treating: (a) epilepsy and diencephalic epilepsy;
(b) Parkinson disease; (c) aberrant temperature control, such as abnormalities of shivering and sweat gland secretion and peripheral vascular blood supply;
(d) aberrant pituitary and hypothalamic functions including abnormal secretion of noradrenaline, dopamine and other hormones; respiratory conditions, for identifying compounds useful in treating abnormal respiration, such as, post-surgical complications of anesthetics; endocrine disorders for identifying compounds useful in treating aberrant secretion of hormones such as treatments for overproduction of hormones including insulin, thyroxin, and adrenalin.

### EXAMPLES

The following examples are included for illustrative purposes only and are not intended to limit the scope of the invention.

### EXAMPLE 1: ISOLATION OF DNA ENCODING THE HUMAN CALCIUM CHANNEL α_{1H-1} SUBUNIT

Using mRNA and TT cells, a degenerate PCR approach was used to isolate nucleic acid encoding an α₁ subunit. Nucleic acid encoding an α_{1H-1} subunit and nucleic acid encoding a subunit designated as α_{1H-2} was isolated. The nucleic acid was introduced into HEK293 cells and *Xenopus* oöcytes and voltage gated calcium channels were expressed. These channels exhibit pharmacological and electrophyiological properties consistent with native LVA, T-type, channels.

### A. Materials and Methods

### Nucleic acid amplification:

The following sense strand 20-mer PCR primer, corresponding to nucleotides 1945-1964 of DNA encoding a human α_{1E} subunit, was synthesized:
AC(A/C/G/T)GTGTT(C/T)CAGATCCTGAC (Primer-1) SEQ ID NO. 4
   An antisense 22-nucleotide PCR primer, corresponding to nucleotides 3919 through 3940 of human α_{1E}, was also synthesized:
T(C/T)CCCTTGAAGAGCTG(A/C/G/T)ACCCC (Primer-2) SEQ ID NO. 1
   The sense and the antisense primers were used in amplification reactions with cDNA prepared from TT cells and Pfu DNA polymerase (Stratagene Inc., San Diego, CA).

**Reaction conditions:** 95°C for 5 minutes followed by 5 cycles of 20 seconds each at 95°C; then 20 seconds at 42 °C; 2.5 minutes at 72°C; and, 30 cycles of 20 seconds each at 95°C followed by 20 seconds at 50°C and finally 2.5 minutes at 72°C. The product of the reaction is referred to herein (below) as "the original PCR products."

A second 5' degenerate oligonucleotide primer was designed corresponding to a portion of the sequence reported for C. *elegans,* cosmid C54D2 (Genebank accession #U37548), as a portion of that sense strand sequence which aligns with a portion of the human α_{1E} subunit DNA sequence between nucleotide 3598 and 3614. This primer had the following sequence:
GA(A/G)ATGATGATGAA(A/G)GT (Primer-3) SEQ ID NO. 10
Primer-3 was used in a nested amplification reaction with the original PCR products and the Primer-2.

**Isolation and Characterization of the clones:** A recombinant cDNA library was constructed in phage vector λgt 10 using poly(A)⁺-selected RNA from the TT cell line. Approximately 1.5x10⁶ were screened with the PCR fragment under high stringency (hybridization: 50% formamide, 5X SSPE, 5X Denhardts, 0.2% SDS, 200µg/ml herring sperm DNA for 16-18 hrs. at 42°C; wash: 6 washes of 30 minutes each in 0.1 X SSPE, 0.1% SDS at 65°C).

**Northern blot analysis:** Multiple tissues were screened in Northern blots using 2µg of poly(A)⁺ RNA per lane (Clontech, Palo Alto, CA). Blots were probed at high stringency, as described above, with labeled fragments generated from the full-length α_{1H} cDNA, i.e., nucleotide -6 to 7390.

**Western blot analysis:** Cellular membranes (total) were isolated from HEK293 cells expressing different α_{1H} subunits; membrane proteins were separated by SDS-PAGE; transferred to nitrocellulose; and, blotted using a polyclonal anti-α_{1H} antisera and TBS-T buffer. Blotted proteins were visualized using the Lumiglo reagent kit (KPL, Gaithersburg, MD) according to the manufacturer's instructions.

### B. RNA isolation

Human medullary thyroid carcinoma cells (TT cells; ATCC Accession No. CRL1803) were grown in DMEM medium supplemented with 10 % fetal calf serum at 37 °C in 5% CO₂ atmosphere and total cytoplasmic RNA was isolated from forty 10 cm plates using a "midi-prep" RNA isolation kit (Qiagen) as per the manufacturer's instructions. The protocol entails the use of the detergent NP40 which lyses the cell membrane under mild conditions such that the nuclear membrane remains intact thereby eliminating incompletely spliced RNA transcripts from the preparation.

PolyA + RNA was isolated from total cytoplasmic RNA using two passes over an oligo(dT)-cellulose column. Briefly, 2-3 mg of total cytoplasmic RNA was resuspended in NETS buffer (500 mM NaCl 10 mM EDTA, 10 mM Tris, pH 7.4, 0.2% SDS) and passed slowly over a column containing 0.5 g of oligo(dT)-cellulose (Collaborative Research) equilibrated in NETS buffer. The column was washed with 30 mls of NETS buffer and polyA + RNA was eluted using about 3 mls of ETS buffer (10 mM EDTA, 10 mM Tris, pH 7.4, 0.2% SDS). The ionic strength of the polyA + RNA-containing buffer was adjusted to 500 mM NaCl and passed over a second oligo(dT)-cellulose column essentially as described above. Following elution from the second column, the polyA + RNA was precipitated twice in ethanol and resuspended in H₂O.

### C. Library construction

Double stranded cDNA (dscDNA) was synthesized according to standard methods (see, *e.g.*, Gubler *et al.* (1985) Gene 25:263-269; Lapeyre *et al.* (1985) Gene 37:215-220). Briefly, first strand cDNA synthesis was initiated using TT cell polyA + RNA as a template and using random primers and Moloney Murine Leukemia Virus reverse transcriptase (MMLV-RT). The second strand was synthesized using a combination of E. coli DNA polymerase, E. coli DNA ligase and RNase H.

Regions of single stranded DNA were converted to double-stranded DNA using T4 DNA polymerase generating blunt-ended double stranded fragments. EcoRI restriction endonuclease site adapters:
5' CGTGCACGTCACGCTAG 3' (SEQ ID NO. 2)
3' GCACGTGCAGTGCGATCTTAA 5' (SEQ ID NO. 3)
were ligated to the double-stranded cDNA using a standard protocol (see, *e.g.*, Sambrook *et al.* (1989) IN: *Molecular Cloning, A Laboratory Manual,* Cold Spring Harbor Laboratory Press, Chapter 8). The double-stranded DNA with the *Eco*RI adapters ligated was purified away from the free or unligated adapters by column chromatography using Sepharose CL-4B resin followed by size selection of the cDNA on a 1.2% agarose gel. After visualizing the resolved DNA using ethidium bromide, two fractions of cDNA, > 3.5 kb and 1.0-3.5 kb, were isolated from the gel and inserted into the vector λgt10.

The ligated λgt10 containing the cDNA insert was packaged into λ phage virions *in vitro* using the Gigapack III Gold packaging (Stratagene, La Jolla, CA) kit. Using this method, phage libraries of ~1.5 x 10⁶ recombinants for cDNA > 3.5 kb fraction and ~10 x 10⁶ recombinants for DNA fraction between 1.0 and 3.5 kB were obtained.

### D. Isolation of DNA encoding a portion of human α₁ calcium channel subunits

DNA encoding a small region of human α₁ subunits encoded in TT cells was isolated using degenerate PCR-based amplification (e.g., see Williams *et al.* (1994) J. Biol. Chem. 269:22347-22357). These amplified fragments were used to generate DNA probes for the isolation of DNA encoding a full-length human α_{1H} calcium channel subunit.

As noted above, two sets of degenerate oligonucleotides were synthesized based on the flanking regions of the II-III loop known to share a high degree of sequence identity amongst known human α₁ calcium channel subunits: 1) two degenerate oligonucleotides complementary to the regions of the IIS5-IIS6 loop were synthesized as 5' upstream primers (SEQ ID NOs. 4 and 5); and 2) two degenerate oligonucleotides complementary to a portion of the IIIS5 transmembrane segment were synthesized as 3' downstream primers (SEQ ID NOs. 6 and 7).

These degenerate oligonucleotides were used as primer pairs in nested PCR amplification reactions using Pfu DNA polymerase (Stratagene, La Jolla, CA) and reactions were performed according to the manfacturer's instructions. Samples were placed in a commercially available thermocycler (Perkin-Elmer) and the amplification reactions were set as follows: 1 cycle, 5 min @ 95 °C; 5 cycles, 20 sec @ 95 °C/20 sec @ 42 °C/2.5 min @ 72 °C; 30 cycles, 20 sec @ 95 °C/20 sec @ 50 °C/2.5 min @ 72 °C; and 1 cycle, 7 min @ 72 °C. Amplified DNA products were subjected to electrophoresis on an agarose gel and gel purified using standard methods.

### E. Amplification of DNA encoding a portion of human α_{1H} calcium channel subunit

To amplify DNA encoding a portion of the human α_{1H} calcium channel subunit, three degenerate oligonucleotides (SEQ ID NOs. 8-10) that share partial complementarity to a region of Domain III were synthesized as 5' primers. This region is encompassed within all of the amplified α₁-encoding fragments of Section C above. Two oligonucleotides based on sequences in IIIS2 (SEQ ID NOs. 8 and 10) were used as 5' primers in conjunction with the 3'IIIS5 transmembrane primers used in the initial PCR reactions (SEQ ID NOs. 6 and 7 to amplify DNA encoding a portion of the human α_{1H} subunit using the amplified products as templates.

The amplified DNA products were subcloned into the pCR-Blunt vector (Invitrogen), plasmid DNA was purified from isolated transformants and the DNA sequence of each insert was determined. A 340 bp fragment (SEQ ID NO. 11; nt 4271 to 4610 of SEQ ID NO. 12) that shares approximately 55-60% sequence identity to known human α₁ calcium channel subunits was identified. This DNA fragment, designated PCR1, was used as a DNA probe to isolate DNA encoding a human α_{1H} calcium channels subunit.

### F. Isolation and characterization of individual clones Hybridization and Washing Conditions

Hybridization of radiolabelled nucleic acids to immobilized DNA for the purpose of screening cDNA libraries, DNA Southern transfers, or northern transfers was routinely performed in standard hybridization conditions (hybridization: 50% deionized formamide, 200 µg/ml sonicated herring sperm DNA (Cat #223646, Boehringer Mannheim Biochemicals, Indianapolis, IN), 5 x SSPE, 5 x Denhardt's, 42° C.; wash :0.2 x SSPE, 0.1 % SDS, 65° C). The recipes for SSPE and Denhardt's and the preparation of deionized formamide are described, for example, in Sambrook *et al.* (1989) *Molecular Cloning, A Laboratory Manual,* Cold Spring Harbor Laboratory Press, Chapter 8). In some hybridizations, lower stringency conditions were used in that 10% deionized formamide replaced 50% deionized formamide described for the standard hybridization conditions.

The washing conditions for removing the non-specific probe from the filters was either high, medium, or low stringency as described below:
1) high stringency: 0.1 x SSPE, 0.1 % SDS, 65°C
2) medium stringency: 0.2 x SSPE, 0.1 % SDS, 50°C
3) low stringency: 1.0 x SSPE, 0.1% SDS, 50°C.
It is understood that equivalent stringencies may be achieved using alternative buffers, salts and temperatures.

Approximately 1.5 x 10⁵ recombinants of the TT cell phage library containing inserts >3.5 kb were plated and duplicate lifts prepared from each plate. The lifts were probed with radiolabelled PCR1 using standard hybridization conditions, the filters were washed and approximately 100 positive plaques were identified. Initially, 5 positives, λ1.201-λ1.205, were selected for plaque purification and characterization.

Restriction endonuclease digestion of purified DNA isolated from λ1.201-λ1.205 with EcoRI indicated that clone 1.201 contains the original insert of ~350 bp PCR1 fragment, whereas clones 1.202, 1.203, 1.204 and 1.205 contain inserts of ~1100, ~4000, ~ 2600 and ~2200 nt, respectively.

### F. Isolation of DNA encoding a human α_{1H} calcium channel subunit and construction of DNA encoding a full-length α_{1H} subunit

### 1. Reference list of partial human α_{1H} clones

The full-length α_{1H} cDNA sequence is set forth in SEQ ID NO. 12. A list of partial cDNA clones used to characterize the α_{1H} sequence and the nucleotide position of each clone relative to the full-length α_{1H} cDNA sequence is shown below. The isolation and characterization of these clones are described below.

| | |
|---|---|
| 1.305 | nt 1 to 3530 of SEQ ID No. 12 |
| 1.205 | nt 2432 to 4658 of SEQ ID No. 12 |
| 1.204 | nt 3154 to 5699 of SEQ ID NO. 12 |
| PCR1 | nt 4271 to 4610 of SEQ ID NO. 12 |
| 1.202 | nt 4372 to 5476 of SEQ ID No. 12 |
| 1.203 | nt 3891 to 7898 of SEQ ID No. 12 |

### 2. Characterizetion of the clones

DNA sequencing of each insert revealed that clone 1.202 contains 1,105 bp insert corresponding to nt 4372 to 5476 of SEQ ID No. 12; clone 1.203 contains 4,008 bp insert corresponding to nt 3891 to 7898 of SEQ ID No. 12; clone 1.204 contains 2,546 bp insert corresponding to nt 3154 to 5699 of SEQ ID NO. 12; and clone 1.205 contains 2,227 bp insert corresponding to nt 2432 to 4658 of SEQ ID No. 12. These four DNA clones contain overlapping sequences that encode an open reading frame of approximately 6.6 kb that encodes a majority of the α_{1H} subunit, including the entire carboxy terminus and the in-frame translational stop codon.

DNA encoding the 5'-end of the human α_{1H} calcium channel subunit was isolated using a 548 bp EcoRl-NcoI restriction endonuclease fragment from the 5'-end of clone 1.205 (nt 2432 to nt 2979 SEQ ID No. 12) to rescreen the TT cell cDNA library under high stringency conditions.
Briefly, DNA encoding the amino terminus of human α_{1H} calcium containing inserts of >3.5 kb was incubated with the purified restriction fragment and hybridized at 42 °C and washed under high stringency conditions as described above.

One recombinant, clone 1.305, was identified that contains a 3,530 nucleotide insert that shares at its 3' end approximately 1.1 kb of sequence identity with the 5'-end of clone 1.205 (~nt 2432 to nt 3530 SEQ ID No. 12) and also contains 2.4 kb of sequence upstream of the EcoRI site located at the 5'-end of clone 1.205 (nt 2433 to 2438 SEQ ID No. 12). This sequence encodes the ATG initiation codon (nt 249 to nt 251 SEQ ID No. 12) and 1,094 amino acids of the amino terminus of the α_{1H} subunit as well as 248 bp of 5'-untranslated sequence, including a consensus ribosome binding site (nt 244 to nt 248 of SEQ ID No. 12).

Two other recombinants were also identified (SEQ ID NOs. 13 and 14) that share approximately 1.1 kb of sequence identity with the 3'-end of clone 1.305 but differ in the length of the DNA sequence corresponding to the extended intracellular loop located between transmembrane Domains I and II.

### 3. Construction of a full-length α_{1H-1}-encoding DNA clone

Portions of these partial cDNA clones can be ligated to generate a full-length α_{1H} cDNA using common restriction endonuclease sites shared amongst the α_{1H}-encoding fragments. A full-length α_{1H} encoding clone was constructed by 1) combining the DNA encoding the 5'-end of α_{1H} present in clone 1.305 with clone 1.205 using a common EcoRI site (nt 2433 to 2438 SEQ ID No. 12); and 2) the resulting clone, which encodes the amino terminus of α_{1H} was combined with the carboxyl terminal sequences of α_{1H} encoded in clone 1.203 using the common EcoRV restriction endonuclease site shared between clone 1.205 and 1.203 (nt 4517-4522 of SEQ ID NO. 12). The resulting full-length human α_{1H} calcium channel subunit is 2,353 amino acid residues in length (SEQ ID NO. 12). The expression construct was assembled in pCDNA1 (Invitrogen, San Diego, CA) and included a consensus ribosome binding site (RBS) followed by the full-length α_{1H} coding sequence (see, for a description of pcDNA1-based vectors containing the RBS, see, *e.g.*, in International PCT application No. PCT/US94/09230, see, also allowed U.S. application Serial No. 08/149,097, U.S. Patent No. 5,851,824, and U.S. Patent No. 5,846,756). The resulting construct was designated pcDNA1α_{1H}RBS.

### EXAMPLE 2: Cloning of human calcium channel α_{1H-2} subunit

T-type channel currents are heterogeneous among different cell types, with varying biophysical and pharmacological profiles, and as shown in this and the following examples can result from expression of different α₁ subunit subtypes in different cells.

### A. Cloning of α_{1H-2}

As described above, PCR Primers-1 and -2,chosen based on an alignment of the human α_{1A}-α_{1E} sequences in the central cytoplasmic loop II/III region and Primer-3 (GA(A/G)ATGATGATGAA(A/G)GT SEQ ID NO. 10) was chosen after considering α₁-related *C. elegans* sequences in cosmid C54D2 aligned with the human α₁-encoding nucleic acid sequences.

The α₁-related encoding nucleic acids were amplified in two steps from TT cellular poly(A) + RNA, using Primers-1 and -2 first in a degenerate amplification reaction followed by Primer-3 and Primer-2 in a nested PCR amplification. This resulted in amplification of a 340 nucleotide fragment that encodes a portion of the α_{1H} subunit. This amplification product was used as a probe to screen the library to isolate nucleic acid clones encoding a full-length α_{1H} subunit.

Using a primer base on the α_{1H-1} sequence and RT-PCR on various tissues, transcripts with an in-frame deletion relative to α_{1H-1} were identified and isolated from the TT cell library. Fragments spanning this deletion were isolated and, when lined up matched the α_{1H-1}, sequence except for a 957 base pair deletion. A full-length clone, designated α_{1H-2} (see SEQ ID NO. 1 6), was constructed from among these fragments, and inserted in the pcDNA1 with the RBS as for α_{1H-1}. α_{1H-2} transcripts were identified in all tissues examined.

Nucleic acid encoding α_{1H-2} results from an alternately spliced RNA and has a 957 nucleotide in-frame deletion relative to α_{1H-1}, as detected in the PCR products from numerous tissues and cells, including TT cellular cDNA" amygdala cDNA, caudate nucleus cDNA, putamen cDNA, heart cDNA, kidney cDNA and liver cDNA. PCR primers were: (i) 5'-primer corresponding to the sense strand of α1H-1 at nucleotide 1373 through 1393; (ii) 3'-primer corresponding to the antisense strand of α1H-1 at nucleotide 2657 through 2680.

SEQ ID Nos. 12 and 15 show the nucleotide sequence of α_{1H-1}. The coding sequence for α_{1H-1} begins at nucleotide 249 and ends at 7310. (SEQ ID Nos. 12 and 15 differ in minor respects,
e.g., amino acid 2230 (bases 6983-6985) is Asp (GAC) in the SEQ ID No. 15 and Glu (GAA) in SEQ ID No. 12).

SEQ ID No. 16 shows the nucleotide sequence of the α_{1H-2} splice variant. The coding sequence for α^{1H-2} begins at 249 and ends at 6353.

### B. Summary

Nucleic acid clones encoding full length α1H T-type channel subtype were isolated from TT cells. Although similar in overall nucleotide sequence topography to other previously cloned HVA α₁ subunits, the α_{1H} subunit contained several unusual features, including a large I-II domain loop, absence of the common α₁ interaction domain, and altered ion selectivity properties. Two isoforms of α_{1H} designated α_{1H-1} and α_{1H-2} were identified. The first α_{1H-1} is the larger of the two, and the second α_{1H-2} is the smaller of the two containing a 957 nucleotide deletion in the I-II loop relative to α_{1H-1}. The nucleotide sequence of α_{1H-1} is set forth in SEQ ID No. 12 and No. 15 and that of α_{1H-2} is set forth in SEQ ID NO. 16. α_{1H-2} contains a 957 nucleotide deletion relative to α_{1H-1} which results in a loss of 319 amino acids (amino acids 470-788 of α_{1H-1}) from within the intracellular loop between domains I and II. The splice variant deletion was identified by PCR in all cells and tissues examined. These include TT-cells, amygdala, caudate nucleus, putamen, heart, kidney and liver cells. In the brain expression is primarily in the amygdala, caudate nucleus and putamen. Liver, kidney and heart have high levels. The coding sequence for α1H-1 begins at nucleotide 249 and ends at nucleotide 7310 while the coding sequence for α_{1H-2} begins at nucleotide 249 and ends at nucleotide 6353.

Polyclonal antiserum was raised to the putative II-III intracellular loop domain of the α1H subunit. Following transient expression in HEK293 cells a protein of the appropriate size was detected by SDS-PAGE and Western blotting. Functional characterization of human α_{1H} channels is provided in EXAMPLE 3.

### EXAMPLE 3: Biophysical and Pharmacological properties of channels containing α_{1H-1} and α_{1H-2} subunits

### A. Materials and Methods

Materials and methods for biophysical and pharmacology study of calcium channel subunits are described in this EXAMPLE and EXAMPLE 4 below with reference to previously cloned subunits. Such methods or other similar methods known to those of skill in the art have been used to study these properties of human α_{1H-1} subunits as described in this Example.

**Electrophysiology:** HEK293 cells were transiently transfected with 6 µg pcDNA1α_{1H}RBS using a standard Ca²⁺ phosphate procedure (see, e.g. , EXAMPLE below, see, also Williams et al. (1992) Neuron, 8:71-84, for transfection procedure). pCMVCD4, a human CD expression plasmid, was included in the transfections as a marker to permit the identification of transfected cells. Prior to recording, cells were washed with mammalian Ringer's solution, incubated for approximately 10 min in a solution containing a 1/1000 dilution of M-450 CD4 Dynabeads (Dynal Inc., Lake Success, NY) and rewashed with mammalian Ringer's solution to remove excess beads. Functional expression of α_{1H} channels in transfected cells was evaluated 24-48 hours following transfection using the whole-cell patch clamp technique. All recordings were performed on single cells at room temperature (19-24°C). Whole-cell currents were recorded using an Axopatch-200A (Axon Instruments, Foster City, CA) or anEPC-9 (HEKA elektronik, Lambrecht, Germany) patch clamp amplifier, low-pass filtered at 1 kHz (-3 dB, 8-pole Bessel filter) and digitized at a rate of 10 kHz, unless otherwise stated. Pipettes were manufactured from borosilicate glass (TW150, WPI, Sarasota, FI), coated with Sylgard (Dow Corning Midland, MI), and had a resistance of 1.1-2.0 MΩ when filled with internal solution. Series resistance was 2-5 MΩ and 70-90% series resistance compensation was generally used. The pipette solution contained (in mM): 135 CsCl, 10 EGTA, 1 MgCl₂, 10 HEPES (pH 7.3, adjusted with Cs-OH). The external solution contained (in mM): 15 BaCl₂ or CaCl₂, 150 Choline C1, 1 MgCl₂, 5 TEA-OH and 10 HEPES (pH 7.3, adjusted with HC1). Single channel recordings were obtained using the cell-attached configuration of the patch-clamp technique. The pipette solution contained (in mM): 110 BaCl₂, 10 HEPES (pH 7.3, adjusted with TEA-OH). The membrane potential of individual HEK293 cells was set to zero with a solution containing (in mM): 140 K-aspartate, 5 EGTA, and 10 HEPES (pH 7.3). Membrane potentials in the single channel recordings were not corrected for liquid junction potential offset (+12 mV). Linear leak and residual capacitive currents were on-line subtracted using a P/4 protocol (whole-cell recording) or scaled single-channel sweeps with no activity (single-channel recordings).

**Drugs:** Mibefradil (Ro 40-5967) was a gift from F. Hoffman-LaRoche. Nimodipine and (-)BayK-8644 were obtained from Research Biochemicals (Natick, MA). The peptide toxins w-CgTx GVIA (conotoxin) and ω-CmTx MVIIC (conotoxin) were obtained from Bachem (Torrance, A). All remaining compounds were obtained from Sigma. Stock solutions were prepared in dimethl sulfoxide (amiloride, nimodipine), ethanol ((-) BayK-8644) or water (verapamil, mibefradil, ethosuximide, ω-CmT× GVIA and ω-CmTx MVIIC) and stored at 4°C. Drugs were prepared fresh on each experimental day from stock solutions and applied via peristaltic pump at a flow rate of <0.5 ml/min. The maximal solvent concentration in the final test solution was <0.1%. At these concentrations these solvents ha no effect on α_{1H}-mediated currents.

***Xenopus* oöcyte studies:** Xenopus laevis frogs were purchased from Nasco (Fort Atkinson, Wisconsin). Oöcytes were incubated in Ca²⁺⁻free solution containing 88 mM NaCl, 1 mM KCl, 0.82 mM MgSO₄, 2.4 mM NaHCO₃, 10 mM Hepes and 1.5 mg/ml collagenase A (Worthington, Freehold NJ; Type 4, 1.5 hr and subsequently Sigma, St. Louis, MO, Type 1A, 0.5 hr.). Following collagenase treatment, oöcytes were transferred to frog Ringer's solution that contained 88mM nACl, 1mM KCl, 0.91 mM CaCl₂, 0.82 mM MgSO₄, 0.33 mM Ca(NO₃)₂, 2.4 mM NaHCO₃ and 10 mM Hepes. Under these conditions, manual removal of the follicle cell layer was not required. Oöcytes were injected with 50 ng (1µg/ml) of *in vitro* transcripts encoding the α_{1H} subunit and incubated for 3-5 days at 19°C prior to recording. The incubation medium was frog Ringer's solution containing penicillin/streptomycin (Sigma; 10 ml/L), gentamicin (Sigma; 1 ml/L and 5% heat-inactivated horse serum (Gibco, Gaithersburg, MD). Microelectrodes were pulled on a horizontal puller (Model P80, Sutter Instruments, Novato, CA); filled with 3 M KCl; and selected for resistances in the range of 0.5-2.0 MΩ. Data were recorded using a GeneClamp 500; digitized at 1-5 KHz; and stored on magnetic disks for analysis offline using pClamp or Axograph software (Axon Instruments). Ba²⁺ or Ca²⁺ currents were recorded in a solution containing 36 mM TEA-OH, 2.5 mM KOH, 75 mM mannitol, 10 mM HEPES and 15 mM Ba(OH)₂ or Ca(OH)₂, respectively at pH 7.3. Currents were leak-subtracted using the P/6 protocol. To block Ca²⁺-activated chloride currents, niflumic acid (300*µ*M) was included in experiments where the relative permeability of α_{1H} channels to Ba²⁺ or Ca²⁺ was measured. All values are reported as mean ± S.D. unless stated otherwise. Drugs (above) were applied via a gravity-fed perfusion system. At the concentrations used herein, solvents had no effect on α_{1H-}mediated currents.

### B. Electrophysiology

### 1. Current-Voltage Properties

The rapid inactivation of α_{1H-1} Ca²⁺ channels was strongly voltage-dependent. The current decay was best described with an exponential function with time constants ranging from 42.2 ± 7.8 to 8.8 ± 3.8 ms at membrane potentials between -50 and + 30 mV (n = 6; data not show). Activation kinetics of α_{1H-1} Ca²⁺ channels were also voltage-dependent with time constants ranging from 9.9 ± 4.7 to 0.9 ± 0.3 ms for membrane potentials between -50 and + 30 mV (n = 8; data not shown). α_{1H-1} Ca²⁺ channels inactivated completely during the 150-ms depolarization. Recovery from inactivation occurred within a period of ∼ 3 s with a fast component (τ = 37 ± 9 ms; 16.5 ± 4.6% of all channels) and a slow component (τ = 37 ± 61 ms; 78 ± 8.5% of all channels; n = 3; data not shown). To confirm the biophysical properties of recombinant α_{1H} channels observed in whole-cell recordings from HEK293 cells, the functional expression of α_{1H} in *Xenopus* oöcytes was tested. Substantial currents (< 1 *µ*A) after injection of α_{1H} transcripts alone was observed.

The current-voltage relationship for Ba²⁺ or Ca²⁺ from traces determined. Following transient transfection of HEK293 cells with a DNA encoding the α_{1H-1} subunit, Ba²⁺ currents that were rapidly activating and inactivating were observed. Ba²⁺ currents (15 m*M*) elicited by step depolarizations to various test potentials from a holding potential of -90-mV were measured. Currents were activated at a test potential of -50 mV, peaked between -20 and -10 mV, and reversed at a membrane potential more positive than + 60 mV. Similar results were obtained with Ca²⁺ (15 mM) as the charge carrier.

### 2. Voltage-Dependence of Activation and Inactivation

FIGURE 1 shows the voltage-dependence of activation (moo) and steady-state inactivation (h) of human α_{1H} calcium channels expressed transiently in HEK cells. Voltage-dependence of activation (moo) was determined from tail current analysis. Tail currents were normalized with respect to the maximum peak tail current obtained at + 60 mV and were plotted (open symbols, mean ± SEM; n = 11) vs. test potential. Data were fitted by the sum of two Boltzman function m∞=FA*[1 + exp (-(Vtest-V1/2,A)/KA)]1 +F_{B}*[1 +exp(-(Vₜₑₛₜ-V_{1/2.B})/k_{B})]⁻¹, F_{A}=0.67, V_{1/2.A}=-21.5mV, k_{A}=7.5, F_{B} = 0.33, V_{1/2.B} = 25.5 mV, k_{B} = 14.7. Steady-state inactivation (h∞) was determined from a holding potential of -100 mV by a test pulse to -20 mV (p1), followed by a 20 second prepulse from -100 mV to -10 mV in 5 mV decrements (pHold) preceding a second test pulse to -20 mV (p2). Normalized current amplitudes were plotted (closed symbols, mean ± SEM; n = 9) vs. holding potential. Data were fitted by a Boltzman function h∞ =[1 + exp((V_{hold}-V_{1/2})/k)]⁻¹, V_{1/2}=-63.9 mV, k = 3.9mV.

### 3. Tail Current Deactivation

Tail current deactivation profiles for α_{1H-1} calcium channels in transiently transfected HEK cells were studied. One hallmark of LVA channels is their slow rate of deactivation, which is reflected in a show decay of tail currents. The time constant of this decay is ~10-fold slower for LVA channels (2-12 ms) than for HVA channels < 300 *µ*s. A slow decay of α_{1H-1} mediated tail currents over a period of ~ 15 ms was observed. In contrast to the monoexponential decay of the tail currents reported for many native T-type Ca²⁺ channels, tail currents from α_{1H-1} channels showed a biexponential decay. At a test potential of -20 mV, the decay rate of the slow component, comprising 88.1 ± 33.8% of the total current, was 2.1 ± 1.06 ms (n = 6), which is similar to those observed in native T-type Ca²⁺ channels. The decay rate of the faster component was 0.64 ± 0.21 ms (n = 6). Slow decay of α_{1H-1}-mediated tail currents were observed over a period of 15 ms.

The voltage dependence of activation of α_{1H-1} containing Ca²⁺ channels was determined from tail-current analysis. Normalized tail-current amplitudes were plotted as a function of test potential and revealed a biphasic activation curve that was well fitted by the sum of two Boltzmann functions (Figure 1). The potentials for half-maximal activation of the individual Boltzmann terms were as follows: V_{½,A}: -25.1 ± 3 3.0 mV; and V_{½,B}: + 25.5 ± 3 9.9 mV (n = 11). A value similar to V_{½,A} has been reported previously for voltage dependence of activation of T-type CA²⁺ channels in the human TT cell line (-27 mV). The value of the second Boltzmann term V_{½,B} is somewhat similar to that reported for HVA Ca²⁺ channels. Using a similar protocol, tail currents of HVA Ca²⁺ channels decay with time constants of < 300 µs, whereas with α_{1H} the most prominent at test potentials close to V_{½,B}. The availability of α_{1H} containing Ca²⁺ channels for opening was dependent on the membrane for potential as shown in FIGURE 1. The potential for half-maximal steady-state inactivation (V_{½}) was - 63.2 ± 2.0 mV (n = 9).

### 4. Kinetics of Activation and Inactivation of α_{1H} Channels

FIGURE 2 shows the kinetics of activation (FIGURE 2A) and inactivation (FIGURE 2B) of human α_{1H} calcium channels. Kinetics of activation and inactivation were determined from current traces by fitting an exponential function to rising (FIGURE 2A) or declining (FIGURE 2B) phase of the current. The voltage-dependence for activation and inactivation follows approximately an exponential function.

### 5. Recovery from Inactivation

Recovery of α_{1H} channels expressed transiently in HEK293 cells from inactivation induced by using a double pulse protocol using depolarizing pulses to -20mV was evaluated. The fraction of recovered channels was plotted vs. interpulse interval and the data point were fitted by a bi-exponential function in the form 1= Ao + A1 exp(-t/τ1) + A2exp(-t/τ2). τ1:35 ms, A1:0.165, τ2:337 ms, A2:0.788.

### 6. Single-Channel Recording from Human α_{1H} calcium channels

Single-channel properties of α_{1H}Ca²⁺ channels in HEK293 cells were determined in cell-attached recordings with 110 mM Ba²⁺ as the charge carrier. Single-channel recordings at a test potential of -30 mV from a patch that contains at least three α_{1H} showed that channel openings occurred in bursts and were clustered mainly in the first third of the 100-ms depolarizing pulse, especially with stronger depolarizations. Occasionally, channel activity was spread throughout the entire sweep. The time course of the ensemble-averaged current recorded at -30mV in 110 m*M* Ba²⁺ was similar to the α_{1H} whole-cell Ba²⁺ current recorded at -40 mV in 15 m*M* Ba²⁺. The currents were compared at different potentials to compensate for the shift in the activation curve to more positive potentials due to the increase in divalent concentration. The unitary current-voltage relationship yielded a unitary slope conductance of 9.06 ± 0.22 pS (n = 4).

### C. Biophysical Characterization of Human α_{1H} calcium channels in Xenopus Oöcytes

### 1. Overview

Cloned human α_{1H} calcium channels were characterized further by transient expression of α_{1H-1} mRNA in *Xenopus* oöcytes. Injection of α_{1H-1} mRNA alone resulted in expression of large currents, i.e., typically > 1 *µ*A when recording in 15 mM Ba²⁺. The α_{1H} channels were activated at approximately -50 mV with peak responses between -30 mV and -40 mV, which is consistent with low voltage activated channels. Permeability of the α_{1H} channels to Ca²⁺ was slightly greater than to Ba²⁺. In contrast with high voltage channel, the α_{1H} channels activated slowly (τ = 5.7 ± 1.0 ms at the peak of the I-V curve, 3.3 ± 0.5 ms at -20mV) and inactivated rapidly (τ = 13.4 ± 1.9 ms at the peak of I-V curve, 12.2 ± 1.5 ms at -20 mV). The α_{1H} channels expressed in oöcytes were sensitive to steady-state inactivation at relatively negative membrane potentials (V1/2 =-64.5 ± 1.0 mV) and recovered quickly from inactivation (τ of recovery = 330 ms). These values are very similar to those obtained from α_{1H} channels expressed in HEK293 cells. The Ba²⁺ currents through α_{1H} channels in oöcytes were sensitive to blocking by Ni²⁺ and Cd²⁺ with IC50 values of 6.3µM and 8.3µM, respectively. Of the antagonists tested, only amiloride (1C50≈16µM) and mibefradil (IC50≈2µM) markedly inhibited α_{1H}-mediated Ba²⁺ currents through α_{1H} channels expressed in oöcytes. Taken together the results indicate that α_{1H} represents a low-voltage activated calcium channel subunit.

### 2. Activation and Inactivation Properties of α_{1H} Channel Ba²⁺ Currents

Current-voltage relationships for Ba²⁺ (15 mM) currents were recorded from single oocytes injected with mRNA encoding the human α_{1H} subunit. Ba²⁺ currents were activated at a membrane potential of about -50 mV and peaked at -30 mV. The relative inactivation rates of human α_{1H} channels were investigated in different oöcyte preparations and compared with inactivation rates of *α*1A*-*2*α*2b*δβ*4a channels; α1B-1α2bδβ3a channels; and, α1E-3α2bδβ1b channels. Ba²⁺ currents were elicited using a voltage command in the range of -120 mV to -30 mV for α_{1H} channels, or -90 mV to 0 mV or + 10 mV for the other respective α_{1A}, α_{1B} and α_{1E} containing channels. The results presented show the relatively electro-negative activation range of α_{1H} channels in comparison with the high-voltage activated α1A-2α2bδβ4a, α1B-1α2bδβ3a and, α1E-3α2bδβ1b calcium channels.

### 3. Permeability, Inactivation and Biophysical Properties of Human α_{1H} Expressed in Xenopus oöcytes

Permeability and inactivation properties of human α_{1H} channels were investigated in oöcytes by studying Ba²⁺ and Ca²⁺ currents. The results show that Ba²⁺ currents were not significantly larger than Ca²⁺ currents in oöcytes expressing the α_{1H} subunit. Results presented in show normalized steady-state inactivation curves for α_{1H}-mediated Ba²⁺ currents, where V1/2 was calculated to be equal to a value of -64.5 ± 1.0 mV. A double pulse protocol, i.e., with increasing time intervals between pulses, was used to examine the recovery of α_{1H} channels from inactivation. The results of relative recovery of channels plotted against the interpulse interval (ms) and demonstrated that α_{1H} channel currents recovered quickly from inactivation, with an average time constant of 330 ms (n = 5).

### 4. Cadmium, Nickel, Amiloride and Mibefradil Antagonize human α_{1H} Channel Ba²⁺ Currents

Cd²⁺ was found to antagonize low-threshold human α_{1H} currents in oöcytes in a concentration dependent manner. By plotting the inhibition of Cd²⁺ as the percentage of the control Ba²⁺ current achieved at different concentration of Cd²⁺, an IC₅₀ of 10.3µM as calculated. Ni²⁺ was also found to antagonize low-threshold human α_{1H} channels in oöcyte, and also in a concentration dependent manner. The inhibition of Ba²⁺ currents produced by different concentrations of Ni²⁺ (n=4 experiments; n_{H} = 0.84) was tested. The calculated IC₅₀ for Ni²⁺ was 6.3µM. Antagonism by NI²⁺ and Ba²⁺ were largely reversible.

In addition, each of Amiloride and Mibefradil blocked low-threshold Ba²⁺ currents in oöcytes in a concentration-dependent manner giving a calculated IC₅₀ of 161µM for Amiloride; mean of 7 experiments, n_{H}=0.62) and mean of 2.1 µM for Mibefridil; mean of 4 experiments, n_{H}=0.71).

These results demonstrate that incorporation of an α_{1H} subunit into functional calcium channels in the membranes of cells, conveys the electrophysiologic and biophysical properties of low-voltage activated, particularly T-type, calcium channels upon those channels. The α_{1H-}containing channels were activated rapidly at relatively negative membrane potentials (i.e., V_{1/2} = 64.5 mV), and were also inactivated rapidly (i.e., τ = 12.2 ms at -20mV). Peak channel open activity was observed at a membrane potential of -30mV. These channels also exhibited approximately equal permeability for Ca²⁺ and Ba²⁺.

Pharmacologic properties of α_{1H} containing channels were also consistent with those of other low-threshold calcium channels. They are blocked by Ni²⁺ (IC₅₀=6.3µM), Cd²⁺ (IC₅₀=10.3µM), Amiloride (IC₅₀= 16.1µM) and Mibedfradil (IC₅₀=2.1µM).

### D. Comparison of calcium channels containing human α_{1H} subunits expressed in HEK293 Cells with those expressed in Xenopus oöcytes

TABLE 4 summarizes the biophysical properties of: (i) human α_{1H-1-}containing calcium channels expressed in HEK293 cells, (ii) human α_{1H-1-}containing channels expressed in *Xenopus* oocytes, and (iv) native T-type calcium channels expressed in various tissues.

**TABLE 4**

| **Biophysical properties of α**_{**1H**}**-containing Ca**^{**2+**} **channels** | | | | |
|---|---|---|---|---|
| **Properties:** | | α_{1H} **HEK293** | α_{1H} ***Xenopus* Oöcytes** | **Native T-type**^{**b**} |
| Relative conductance | | Ba²⁺≡ Ca²⁺ | Ba²⁺≅ Ca²⁺ | Ba²⁺ ≅ Ca²⁺ |
| conductance [pS] | | 9.06 ± 0.22 | n.d. | 5-9 |
| Activation | | | | |
| | kinetics, τ[ms] | 2.8 ± 0.5^{c} | 3.3 ± 0.5^{c} | 2 to 8 |
| | V_{1/2}[mV] | -25.1 ± 3.9 | n.d. | -60 to -45 |
| | | 25.5 ± 9.9 | | |
| Inactivation | | 16.9 ± 5.3^{c} | 23.3 ± 1.5^{c} | 10 to 30 |
| | kinetics, τ[ms] | -63.2 ± 2.0 | -64.5 ± 1.0 | -100 to -50 |
| | V_{1/2}[mV] | 0.64 ± 0.21 | n.d. | 2 to 12 |
| Tail deactivation τ[ms] | | 2.1 ± 1.06 | | |

| | | | | |
|---|---|---|---|---|
| *b* Huguenard (1996) Annual Rev. Physiol. 58:329-348; c determined at -20 mV test potential; n.d. not determined | | | | |

### E. Properties of calcium channels containing α_{1H-2} subunits

### Summary Discussion

The biophysical properties of α_{1H-2}, revealed a shift in the V_{1/2} of isochronic inactivation (20 seconds) to -73 mV compared to a V_{1/2} of -62.5 mV for α_{1H-1}. The V_{1/2} of α_{1H-2}, thus exhibits a range closer to V_{1/2} values reported for certain native T-type calcium channels (Huguenard (1996) Annual Rev. Physiol. 58:329-348). For example, under similar recording conditions the V_{1/2} of isochronic inactivation for T-channels in rate dorsal horn neurons (DHN) is reported to be -82 mV, while the V_{1/2} recorded in rate dorsal lateral geniculate neurons (LGN) is -64 mV. In addition, the V_{1/2} of α_{1H-2} more closely approximates the V 1 /2 in native rat DHN compared to the value for α_{1H-1}, which, instead, comes closer to the value recorded for T-type calcium channels in LGN. Thus, the observed differences the amino acid sequence of the α_{1H-1} and α_{1H-2} subunits appears linked to differences in tissue distribution of these two different forms of the α_{1H} channel. These results also provide basis for understanding the observed different broad ranges of values that have been reported for the V_{1/2} inactivation of T-type calcium channels (-100 to -50 mV) in different tissues (see, *e.g.*, Huguenard (1996) Annual Rev. Physiol. 58:329-348).

### F. Summary of Biophysical Properties of Human α_{1H} Containing calcium channels

TABLE 5 summarizes the biophysical properties of calcium channels containing the human α_{1H} subunits.

**TABLE 5**

| **Comparison of biophysical parameters of α**_{**1H**} **subunits transiently expressed in HEK293 cells using 15 MM Ba**^{**2+**} **as the charge carrier:** | | | | |
|---|---|---|---|---|
| | **Parameter** | **α**_{**1H-1**} | **α**_{**1H-2**} | **Statistical significance** |
| Current voltage relationship Isochronic inactivation (20 seconds) | max current at x [mV] | -10 | -20 | p<0.05 |
| | V_{1/2}[mV] | -62.5 | -73 | p<0.05 |
| | Slope | -3.45 | -3.82 | no (0.279) |
| Steady-state activation | V_{1/2.A}[mV] | -23.7 | -33.8 | p<0.05 |
| | Slope_{A} | 8.03 | 5.51 | p<0.05 |
| | Fraction_{A} | 0.617 | 0.519 | no (0.133) |
| | V_{1/2.B}[mV] | 23.1 | 10.7 | p<0.05 |
| | Slope_{B} | 10.9 | 11.6 | no (0.742) |

| | | | | |
|---|---|---|---|---|
| α_{1H-1} corresponds to the wild type form of the subunit; α_{1H-2} to the splice variant form; Steady-state activation from Boltzman fit in the form: m∞ = Fractionₐ* [1 +exp(-(Vₜₑₛₜ-V_{1/2,A})/Slope_{A})]⁻¹ +(1-Fraction_{A})*[1 +exp(-(Vₜₑₛₜ₋V_{1/2,B}/Slope_{B})]⁻¹; Isochronic inactivation (or steady-state inactivation) from Boltzman fit in the form: h∞= =[1 +exp((Vₜₑₛₜ-V_{1/2})/Slope)]⁻¹ | | | | |

### G. Pharmacologic Profile of Human α_{1H} calcium channels

The sensitivity of α_{1H}Ca²⁺ channels expressed in HEK293 cells to several agents known to act on VGCCs (Table below) was tested. α_{1H-}mediated currents were 16-fold more sensitive to Ni²⁺ (IC₅₀=6.6 *µM)* than to Cd²⁺ (IC₅₀ = 104*µM*). Currents were also inhibited by the T-type channel antagonists amiloride (IC₅₀ = 167*µM*) and mibefradil (51.0 ± 10.0% at 1 *µM;* n = 5). In contrast, the T-type channel antagonist ethosuximide produced little inhibition of α_{1H}-mediated currents (7.2 ± 1.8% inhibition at 300 *µM*; n = 5). The calcium channel inhibitor verapamil, the L-type antagonist nimodipine, and the L-type agonist (-)-Bay K 8644 had little effect on α_{1H} channels at a concentration of 1 *µM*. A higher concentration (10 *µM*) of nimodipine or (-)-Bay K 8644 produced a marked inhibition (43.7 ± 4.1 %, n = 4, and 18.1 ± 9.1 %, n = 5, respectively). The peptide toxins ω-CgTx GVIA and ω-CmTx MVIIC at a concentration of 1 µM provided little or no inhibition of α_{1H}-mediated currents.

Pharmacological studies reveal the following rank order of potency for inhibition of α_{1H-1}-containing channels: ni²⁺ (IC50: 6.6 µM) ≈ Mibefradil (51 % at 1 µM) > Cd²⁺ (IC50: 104 µM) > Amiloride (IC50: 1 67 µM) > > Ethosuximide (7% at 300 µM). Nimodipine, Verapamil, ω-CgTx GVIA and ω-CmTx MVIIC had little effect (0-17%) at a concentration of 1 µ*M*. These findings demonstrate that α_{1H}-containing calcium channels have properties corresponding to native LVA, or T-type calcium channels.

Table 6 summarizes the pharmacological profile of human α_{1H} containing calcium channels expressed in HEK293 cells. With the exception of w-CmTx MVIIC, in all cases the charge carrier was 15 mM Ba²⁺. In the case of w-CmTx MVIIC the charge carrier for was 2 mM Ba²⁺ because w-CmTx MVIIC was a more effective inhibitor at lower divalent concentrations. Values for % block are mean ± SD(n). IC₅₀ values were calculated from sigmoidal curve fitting data (Prism, Graphpad Inc.) for data points from 3 to 6 determinations.

**TABLE 6**

| **Pharmacology of α**_{**1H**} **Ca**^{**2+**} **Channels Expressed in HEK293 Cells** | | |
|---|---|---|
| **Compound** | **Concentration** | **% Inhibition of Control Response or IC**_{**50**} |
| Cd²⁺ | range | 104µM |
| Ni²⁺ | range | 6.6µM |
| Amiloride | range | 167µM |
| Mibefradil | 1 µM | 51.0 ± 10.0%(5) |
| Ethosuximide | 300 µM | 7.2 ± 1.8%(5) |
| Verapamil | | |
| Nimodipine | 1 µM | 17.2 ± 1.3%(3) |
| | 1 µM | 3.4 ± 1.1 %(4) |
| (-)BayK- | 10 µM | 43.7 ± 4.1 %(4) |
| 8644 | 1 µM | 0.4 ± 0.8%(3) |
| ω-CgTx | 10*µ*M | 18.1 ± 9.1 %(5) |
| GVIA | 1 *µ*M | 0%(3) |
| ω-CmTx | | |
| MVIIC | 1 *µ*M | 8.6 ± 11.5%(3) |

### EXAMPLE 4: RECOMBINANT EXPRESSION OF HUMAN NEURONAL CALCIUM CHANNEL SUBUNIT-ENCODING cDNA AND RNA TRANSCRIPTS IN MAMMALIAN CELLS

The methods and assays described in this example, may be employed using the nucleic encoding an α_{1H} subunit in place of the α₁ subunits exemplified below. Of particular interest are cells that express the α_{1H} subunit alone, as homomers, monomers or multimers, or in combination with selected α₂ subunits.

### A. Recombinant Expression of the Human Neuronal Calcium Channel α₂ subunit cDNA in DG44 Cells

### 1. Stable transfection of DG44 cells

DG44 cells (dhfr Chinese hamster ovary cells; see, *e.g.,* Urlaub, G. *et al.* (1986) *Som. Cell Molec. Genet. 12*:555-566) obtained from Lawrence Chasin at Columbia University were stably transfected by CaPO₄ precipitation methods (Wigler *et al.* (1979) *Proc. Natl. Acad. Sci. USA 76*:1373-1376) with pSV2dhfr vector containing the human neuronal calcium channel α₂-subunit cDNA for polycistronic expression/selection in transfected cells. Transfectants were grown on 10% DMEM medium without hypoxanthine or thymidine in order to select cells that had incorporated the expression vector. Twelve transfectant cell lines were established as indicated by their ability to survive on this medium.

### 2. Analysis of α₂ subunit cDNA expression in transfected DG44 cells

Total RNA was extracted according to the method of Birnboim ((1988) *Nuc. Acids Res. 16*:1487-1497) from four of the DG44 cell lines that had been stably transfected with pSV2dhfr containing the human neuronal calcium channel α₂ subunit cDNA. RNA (~ 15 µg per lane) was separated on a 1 % agarose formaldehyde gel, transferred to nitrocellulose and hybridized to the random-primed human neuronal calcium channel α₂ cDNA (hybridization: 50% formamide, 5 x SSPE, 5 x Denhardt's, 42° C.; wash :0.2 x SSPE, 0.1 % SDS, 65° C.). Northern blot analysis of total RNA from four of the DG44 cell lines that had been stably transfected with pSV2dhfr containing the human neuronal calcium channel α₂ subunit cDNA revealed that one of the four cell lines contained hybridizing mRNA the size expected for the transcript of the α₂ subunit cDNA (5000 nt based on the size of the cDNA) when grown in the presence of 10 mM sodium butyrate for two days. Butyrate nonspecifically induces transcription and is often used for inducing the SV40 early promoter (Gorman, C. and Howard, B. (1983) *Nucleic Acids Res. 11*:1631). This cell line, 44α₂-9, also produced mRNA species smaller (several species) and larger (6800 nt) than the size expected for the transcript of the α₂ cDNA (5000 nt) that hybridized to the α₂ cDNA-based probe. The 5000- and 6800-nt transcripts produced by this transfectant should contain the entire α₂ subunit coding sequence and therefore should yield a full-length α₂ subunit protein. A weakly hybridizing 8000-nucleotide transcript was present in untransfected and transfected DG44 cells. Apparently, DG44 cells transcribe a calcium channel α₂ subunit or similar gene at low levels. The level of expression of this endogenous α₂ subunit transcript did not appear to be affected by exposing the cells to butyrate before isolation of RNA for northern analysis.

Total protein was extracted from three of the DG44 cell lines that had been stably transfected with pSV2dhfr containing the human neuronal calcium channel α₂ subunit cDNA. Approximately 10⁷ cells were sonicated in 300 *µ*l of a solution containing 50 mM HEPES, 1 mM EDTA, 1 mM PMSF. An equal volume of 2x loading dye (Laemmli, U.K. (1970). *Nature 227*:680) was added to the samples and the protein was subjected to electrophoresis on an 8% polyacrylamide gel and then electrotransferred to nitrocellulose. The nitrocellulose was incubated with polyclonal guinea pig antisera (1 :200 dilution) directed against the rabbit skeletal muscle calcium channel α₂ subunit (obtained from K. Campbell, University of lowa) followed by incubation with [¹²⁵I]-protein A. The blot was exposed to X-ray film at -70° C. Reduced samples of protein from the transfected cells as well as from untransfected DG44 cells contained immunoreactive protein of the size expected for the α₂ subunit of the human neuronal calcium channel (130-150 kDa). The level of this immunoreactive protein was higher in 44α₂-9 cells that had been grown in the presence of 10 mM sodium butyrate than in 44α₂-9 cells that were grown in the absence of sodium butyrate. These data correlate well with those obtained in northern analyses of total RNA from 44α₂-9 and untransfected DG44 cells. Cell line 44_{α2}-9 also produced a 110 kD immunoreactive protein that may be either a product of proteolytic degradation of the full-length α₂ subunit or a product of translation of one of the shorter (< 5000 nt) mRNA produced in this cell line that hybridized to the α₂ subunit cDNA probe.

### B. Expression of DNA encoding human neuronal calcium channel α₁, α₂ and β₁, subunits in HEK cells

Human embryonic kidney cells (HEK 293 cells) were transiently and stably transfected with human neuronal DNA encoding calcium channel subunits. Individual transfectants were analyzed electrophysiologically for the presence of voltage-activated barium currents and functional recombinant voltage-dependent calcium channels were analyzed.

### 1. Transfection of HEK 293 cells

Separate expression vectors containing DNA encoding human neuronal calcium channel α_{1D}, α₂ and β₁ subunits, plasmids pVDCCIII(A), pHBCaCHα₂A, and pHBCaCHβ₁ₐRBS(A), respectively, were constructed as described in International PCT application No. PCT/US94/09230, see, also allowed U.S. application Serial No. 08/149,097. These three vectors were used to transiently co-transfect HEK 293 cells. For stable transfection of HEK 293 cells, vector pHBCaCHβ_{1b}RBS(A) was used in place of pHBCaCHβ₁ₐRBS(A) to introduce the DNA encoding the β₁ subunit into the cells along with pVDCCIII(A) and pHBCaCHα₂A.

### a. Transient transfection

Expression vectors pVDCCIII(A), pHBCaCHα₂A and pHBCaCH/β₁ₐRBS(A) were used in two sets of transient transfections of HEK 293 cells (ATCC Accession No. CRL1573). In one transfection procedure, HEK 293 cells were transiently cotransfected with the α₁ subunit cDNA expression plasmid, the α₂ subunit cDNA expression plasmid, the β₁ subunit cDNA expression plasmid and plasmid pCMVβgal (Clontech Laboratories, Palo Alto, CA). Plasmid pCMVβgal contains the *lacZ* gene (encoding *E. coli* β-galactosidase) fused to the cytomegalovirus (CMV) promoter and was included in this transfection as a marker gene for monitoring the efficiency of transfection. In the other transfection procedure, HEK 293 cells were transiently co-transfected with the α₁ subunit cDNA expression plasmid pVDCCIII(A) and pCMVβgal. In both transfections, 2-4 x 10⁶ HEK 293 cells in a 10-cm tissue culture plate were transiently co-transfected with 5 µg of each of the plasmids included in the experiment according to standard CaPO₄ precipitation transfection procedures (Wigler *et al.* (1979) *Proc. Natl. Acad. Sci. USA 76*:1373-1376). The transfectants were analyzed for β-galactosidase expression by direct staining of the product of a reaction involving β-galactosidase and the X-gal substrate (Jones, J.R. (1986) *EMBO 5*:3133-3142) and by measurement of β-galactosidase activity (Miller, J.H. (1972) Experiments in Molecular Genetics, pp. 352-355, Cold Spring Harbor Press). To evaluate subunit cDNA expression in these transfectants, the cells were analyzed for subunit transcript production (northern analysis), subunit protein production (immunoblot analysis of cell lysates) and functional calcium channel expression (electrophysiological analysis).

### b. Stable transfection

HEK 293 cells were transfected using the calcium phosphate transfection procedure (*Current Protocols in Molecular Biology,* Vol. 1, Wiley Inter-Science, Supplement 14, Unit 9.1.1-9.1.9 (1990)). Ten-cm plates, each containing one-to-two million HEK 293 cells, were transfected with 1 ml of DNA/calcium phosphate precipitate containing 5 µg pVDCCIII(A), 5 µg pHBCaCHα₂A, 5µg pHBCaCHβ_{1b}RBS(A), 5 µg pCMVBgal and 1 µg pSV2neo (as a selectable marker). After 10-20 days of growth in media containing 500 µg G418, colonies had formed and were isolated using cloning cylinders.

### 2. Analysis of HEK 293 cells transiently transfected with DNA encoding human neuronal calcium channel subunits

### a. Analysis of β-galactosidase expression

Transient transfectants were assayed for β-galactosidase expression by β-galactosidase activity assays (Miller, J.H., (1972) Experiments in Molecular Genetics, pp. 352-355, Cold Spring Harbor Press) of cell lysates (prepared as described in International PCT application No. PCT/US94/09230, see, also allowed U.S. application Serial No. 08/149,097) and staining of fixed cells (Jones, J.R. (1986) *EMBO 5*:3133-3142) . The results of these assays indicated that approximately 30% of the HEK 293 cells had been transfected.

### b. Northern analysis

PolyA + RNA was isolated using the Invitrogen Fast Trak Kit (In Vitrogen, San Diego, CA) from HEK 293 cells transiently transfected with DNA encoding each of the α₁, α₂ and β₁ subunits and the *lacZ* gene or the α₁ subunit and the *lacZ* gene. The RNA was subjected to electrophoresis on an agarose gel and transferred to nitrocellulose. The nitrocellulose was then hybridized with one or more of the following radiolabeled probes: the *lacZ* gene, human neuronal calcium channel α_{1D} subunit-encoding cDNA, human neuronal calcium channel α₂ subunit-encoding cDNA or human neuronal calcium channel β₁ subunit-encoding cDNA. Two transcripts that hybridized with the α₁ subunit-encoding cDNA were detected in HEK 293 cells transfected with the DNA encoding the α₁, α₂, and β₁, subunits and the *lacZ* gene as well as in HEK 293 cells transfected with the α₁ subunit cDNA and the *lacZ* gene. One mRNA species was the size expected for the transcript of the α₁ subunit cDNA (8000 nucleotides). The second RNA species was smaller (4000 nucleotides) than the size expected for this transcript. RNA of the size expected for the transcript of the *lacZ* gene was detected in cells transfected with the α₁, α₂ and β₁ subunit-encoding cDNA and the *lacZ* gene and in cells transfected with the α₁ subunit cDNA and the *lacZ* gene by hybridization to the *lacZ* gene sequence.

RNA from cells transfected with the α₁, α₂ and β₁ subunit-encoding cDNA and the *lacZ* gene was also hybridized with the α₂ and β₁ subunit cDNA probes. Two mRNA species hybridized to the α₂ subunit cDNA probe. One species was the size expected for the transcript of the α₂ subunit cDNA (4000 nucleotides). The other species was larger (6000 nucleotides) than the expected size of this transcript. Multiple RNA species in the cells co-transfected with α₁, α₂ and β₁ subunit-encoding cDNA and the *lacZ* gene hybridized to the β₁ subunit cDNA probe. Multiple β subunit transcripts of varying sizes were produced since the β subunit cDNA expression vector contains two potential polyA⁺ addition sites.

### c. Electrophysiological analysis

Individual transiently transfected HEK 293 cells were assayed for the presence of voltage-dependent barium currents using the whole-cell variant of the patch clamp technique (Hamill et al. (1981). *Pflugers Arch. 391*:85*-*100). HEK 293 cells transiently transfected with pCMVβgal only were assayed for barium currents as a negative control in these experiments. The cells were placed in a bathing solution that contained barium ions to serve as the current carrier. Choline chloride, instead of NaCl or KCl, was used as the major salt component of the bath solution to eliminate currents through sodium and potassium channels. The bathing solution contained 1 mM MgCl₂ and was buffered at pH 7.3 with 10 mM HEPES (pH adjusted with sodium or tetraethylammonium hydroxide). Patch pipettes were filled with a solution containing 135 mM CsCl, 1 mM MgCl₂, 10 mM glucose, 10 mM EGTA, 4 mM ATP and 10 mM HEPES (pH adjusted to 7.3 with tetraethylammonium hydroxide). Cesium and tetraethylammonium ions block most types of potassium channels. Pipettes were coated with Sylgard (Dow-Corning, Midland, MI) and had resistances of 1-4 megohm. Currents were measured through a 500 megohm headstage resistor with the Axopatch IC (Axon Instruments, Foster City, CA) amplifier, interfaced with a Labmaster (Scientific Solutions, Solon, OH) data acquisition board in an IBM-compatible PC. PClamp (Axon Instruments) was used to generate voltage commands and acquire data. Data were analyzed with pClamp or Quattro Professional (Borland International, Scotts Valley, CA) programs.

To apply drugs, "puffer" pipettes positioned within several micrometers of the cell under study were used to apply solutions by pressure application. The drugs used for pharmacological characterization were dissolved in a solution identical to the bathing solution. Samples of a 10 mM stock solution of Bay K 8644 (RBI, Natick, MA), which was prepared in DMSO, were diluted to a final concentration of 1 µM in 15 mM Ba²⁺ -containing bath solution before they were applied.

Twenty-one negative control HEK 293 cells (transiently transfected with the *lacZ* gene expression vector pCMVβgal only) were analyzed by the whole-cell variant of the patch clamp method for recording currents. Only one cell displayed a discernable inward barium current; this current was not affected by the presence of 1 *µ*M Bay K 8644. In addition, application of Bay K 8644 to four cells that did not display Ba²⁺ currents did not result in the appearance of any currents.

Two days after transient transfection of HEK 293 cells with α₁, α₂ and β₁ subunit-encoding cDNA and the *lacZ* gene, individual transfectants were assayed for voltage-dependent barium currents. The currents in nine transfectants were recorded. Because the efficiency of transfection of one cell can vary from the efficiency of transfection of another cell, the degree of expression of heterologous proteins in individual transfectants varies and some cells do not incorporate or express the foreign DNA. Inward barium currents were detected in two of these nine transfectants. In these assays, the holding potential of the membrane was -90 mV. The membrane was depolarized in a series of voltage steps to different test potentials and the current in the presence and absence of 1 µM Bay K 8644 was recorded. The inward barium current was significantly enhanced in magnitude by the addition of Bay K 8644. The largest inward barium current (~160 pA) was recorded when the membrane was depolarized to 0 mV in the presence of 1 *µ*M Bay K 8644. A comparison of the I-V curves, generated by plotting the largest current recorded after each depolarization versus the depolarization voltage, corresponding to recordings conducted in the absence and presence of Bay K 8644 illustrated the enhancement of the voltage-activated current in the presence of Bay K 8644.

Pronounced tail currents were detected in the tracings of currents generated in the presence of Bay K 8644 in HEK 293 cells transfected with α₁, *α*₂ and β₁ subunit-encoding cDNA and the *lacZ* gene, indicating that the recombinant calcium channels responsible for the voltage-activated barium currents recorded in this transfected appear to be DHP-sensitive.

The second of the two transfected cells that displayed inward barium currents expressed a ~50 pA current when the membrane was depolarized from -90 mV. This current was nearly completely blocked by 200 µM cadmium, an established calcium channel blocker.

Ten cells that were transiently transfected with the DNA encoding the α₁ subunit and the *lacZ* gene were analyzed by whole-cell patch clamp methods two days after transfection. One of these cells displayed a 30 pA inward barium current. This current amplified 2-fold in the presence of 1 µM Bay K 8644. Furthermore, small tail currents were detected in the presence of Bay K 8644. These data indicate that expression of the human neuronal calcium channel α_{1D} subunit-encoding cDNA in HEK 293 yields a functional DHP-sensitive calcium channel.

### 3. Analysis of HEK 293 cells stably transfected with DNA encoding human neuronal calcium channel subunits

Individual stably transfected HEK 293 cells were assayed electrophysiologically for the presence of voltage-dependent barium currents as described for electrophysiological analysis of transiently transfected HEK 293 cells (International PCT application No. PCT/US94/09230, see, also allowed U.S. application Serial No. 08/149,097). In an effort to maximize calcium channel activity via cyclic-AMP-dependent kinase-mediated phosphorylation (Pelzer, et al. (1990) *Rev. Physiol. Biochem. Pharmacol. 114*:107-207), cAMP (Na salt, 250 µM) was added to the pipet solution and forskolin (10 µM) was added to the bath solution in some of the recordings. Qualitatively similar results were obtained whether these compounds were present or not.

Barium currents were recorded from stably transfected cells in the absence and presence of Bay K 8644 (1 µM). When the cell was depolarized to -10 mV from a holding potential of -90 mV in the absence of Bay K 8644, a current of approximately 35pA with a rapidly deactivating tail current was recorded. During application of Bay K 8644, an identical depolarizing protocol elicited a current of approximately 75 pA, accompanied by an augmented and prolonged tail current. The peak magnitude of currents recorded from this same cell as a function of a series of depolarizing voltages were assessed. The responses in the presence of Bay K 8644 not only increased, but the entire current-voltage relation shifted about -10 mV. Thus, three typical hallmarks of Bay K 8644 action, namely increased current magnitude, prolonged tail currents, and negatively shifted activation voltage, were observed, clearly indicating the expression of a DHP-sensitive calcium channel in these stably transfected cells. No such effects of Bay K 8644 were observed in untransfected HEK 293 cells, either with or without cAMP or forskolin.

### C. Use of pCMV-based vectors and pcDNA1-based vectors for expression of DNA encoding human neuronal calcium channel subunits

### 1. Preparation of constructs

Additional expression vectors were constructed using pCMV. The full-length α_{1D} cDNA from pVDCCIII(A) (see International PCT application No. PCT/US94/09230, see, also allowed U.S. application Serial No. 08/149,097), the full-length α₂ cDNA, contained on a 3600 bp *Eco*RI fragment from HBCaCHα₂ (International PCT application No. PCT/US94/09230, see, also allowed U.S. application Serial No. 08/149,097) and a full-length β₁ subunit cDNA from pHBCaCHβ_{1b}RBS(A) (see International PCT application No. PCT/US94/09230, see, also allowed U.S. application Serial No. 08/149,097) were separately subcloned into plasmid pCMVβgal. Plasmid pCMVβgal was digested with *Not*I to remove the *lacZ* gene. The remaining vector portion of the plasmid, referred to as pCMV, was blunt-ended at the *Not*I sites. The full-length α₂-encoding DNA and β₁-encoding DNA, contained on separate *Eco*RI fragments, were isolated, blunt-ended and separately ligated to the blunt-ended vector fragment of pCMV locating the DNA between the CMV promoter and SV40 polyadenylation sites in pCMV. To ligate the α_{1D}-encoding cDNA with pCMV, the restriction sites in the polylinkers immediately 5' of the CMV promoter and immediately 3' of the SV40 polyadenylation site were removed from pCMV. A polylinker was added at the *Not*I site. The polylinker had the following sequence of restriction enzyme recognition sites: The α_{1D}-encoding DNA, isolated as a *Bam*HI/*Xho*I fragment from pVDCCIII(A), was then ligated to XbaII/*Sal*I-digested pCMV to place it between the CMV promoter and SV40 polyadenylation site.

Plasmid pCMV contains the CMV promoter as does pcDNA1, but differs from pcDNA in the location of splice donor/splice acceptor sites relative to the inserted subunit-encoding DNA. After inserting the subunit-encoding DNA into pCMV, the splice donor/splice acceptor sites are located 3' of the CMV promoter and 5' of the subunit-encoding DNA start codon. After inserting the subunit-encoding DNA into pcDNA1, the splice donor/splice acceptor sites are located 3' of the subunit cDNA stop codon.

### 2. Transfection of HEK 293 cells

HEK 293 cells were transiently co-transfected with the α_{1D}, α₂ and β₁ subunit-encoding DNA in pCMV or with the α_{1D}, α₂ and β subunit-encoding DNA in pcDNA1 (vectors pVDCCIII(A), pHBCaCHα₂A and pHBCaCHβ_{1b}RBS(A), respectively (see, International PCT application No. PCT/US94/09230, see, also allowed U.S. application Serial No. 08/149,097). Plasmid pCMVβgal was included in each transfection as a measure of transfection efficiency. The results of β-galactosidase assays of the transfectants (International PCT application No. PCT/US94/09230, see, also allowed U.S. application Serial No. 08/149,097), indicated that HEK 293 cells were transfected equally efficiently with pCMV- and pcDNA1-based plasmids. The pcDNA1-based plasmids, however, are presently preferred for expression of calcium channel receptors.

### D. Expression in Xenopus laevis oöcytes of RNA encoding human neuronal calcium channel subunits

Various combinations of the transcripts of DNA encoding the human neuronal α_{1D}, α₂ and β₁ subunits prepared *in vitro* were injected into *Xenopus laevis* oöcytes. Those injected with combinations that included a_{1D} exhibited voltage-activated barium currents.

### 1. Preparation of transcripts

Transcripts encoding the human neuronal calcium channel α_{1D}, α₂ and β₁ subunits were synthesized according to the instructions of the mCAP mRNA CAPPING KIT (Strategene, La Jolla, CA catalog #200350). As described in International PCT application No. PCT/US94/09230, see, also allowed U.S. application Serial No. 08/149,097, plasmids pVDCC III.RBS(A), containing pcDNA1 and the α_{1D} cDNA that begins with a ribosome binding site and the eighth ATG codon of the coding sequence plasmid pHBCaCHα₁A containing pcDNA1 and an α₂ subunit cDNA, and plasmid pHBCaCHβ_{1b}RBS(A) containing pcDNA1 and the β₁ DNA lacking intron sequence and containing a ribosome binding site were linearized by restriction digestion. The α_{1D} cDNA- and α₂ subunit-encoding plasmids were digested with *Xho*I*,* and the β₁ subunit- encoding plasmid was digested with *Eco*RV. The DNA insert was transcribed with T7 RNA polymerase.

### 2. Injection of oöcytes

*Xenopus laevis* oöcytes were isolated and defolliculated by collagenase treatment and maintained in 100 mM NaCl, 2 mM KC1, 1.8 mM CaCl₂, 1 mM MgCl₂, 5 mM HEPES, pH 7.6, 20 µg/ml ampicillin and 25 µg/m) streptomycin at 19-25°C for 2 to 5 days after injection and prior to recording. For each transcript that was injected into the oöcyte, 6 ng of the specific mRNA was injected per cell in a total volume of 50 nl.

### 3. Intracellular voltage recordings

Injected oöcytes were examined for voltage-dependent barium currents using two-electrode voltage clamp methods (Dascal, N. (1987) *CRC Crit. Rev. Biochem. 22*:317). The pClamp (Axon Instruments) software package was used in conjunction with a Labmaster 125 kHz data acquisition interface to generate voltage commands and to acquire and analyze data. Quattro Professional was also used in this analysis. Current signals were digitized at 1-5 kHz, and filtered appropriately. The bath solution contained of the following: 40 mM BaCl₂, 36 mM tetraethylammonium chloride (TEA-CI), 2 mM KCl, 5 mM 4-aminopyridine, 0.15 mM niflumic acid, 5 mM HEPES, pH 7.6.

### a. Electrophysiological analysis of oöcytes injected with transcripts encoding the human neuronal calcium channel α₁, α₂ and β₁-subunits

Uninjected oöcytes were examined by two-electrode voltage clamp methods and a very small (25 nA) endogenous inward Ba²⁺ current was detected in only one of seven analyzed cells.

Oöcytes coinjected with α_{1D}, α₂ and β₁ subunit transcripts expressed sustained inward barium currents upon depolarization of the membrane from a holding potential of -90 mV or -50 mV (154 ± 129 nA, n = 21). These currents typically showed little inactivation when test pulses ranging from 140 to 700 msec. were administered. Depolarization to a series of voltages revealed currents that first appeared at approximately -30 mV and peaked at approximately 0 mV.

Application of the DHP Bay K 8644 increased the magnitude of the currents, prolonged the tail currents present upon repolarization of the cell and induced a hyperpolarizing shift in current activation. Bay K 8644 was prepared fresh from a stock solution in DMSO and introduced as a 10x concentrate directly into the 60 µl bath while the perfusion pump was turned off. The DMSO concentration of the final diluted drug solutions in contact with the cell never exceeded 0.1 %. Control experiments showed that 0.1% DMSO had no effect on membrane currents.

Application of the DHP antagonist nifedipine (stock solution prepared in DMSO and applied to the cell as described for application of Bay K 8644) blocked a substantial fraction (91 ± 6%, n = 7) of the inward barium current in oöcytes coinjected with transcripts of the α_{1D}, α₂ and β₁ subunits. A residual inactivating component of the inward barium current typically remained after nifedipine application. The inward barium current was blocked completely by 50 µM Cd²⁺, but only approximately 15% by 100 *µ*M Ni²⁺.

The effect of ω-CgTX-GVIA on the inward barium currents in oöcytes co-injected with transcripts of the α_{1D}, *α*₂*,* and β₁ subunits was investigated. ω-CgTX-GVIA (Bachem, Inc., Torrance CA) was prepared in the 15 mM BaCl₂ bath solution plus 0.1 % cytochrome C (Sigma) to serve as a carrier protein. Control experiments showed that cytochrome C had no effect on currents. A series of voltage pulses from a -90 mV holding potential to 0 mV were recorded at 20 msec. intervals. To reduce the inhibition of ωCgTX binding by divalent cations, recordings were made in 15 mM SaCl₂, 73.5 mM tetraethylammonium chloride, and the remaining ingredients identical to the 40 mM Ba²⁺ recording solution. Bay K 8644 was applied to the cell prior to addition to ωCgTX in order to determine the effect of ωCgTX on the DHP-sensitive current component that was distinguished by the prolonged tail currents. The inward barium current was blocked weakly (54 ± 29%, n = 7) and reversibly by relatively high concentrations (10-15 µM) of ωCgTX. The test currents and the accompanying tail currents were blocked progressively within two to three minutes after application of wCgTX, but both recovered partially as the wCgTX was flushed from the bath.

### b. Analysis of oöcytes injected with transcripts encoding the human neuronal calcium channel α_{1D} or transcripts encoding an α_{1D} and other subunits

The contribution of the α₂ and β₁ subunits to the inward barium current in oöcytes injected with transcripts encoding the α_{1D}, α₂ and β₁ subunits was assessed by expression of the α_{1D} subunit alone or in combination with either the β₁ subunit or the α₂ subunit. In oöcytes injected with only the transcript of a α_{1D} cDNA, no Ba²⁺ currents were detected (n = 3). In oöcytes injected with transcripts of α_{1D} and β₁ encoding DNA, small (108 ± 39 nA) Ba²⁺ currents were detected upon depolarization of the membrane from a holding potential of -90 mV that resembled the currents observed in cells injected with transcripts of α_{1D}, α₂ and β₁ encoding DNA, although the magnitude of the current was less. In two of the four oöcytes injected with transcripts of the α_{1D}-encoding and β₁-encoding DNA, the Ba²⁺ currents exhibited a sensitivity to Bay K 8644 that was similar to the Bay K 8644 sensitivity of Ba²⁺ currents expressed in oöcytes injected with transcripts encoding the α_{1D} α₁-, *α*₂. and β₁ subunits.

Three of five oöcytes injected with transcripts encoding the α_{1D} and α₂ subunits exhibited very small Ba²⁺ currents (15-30 nA) upon depolarization of the membrane from a holding potential of -90 mV. These barium currents showed little or no response to Bay K 8644.

### c. Analysis of oöcytes injected with transcripts encoding the human neuronal calcium channel α₂ and/or β₁ subunit

To evaluate the contribution of the α_{1D} α₁-subunit to the inward barium currents detected in oöcytes co-injected with transcripts encoding the α_{1D}, α₂ and β₁ subunits, oöcytes injected with transcripts encoding the human neuronal calcium channel α₂ and/or β₁ subunits were assayed for barium currents. Oöcytes injected with transcripts encoding the α₂ subunit displayed no detectable inward barium currents (n = 5). Oöcytes injected with transcripts encoding a β₁ subunit displayed measurable (54 ± 23 nA, n = 5) inward barium currents upon depolarization and oöcytes injected with transcripts encoding the α₂ and β₁ subunits displayed inward barium currents that were approximately 50% larger (80 ± 61 nA, n = 18) than those detected in oöcytes injected with transcripts of the β₁₋encoding DNA only.

The inward barium currents in oöcytes injected with transcripts encoding the β₁ subunit or α₂ and β₁ subunits typically were first observed when the membrane was depolarized to -30 mV from a holding potential of -90 mV and peaked when the membrane was depolarized to 10 to 20 mV. Macroscopically, the currents in oöcytes injected with transcripts encoding the α₂ and β₁ subunits or with transcripts encoding the β₁ subunit were indistinguishable. In contrast to the currents in oöcytes co-injected with transcripts of α_{1D}, α₂ and β₁ subunit encoding DNA, these currents showed a significant inactivation during the test pulse and a strong sensitivity to the holding potential. The inward barium currents in oöcytes co-injected with transcripts encoding the α₂ and β₁ subunits usually inactivated to 10-60% of the peak magnitude during a 140-msec pulse and were significantly more sensitive to holding potential than those in oöcytes co-injected with transcripts encoding the α_{1D}, α₂ and β₁ subunits. Changing the holding potential of the membranes of oöcytes co-injected with transcripts encoding the α₂ and β₁ subunits from -90 to -50 mV resulted in an approximately 81 % (n = 11) reduction in the magnitude of the inward barium current of these cells. In contrast, the inward barium current measured in oöcytes co-injected with transcripts encoding the α_{1D}, α₂ and β₁ subunits were reduced approximately 24% (n = 11) when the holding potential was changed from -90 to -50 mV.

The inward barium currents detected in oöcytes injected with transcripts encoding the α₂ and β₁ subunits were pharmacologically distinct from those observed in oöcytes co-injected with transcripts encoding the α_{1D}, α₂ and β₁ subunits. Oöcytes injected with transcripts encoding the α₂ and β₁ subunits displayed inward barium currents that were insensitive to Bay K 8644 (n = 11). Nifedipine sensitivity was difficult to measure because of the holding potential sensitivity of nifedipine and the current observed in oöcytes injected with transcripts encoding the α₂ and β₁ subunits. Nevertheless, two oöcytes that were co-injected with transcripts encoding the α₂ and β₁ subunits displayed measurable (25 to 45 nA) inward barium currents that were insensitive to nifedipine (5 to 10 µM), when depolarized from a holding potential of -50 mV. The inward barium currents in oöcytes injected with transcripts encoding the α₂ and β₁ subunits showed the same sensitivity to heavy metals as the currents detected in oöcytes injected with transcripts encoding the α_{1D}, α₂ and β₁ subunits.

The inward barium current detected in oöcytes injected with transcripts encoding the human neuronal α₂ and β₁ subunits has pharmacological and biophysical properties that resemble calcium currents in uninjected *Xenopus* oöcytes. Because the amino acids of this human neuronal calcium channel β₁ subunit lack hydrophobic segments capable of forming transmembrane domains. It is unlikely that recombinant β₁ subunits alone form an ion channel, but rather that an endogenous α₁ subunit exists in oöcytes and that the activity mediated by such an α₁ subunit is enhanced by expression of a human neuronal β₁ subunit.

While the subject matter of the invention has been described with some specificity, modifications apparent to those with ordinary skill in the art may be made without departing from the scope of the invention. Since such modifications will be apparent to those of skill in the art, it is intended that this invention be limited only by the scope of the appended claims.

## Claims

1. An isolated nucleic acid fragment that encodes a low-voltage activated α_{1H} subunit of an animal calcium channel wherein the sequence of nucleotides comprises the coding portion of the sequence of nucleotides set forth in any of SEQ ID Nos. 12-16.

2. The nucleic acid of claim 1 which encodes an α_{1H-1} or α_{1H-2} subunit which comprises the coding portion of the sequence of nucleotides set forth in any of SEQ ID Nos. 12, 15 or 16.

3. A eukaryotic cell, comprising heterologous nucleic acid that encodes an α_{1H}-subunit, wherein the α_{1H}-subunit is encoded by the nucleic acid of claim 1 or 2.

4. The cell of claim 3, further comprising heterologous nucleic acid that encodes a α₂δ-subunit of a calcium channel.

5. The eukaryotic cell of claim 3 or claim 4 that has a functional heterologous calcium channel that contains at least one subunit encoded by the heterologous nucleic acid of claims 1 or 2.

6. The eukaryotic cell of any of claims 3-5 selected from the group consisting of HEK 293 cells, Chinese hamster ovary cells, African green monkey cells, and mouse L cells.

7. The eukaryotic cell of any of claims 3-5 that is an amphibian oöcyte.

8. The eukaryotic cell of claim 5, wherein the heterologous calcium channel comprises a plurality of α_{1H}-subunits encoded by the coding portion of the sequence of nucleotides set forth in any of SEQ ID Nos. 12-16.

9. The eukaryotic cell of claim 8, wherein the α_{1H}-subunits comprise a homomer.

10. The eukaryotic cell of any one of claims 3-9, wherein the heterologous nucleic acid encodes a T-type calcium channel.

11. A method of identifying a compound that modulates the activity of a calcium channel that contains an α_{1H} subunit, comprising;
suspending a eukaryotic cell of any of claims 3-10 containing a functional calcium channel containing an α_{1H} subunit in a solution containing the compound and a calcium channel selective ion:
depolarizing the cell membrane of the cell; and
detecting the current or ions flowing into the cell,
wherein:
the current that is detected is different from that produced by depolarizing the same cell prior to the addition of the compound or an identical test cell preparation run in parallel to which the compound is not later added, in the presence of the same calcium channel selective ion and comparing, if necessary, with a control.

12. The method of claim 11, wherein prior to the depolarization step the cell is maintained at a holding potential which substantially inactivates calcium channels that are endogenous to the cell.

13. The method of claim 12, wherein:
the cell is an amphibian oöcyte;
the heterologous subunits are encoded by nucleic acid injected into the oöcyte; and
the heterologous subunits include an α_{1H}-subunit encoded by the nucleic acid of claim 1 or 2.

14. The method of claims 11 or 12, wherein the cell in an HEK cell and the heterologous subunit is encoded by the heterologous nucleic acid of claim 1 or 2.

15. A substantially pure α_{1H}-subunit encoded by the nucleic acid molecule of claim 1 or 2.

16. A method for identifying nucleic acids that encode a α_{1H}-subunit of a calcium channel which is encoded by the coding portion of the sequence of nucleotides set forth in any of SEQ ID Nos. 12-16, comprising hybridizing under conditions of at least low stringency (1.0 x SSPE, 0.1% SDS, 50°C) an RNA or DNA probe of at least 16 bases in length, comprising at least 16 contiguous nucleic acid bases from a sequence of nucleotides that encodes an α_{1H}-subunit of a calcium channel which is encoded by the coding portion of the sequence of nucleotides set forth in any of SEQ ID Nos. 12-16.

17. The method of claim 16 wherein the probe contains at least 30 contiguous nucleic acid bases that encode the α_{1H}-subunit of a calcium channel which is encoded by the nucleic acid of claim 1 or 2.

18. The method of claim 16 or 17, wherein the hybridization is effected under conditions of high stringency (0.1 SSPE, 0.1% SDS, 65°C).

19. A method for identifying cells or tissues that express a calcium channel α_{1H} subunit-encoding nucleic acid which α_{1H}-subunit is encoded by the coding portion of the sequence of nucleotides set forth in any of SEQ ID Nos. 12-16, comprising hybridizing under conditions of at least low stringency (1.0 SSPE, 0.1 % SDS, 50°C) a probe as defined in claim 16 or 17 with mRNA expressed in the cells or tissues or cDNA produced from the mRNA, and thereby identifying cells or tissue that express mRNA that encodes the subunit.

20. The method of claim 19, wherein hybridization is effected under conditions of high stringency (0.1 SSPE, 0.1% SDS, 65°C).

21. An isolated protein molecule, comprising the sequence of amino acids encoded by nucleotides 1506 to 2627 of SEQ ID No. 12.

22. The isolated nucleic acid molecule comprising the sequence of nucleotides set forth in nucleotides 1506 to 2627 of SEQ ID No. 12.

23. The nucleic acid of claim 1, 2 or 22 that is RNA.

24. The nucleic acid of 1, 2 or 22 that is DNA.

25. The cell of any one of claims 3-10, further comprising nucleic acid that encodes a reporter gene construct containing a reporter gene in operative linkage with one or more transcriptional control elements that is regulated by a calcium channel.

26. A method of identifying compounds that modulate the activity of a low-voltage activated calcium channel, the method comprising:
comparing the difference in the amount of transcription of the reporter gene in the cell of claim 25 in the presence of the compound with the amount of transcription in the absence of the compound, or with the amount of transcription in the absence of the heterologous calcium channel, whereby compounds that modulate the activity of the heterologous calcium channel in the cell are identified.

27. A screening assay for identifying a compound that modulates the activity of a low-voltage activated (LVA) calcium channel comprising the steps of:
contacting the test compound with a functional cell of any one of claims 3 to 10; and
measuring the activity of the LVA calcium channel in the cell before and after the addition of the test compound or in a comparable cell that does not express the LVA calcium channel; and
determining that the test compound modulates the activity of the LVA calcium channel if the measurement after compound addition is different from the measurement before the compound addition or if the measurement in the presence of the LVA calcium channel is different from the measurement in the absence of the LVA calcium channel

28. The method of claim 27, wherein the cell expresses a low-voltage calcium channel having a relative conductance of Ba²+ of about 5 pS to about 9 pS, an activation time of about 2 to about 8 milliseconds, a kinetics of activation V_{1/2} value of about -60 millivolts to about 26 millivolts, an inactivation time of about 10 to about 30 milliseconds, a kinetics of inactivation V_{1/2} value of about -100 millivolts to about -500 millivolts, and a tail deactivation time of about 2 to about 12 milliseconds.

## Patentansprüche

1. Isoliertes Nukleinsäurefragment, welches für eine durch niedrige Spannung aktivierte α_{1H}-Untereinheit eines tierischen Calciumkanals codiert, wobei die Nukleotidsequenz den codierenden Anteil der in irgendeiner der SEQ-Nrn. 12-16 angegebenen Nukleotidsequenz umfasst.

2. Nukleinsäure nach Anspruch 1, welche für eine oder α_{1H-2}-Untereinheit codiert, die den codierenden Anteil der in irgendeiner der SEQ-Nrn. 12, 15 oder 16 angegebenen Nukleotidsequenz umfasst.

3. Eukaryotische Zelle, umfassend heterologe Nukleinsäure, die für eine α_{1H}-Untereinheit codiert, wobei die α_{1H}-Untereinheit von der Nukleinsäure nach Anspruch 1 oder 2 codiert wird.

4. Zelle nach Anspruch 3, ferner umfassend heterologe Nukleinsäure, die für eine α₂δ-Untereinheit eines Calciumkanals codiert.

5. Eukaryotische Zelle nach Anspruch 3 oder Anspruch 4, welche einen funktionellen heterologen Calciumkanal aufweist, der mindestens eine Untereinheit enthält, welche von der heterologen Nukleinsäure nach Anspruch 1 oder 2 codiert wird.

6. Eukaryotische Zelle nach irgendeinem der Ansprüche 3-5, ausgewählt aus der Gruppe, die aus HEK 293-Zellen, Eierstockzellen des Chinesischen Hamsters, Zellen der Afrikanischen Grünen Meerkatze und Maus-L-Zellen besteht.

7. Eukaryotische Zelle nach irgendeinem der Ansprüche 3-5, die ein Amphibien-Oozyt ist.

8. Eukaryotische Zelle nach Anspruch 5, wobei der heterologe Calciumkanal eine Mehrzahl von α_{1H}-Untereinheiten umfasst, die von dem codierenden Anteil der in irgendeiner der SEQ-Nrn. 12-16 angegebenen Nukleotidsequenz codiert werden.

9. Eukaryotische Zelle nach Anspruch 8, wobei die α_{1H}-Untereinheiten ein Homomer umfassen.

10. Eukaryotische Zelle nach irgendeinem der Ansprüche 3-9, wobei die heterologe Nukleinsäure für einen Calciumkanal vom T-Typ codiert.

11. Verfahren zur Identifizierung einer Verbindung, welche die Aktivität eines Calciumkanals moduliert, der eine α_{1H}-Untereinheit enthält, umfassend
Suspendieren einer eukaryotischen Zelle nach irgendeinem der Ansprüche 3-10, die einen funktionellen Calciumkanal enthält, der eine α_{1H}-Untereinheit enthält, in einer Lösung, welche die Verbindung und ein calciumkanal-selektives lon enthält; Depolarisieren der Zellmembran der Zelle; und
Detektieren des Stroms oder der Ionen, welche(r) in die Zelle fließt/en,
wobei der detektierte Strom sich von demjenigen unterscheidet, welcher durch Depolarisieren derselben Zelle vor der Zugabe der Verbindung oder einer parall getesteten identischen Testzellpräparation, zu der die Verbindung später nicht hinzugefügt wird, in Gegenwart desselben calciumkanal-selektiven Ions erzeugt wird,
und, erforderlichenfalls,
Vergleichen mit einer Kontrolle.

12. Verfahren nach Anspruch 11, wobei vor dem Depolarisierungsschritt die Zelle bei einem Haltepotential gehalten wird, welches im Wesentlichen Calciumkanäle inaktiviert, die für die Zelle endogen sind.

13. Verfahren nach Anspruch 12, wobei
die Zelle ein Amphibien-Oozyt ist;
die heterologen Untereinheiten von Nukleinsäure codiert werden, die in den Oozyten injiziert wurde; und
die heterologen Untereinheiten eine α_{1H}-Untereinheit einschließen, die von der Nukleinsäure nach Anspruch 1 oder 2 codiert wird.

14. Verfahren nach Anspruch 11 oder 12, wobei die Zelle eine HEK-Zelle ist und die heterologe Untereinheit von der heterologen Nukleinsäure nach Anspruch 1 oder 2 codiert wird.

15. Im Wesentlichen reine α_{1H}-Untereinheit, die von dem Nukleinsäuremolekül nach Anspruch 1 oder 2 codiert wird.

16. Verfahren zur Identifizierung von Nukleinsäuren, die für eine α_{1H}-Untereinheit eines Calciumkanals, welche von dem codierenden Anteil der in irgendeiner der SEQ-Nrn. 12-16 angegebenen Nukleotidsequenz codiert wird, codieren, umfassend das Hybridisieren einer RNA- oder DNA-Sonde von mindestens 16 Basen Länge, umfassend mindestens 16 aufeinanderfolgende Nukleinsäurebasen von einer Nukleotidsequenz, die für eine α_{1H}-Untereinheit eines Calciumkanals codiert, welche von dem codierenden Anteil der in irgendeiner der SEQ-Nrn. 12-16 angegebenen Nukleotidsequenz codiert wird, unter Bedingungen von mindestens niedriger Stringenz (1,0 x SSPE, 0,1 % SDS, 50 °C).

17. Verfahren nach Anspruch 16, wobei die Sonde mindestens 30 aufeinanderfolgende Nukleinsäurebasen, welche für die α_{1H}-Untereinheit eines Calciumkanals, die von der Nukleinsäure nach Anspruch 1 oder 2 codiert wird, codieren, umfasst.

18. Verfahren nach Anspruch 16 oder 17, wobei die Hybridisierung unter Bedingungen hoher Stringenz (0,1 x SSPE, 0,1 % SDS, 65 °C) durchgeführt wird.

19. Verfahren zur Identifizierung von Zellen oder Geweben, welche eine für eine α_{1H-}Untereinheit eines Calciumkanals codierende Nukleinsäure exprimieren, wobei die α_{1H}-Untereinheit von dem codierenden Anteil der in irgendeiner der SEQ-Nrn. 12-16 angegebenen Nukleotidsequenz codiert wird, umfassend das Hybridisieren einer Sonde wie in Anspruch 16 oder 17 definiert mit mRNA, die in den Zellen oder Geweben exprimiert wurde, oder mit cDNA, die von der mRNA hergestellt wurde, unter Bedingungen von mindestens niedriger Stringenz (1,0 x SSPE, 0,1 % SDS, 50 °C), wodurch Zellen oder Gewebe, die mRNA exprimieren, welche für die Untereinheit codiert, identifiziert werden.

20. Verfahren nach Anspruch 19, wobei die Hybridisierung unter Bedingungen hoher Stringenz (0,1 x SSPE, 0,1 % SDS, 65°C) durchgeführt wird.

21. Isoliertes Proteinmolekül, umfassend die Sequenz von Aminosäuren, die von den Nukleotiden 1506 bis 2627 von SEQ-ID-Nr. 12 codiert wird.

22. Isoliertes Nukleinsäuremolekül, umfassend die Sequenz von Nukleotiden, die in den Nukleotiden 1506 bis 2627 von SEQ-ID-Nr. 12 angegeben wird.

23. Nukleinsäure nach Anspruch 1, 2 oder 22, die RNA ist.

24. Nukleinsäure nach Anspruch 1, 2 oder 22, die DNA ist.

25. Zelle nach irgendeinem der Ansprüche 3-10, welche ferner Nukleinsäure umfasst, die für ein Reportergenkonstrukt, enthaltend ein Reportergen in funktionsfähiger Verknüpfung mit einem oder mehreren transkriptionalen Kontrollelement(en), reguliert von einem Calciumkanal, codiert.

26. Verfahren zur Identifizierung von Verbindungen, welche die Aktivität eines durch niedrige Spannung aktivierten Calciumkanals modulieren, welches Verfahren umfasst:
Vergleichen des Unterschieds im Ausmaß der Transkription des Reportergens in der Zelle nach Anspruch 25 in Anwesenheit der Verbindung mit dem Ausmaß der Transkription in Abwesenheit der Verbindung oder mit dem Ausmaß der Transkription in Abwesenheit des heterologen Calciumkanals, wodurch Verbindungen identifiziert werden, welche die Aktivität des heterologen Calciumkanals in der Zelle modulieren.

27. Screening-Assay zur Identifizierung einer Verbindung, welche die Aktivität eines durch niedrige Spannung aktivierten (LVA) Calciumkanals moduliert, umfassend die Schritte:
Kontaktieren der Testverbindung mit einer funktionellen Zelle nach irgendeinem der Ansprüche 3 bis 10; und
Messen der Aktivität des LVA-Calciumkanals in der Zelle vor und nach der Zugabe der Testverbindung oder in einer vergleichbaren Zelle, welche den LVA-Calciumkanal nicht exprimiert; und
Feststellen, dass die Testverbindung die Aktivität des LVA-Calciumkanals moduliert, wenn sich die Messung nach der Zugabe der Verbindung von der Messung vor der Zugabe der Verbindung unterscheidet oder sich die Messung in Anwesenheit des LVA-Calciumkanals von der Messung in Abwesenheit des LVA-Calciumkanals unterscheidet.

28. Verfahren nach Anspruch 27, wobei die Zelle einen Niederspannungs-Calciumkanal mit einer relativen Leitfähigkeit für Ba²⁺ von etwa 5 pS bis etwa 9 pS, einer Aktivierungszeit von etwa 2 bis etwa 8 Millisekunden, einem Aktivierungskinetik-V_{1/2-}Wert von etwa - 60 Millivolt bis etwa 26 Millivolt, einer Inaktivierungszeit von etwa 10 bis etwa 30 Millisekunden, einem Inaktivierungskinetik-V_{1/2}-Wert von etwa -100 Millivolt bis etwa - 500 Millivolt und einer Tail-Deaktivierungszeit von etwa 2 bis etwa 12 Millisekunden exprimiert.

## Revendications

1. Fragment d'acide nucléique isolé qui code une sous-unité α1H activée à basse tension d'un canal calcique animal, dans lequel la séquence de nucléotides comprend la partie codante de la séquence de nucléotides présentée dans l'une quelconque parmi les SEQ ID n° 12 à 16.

2. Acide nucléique selon la revendication 1, qui code une sous-unité α1H-1 ou α1H-2 qui comprend la partie codante de la séquence de nucléotides présentée dans l'une quelconque parmi les SEQ ID n° 12, 15 et 16.

3. Cellule eucaryote comprenant un acide nucléique hétérologue qui code une sous-unité α1H, dans laquelle la sous-unité α1H est codée par l'acide nucléique selon la revendication 1 ou 2.

4. Cellule selon la revendication 3, comprenant en outre un acide nucléique hétérologue qui code une sous-unité α2δ d'un canal calcique.

5. Cellule eucaryote selon la revendication 3 ou la revendication 4, qui a un canal calcique hétérologue fonctionnel qui contient au moins une sous-unité codée par l'acide nucléique hétérologue selon la revendication 1 ou 2.

6. Cellule eucaryote selon l'une quelconque des revendications 3 à 5, choisie dans le groupe constitué par les cellules HEK 293, les cellules d'ovaire de hamster chinois, les cellules de singe vert africain et les cellules L de souris.

7. Cellule eucaryote selon l'une quelconque des revendications 3 à 5, qui est un ovocyte amphibien.

8. Cellule eucaryote selon la revendication 5, dans laquelle le canal calcique hétérologue comprend une pluralité de sous-unités α1H codées par la partie codante de la séquence de nucléotides présentée dans l'une quelconque parmi les SEQ ID n° 12 à 16.

9. Cellule eucaryote selon la revendication 8, dans laquelle les sous-unités α1H comprennent un homomère.

10. Cellule eucaryote selon l'une quelconque des revendications 3 à 9, dans laquelle l'acide nucléique hétérologue code un canal calcique de type T.

11. Procédé d'identification d'un composé qui module l'activité d'un canal calcique qui contient une sous-unité α1H, consistant à :
mettre en suspension une cellule eucaryote selon l'une quelconque des revendications 3 à 10 contenant un canal calcique fonctionnel contenant une sous-unité α1H dans une solution contenant le composé et un ion sélectif du canal calcique ;
dépolariser la membrane cellulaire de la cellule ; et
détecter le courant ou les ions s'écoulant dans la cellule,
dans lequel :
le courant qui est détecté est différent de celui produit par la dépolarisation de la même cellule, avant l'addition du composé ou la préparation d'une cellule test identique effectuée en parallèle à laquelle le composé n'est pas ajouté ultérieurement, en présence du même ion sélectif de canal calcique et en comparant, si nécessaire, avec un témoin.

12. Procédé selon la revendication 11, dans lequel avant l'étape de dépolarisation, la cellule est maintenue à un potentiel de maintien qui inactive sensiblement les canaux calciques endogènes à la cellule.

13. Procédé selon la revendication 12, dans lequel:
la cellule est un ovocyte amphibien ;
les sous-unités hétérologues sont codées par un acide nucléique injecté dans l'ovocyte ; et
les sous-unités hétérologues comprennent une sous-unité α1H codée par l'acide nucléique selon la revendication 1 ou 2.

14. Procédé selon la revendication 11 ou 12, dans lequel la cellule est une cellule HEK et la sous-unité hétérologue est codée par l'acide nucléique hétérologue selon la revendication 1 ou 2.

15. Sous-unité α1H essentiellement pure codée par la molécule d'acide nucléique selon la revendication 1 ou 2.

16. Procédé d'identification d'acides nucléiques qui codent une sous-unité α1H d'un canal calcique qui est codée par la partie codante de la séquence de nucléotides présentée dans l'une quelconque parmi les SEQ ID n° 12 à 16, comprenant l'hybridation dans des conditions de stringence au moins faible (1,0 x SSPE, 0,1 % SDS, 50°C) d'une sonde d'ARN ou d'ADN d'au moins 16 bases de long, comprenant au moins 16 bases d'acide nucléique contiguës d'une séquence de nucléotides qui code une sous-unité α_{1H} d'un canal calcique qui est codée par la partie codante de la séquence de nucléotides présentée selon l'une quelconque parmi les SEQ ID n° 12 à 16.

17. Procédé selon la revendication 16, dans lequel la sonde contient au moins 30 bases d'acide nucléique contiguës qui codent la sous-unité α1H d'un canal calcique qui est codée par l'acide nucléique selon la revendication 1 ou 2.

18. Procédé selon la revendication 16 ou 17, dans lequel l'hybridation est effectuée dans des conditions de forte stringence (0,1 SSPE, 0,1 % SDS, 65°C).

19. Procédé d'identification de cellules ou de tissus qui expriment un acide nucléique codant la sous-unité α1H d'un canal calcique, laquelle sous-unité α1H est codée par la partie codante de la séquence de nucléotides présentée dans l'une quelconque parmi les SEQ ID n° 12 à 16, comprenant l'hybridation dans des conditions de stringence au moins faible (1,0 SSPE, 0,1 % SDS, 50°C) d'une sonde telle que définie selon la revendication 16 ou 17 avec l'ARNm exprimé dans les cellules ou les tissus ou l'ADNc produit à partir de l'ARNm, et ainsi l'identification des cellules ou du tissu qui expriment l'ARNm qui code la sous-unité.

20. Procédé selon la revendication 19, dans lequel l'hybridation est effectuée dans des conditions de stringence forte (0,1 SSPE, 0,1 % SDS, 65°C).

21. Molécule protéique isolée, comprenant la séquence d'acides aminés codée par les nucléotides 1506 à 2627 de SEQ ID n° 12.

22. Molécule d'acide nucléique isolée, comprenant la séquence de nucléotides présentée dans les nucléotides 1506 à 2627 de SEQ ID n° 12.

23. Acide nucléique selon la revendication 1, 2 ou 22, qui est un ARN.

24. Acide nucléique selon la revendication 1, 2 ou 22, qui et un ADN.

25. Cellule selon l'une quelconque des revendications 3 à 10, comprenant en outre un acide nucléique qui code une construction de gène rapporteur contenant un gène rapporteur en liaison opérationnelle avec un ou plusieurs éléments de contrôle transcriptionnel qui est régulé par un canal calcique.

26. Procédé d'identification de composés qui modulent l'activité d'un canal calcique activé à basse tension, le procédé consistant à :
comparer la différence de quantité de transcription du gène rapporteur dans la cellule selon la revendication 25 en présence du composé ave la quantité de transcription en l'absence du composé, ou avec la quantité de transcription en l'absence du canal calcique hétérologue, moyennant quoi les composés qui modulent l'activité du canal calcique hétérologue dans la cellule sont identifiés.

27. Test de criblage pour identifier un composé qui module l'activité d'un canal calcique activé à basse tension (LVA) comprenant les étapes de :
mettre en contact le composé test avec une cellule fonctionnelle selon l'une quelconque des revendications 3 à 10 ; et
mesurer l'activité du canal calcique LVA dans la cellule avant et après l'addition du composé test ou dans une cellule comparable qui n'exprime pas le canal calcique LVA ; et
déterminer que le composé test module l'activité du canal calcique LVA si la mesure après l'addition du composé est différente de la mesure avant l'addition du composé ou si la mesure en présence du canal calcique LVA est différente de la mesure en l'absence du canal calcique LVA.

28. Procédé selon la revendication 27, dans lequel la cellule exprime un canal calcique basse tension ayant une conductance relative du Ba²⁺ d'environ 5 à environ 9 pS, un temps d'activation d'environ 2 à environ 8 millisecondes, une valeur V_{1/2} de cinétique d'activation d'environ - 60 à environ 26 millivolts, un temps d'inactivation d'environ 10 à environ 30 millisecondes, une valeur V_{1/2} de cinétique d'inactivation d'environ - 100 à environ - 500 millivolts, et un temps de désactivation de queue d'environ 2 à environ 12 millisecondes.


### SEQUENCE LISTING

(1) GENERAL INFORMATION:
   (i) APPLICANT:
      (A) NAME: SIBIA Neurosciences, Inc.
      (B) STREET: 505 Coast Boulevard South, Suite 300
      (C) CITY: La Jolla
      (D) STATE: California
      (E) COUNTRY: US
      (F) POSTAL CODE (ZIP): 92037-4641
   (i) INVENTOR/APPLICANT:
      (A) NAME: Mark E. Williams
      (B) STREET: 946 Jasmine Court
      (C) CITY: Carlsbad
      (D) STATE: California
      (E) COUNTRY: USA
      (F) POSTAL CODE (ZIP): 92009
   (i) INVENTOR/APPLICANT:
      (A) NAME: Kenneth A. Stauderman
      (B) STREET: 3615 Lotus Dr.
      (C) CITY: San Diego
      (D) STATE: California
      (E) COUNTRY: USA
      (F) POSTAL CODE (ZIP): 92106
   (i) INVENTOR/APPLICANT:
      (A) NAME: Michael M. Harpold
      (B) STREET: 1462 Encina Road
      (C) CITY: Sante Fe
      (D) STATE: New Mexico
      (E) COUNTRY: USA
      (F) POSTAL CODE (ZIP): 87505-4726
   (i) INVENTOR/APPLICANT:
      (A) NAME: Michael Hans
      (B) STREET:2635 Clemente Terrace
      (C) CITY: San Diego
      (D) STATE: California
      (E) COUNTRY: USA
      (F) POSTAL CODE (ZIP): 92122
   (i) INVENTOR/APPLICANT:
      (A) NAME: Arturo Urrutia
      (B) STREET: 778 Beech Avenue
      (C) CITY: Chula Vista
      (D) STATE: California
      (E) COUNTRY: USA
      (F) POSTAL CODE (ZIP): 91910

      (A) NAME: Mark S. Washburn
      (B) STREET: 1535 Kings Cross Drive
      (C) CITY: Cardiff
      (D) STATE: California
      (E) COUNTRY: USA
      (F) POSTAL CODE (ZIP): 92007
   (ii) TITLE OF INVENTION: CALCIUM CHANNEL COMPOSITIONS AND METHODS
   (iii) NUMBER OF SEQUENCES: 16
   (iv) CORRESPONDENCE ADDRESS:
      (A) ADDRESSEE:Heller Ehrman White & McAuliffe.
      (B) STREET: 4250 Executive Square, 7th Floor
      (C) CITY: La Jolla
      (D) STATE: California
      (E) COUNTRY: US
      (F) ZIP: 92037
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ Version 1.5 and Patentin 2.0
   (vi) CURRENT APPLICATION DATA:
      (A) APPLICATION NUMBER:
      (B) FILING DATE: 03-DEC-1998
      (C) CLASSIFICATION:
   (vii) PRIOR APPLICATION DATA:
      (A) APPLICATION NUMBER: 09/188,932
      (B) FILING DATE: 10-NOV-1998
      (C) CLASSIFICATION:
   (vii) PRIOR APPLICATION DATA:
      (A) APPLICATION NUMBER: 08/984,709
      (B) FILING DATE: 03-DEC-1997
      (C) CLASSIFICATION:
   (viii) ATTORNEY/AGENT INFORMATION:
      (A) NAME: Seidman, Stephanie L.
      (B) REGISTRATION NUMBER: 33,779
      (C) REFERENCE/DOCKET NUMBER: 24735-9815PC
   (ix) TELECOMMUNICATION INFORMATION:
      (A) TELEPHONE: (619) 450-8400
      (B) TELEFAX: (619) 450-8499
(2) INFORMATION FOR SEQ ID NO:1:
   (i) SEQUENCE CHARACTERISTICS:
      (A) LENGTH: 22 base pairs
      (B) TYPE: nucleic acid
      (C) STRANDEDNESS: single
      (D) TOPOLOGY: unknown
   (i) INVENTOR/APPLICANT:
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:1:
      TYCCCTTGAA GAGCTGNACC CC 22
(2) INFORMATION FOR SEQ ID NO:2:
   (i) SEQUENCE CHARACTERISTICS:
      (A) LENGTH: 17 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:2:
      CGTGCACGTC ACGCTAG 17
(2) INFORMATION FOR SEQ ID NO:3:
   (i) SEQUENCE CHARACTERISTICS:
      (A) LENGTH: 21 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:3:
      AATTCTAGCG TGACGTGCAC G 21
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:4:
      ACNGTGTTYC AGATCCTGAC 2
(2) INFORMATION FOR SEQ ID NO:5:
   (i) SEQUENCE CHARACTERISTICS:
      (A) LENGTH: 23 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:5:
      ATCCTGACNG GNGARGACTG GAA 23
(2) INFORMATION FOR SEQ ID NO:6:
   (i) SEQUENCE CHARACTERISTICS:
      (A) LENGTH: 22 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:6:
      TYCCCTTGAA GAGCTGNACN GC 22
(2) INFORMATION FOR SEQ ID NO:7:
   (i) SEQUENCE CHARACTERISTICS:
      (A) LENGTH: 22 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:7:
      TYCCCTTGA AGAGCTGNAC CCC 22
(2) INFORMATION FOR SEQ ID NO:8:
   (i) SEQUENCE CHARACTERISTICS:
      (A) LENGTH: 17 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:8:
      AACTGYATYA CCCTGGC 17
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:9:
      ATYACCCTGG CNATGGAGCG 20
(2) INFORMATION FOR SEQ ID NO:10:
   (i) SEQUENCE CHARACTERISTICS:
      (A) LENGTH: 17 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:10:
      GARATGATGA TGAARGT 17
(2) INFORMATION FOR SEQ ID NO:11:
   (i) SEQUENCE CHARACTERISTICS:
      (A) LENGTH: 342 base pairs
      (B) TYPE: nucleic acid
      (C) STRANDEDNESS: single
      (D) TOPOLOGY: unknown
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:11:
(2) INFORMATION FOR SEQ ID NO : 12 :
   (i) SEQUENCE CHARACTERISTICS:
      (A) LENGTH: 7898 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (ix) FEATURE:
      (A) NAME/KEY: Coding Sequence
      (B) LOCATION: 249...7307
      (D) OTHER INFORMATION:
   (xi) SEQUENCE DESCRIPTION: SEQ ID NO:12:
   CGAGGCCGCC GCCGTCGCCT CCGCCGGGCG AGCCGGAGCC GGAGTCGAGC CGCGGCCGGG      60
   AGCCGGGCGG GCTGGGGACG CGGGCCGGGG GCGGAGGCGC TGGGGGCCGG GGCCGGGGCC     120
   GGGGGCGGAG GCGCTGGGGG CCGGGGCCGG GGCCGGGCGC CGAGCGGGGT CCGCGGTGAC     180
   CGCGCCGCCC GGGCGATGCC CGCGGGGACG CCGCCGGCCA GCAGAGCGAG GTGCTGCCGG     240
   CCGCCACC ATG ACC GAG GGC GCA CGG GCC GCC GAC GAG GTC CGG GTG CCC     290
            Met Thr Glu Gly Ala Arg Ala Ala Asp Glu Val Arg Val Pro
             1               5                  10
   CTG GGC GCG CCG CCC CCT GGC CCT GCG GCG TTG GTG GGG GCG TCC CCG      338
   Leu Gly Ala Pro Pro Pro Gly Pro Ala Ala Leu Val Gly Ala Ser Pro
   15                  20                  25                  30
   GAG AGC CCC GGG GCG CCG GGA CGC GAG GCG GAG CGG GGG TCC GAG CTC      386
   Glu Ser Pro Gly Ala Pro Gly Arg Glu Ala Glu Arg Gly Ser Glu Leu
                    35                  40                  45
   GGC GTG TCA CCC TCC GAG AGC CCG GCG GCC GAG CGC GGC GCG GAG CTG      434
   Gly Val Ser Pro Ser Glu Ser Pro Ala Ala Glu Arg Gly Ala Glu Leu
                50                  55                  60
   GGT GCC GAC GAG GAG CAG CGC GTC CCG TAC CCG GCC TTG GCG GCC ACG      482
   Gly Ala Asp Glu Glu Gln Arg Val Pro Tyr Pro Ala Leu Ala Ala Thr
            65                  70                  75
   GTC TTC TTC TGC CTC GGT CAG ACC ACG CGG CCG CGC AGC TGG TGC CTC      530
   Val Phe Phe Cys Leu Gly Gln Thr Thr Arg Pro Arg Ser Trp Cys Leu
        80                  85                  90
   CGG CTG GTC TGC AAC CCA TGG TTC GAG CAC GTG AGC ATG CTG GTA ATC      578
   Arg Leu Val Cys Asn Pro Trp Phe Glu His Val Ser Met Leu Val Ile
    95                 100                 105                 110
   ATG CTC AAC TGC GTG ACC CTG GGC ATG TTC CGG CCC TGT GAG GAC GTT      626
   Met Leu Asn Cys Val Thr Leu Gly Met Phe Arg Pro Cys Glu Asp Val
                   115                 120                 125
   GAG TGC GGC TCC GAG CGC TGC AAC ATC CTG GAG GCC TTT GAC GCC TTC      674
   Glu Cys Gly Ser Glu Arg Cys Asn Ile Leu Glu Ala Phe Asp Ala Phe
               130                 135                 140
   ATT TTC GCC TTT TTT GCG GTG GAG ATG GTC ATC AAG ATG GTG GCC TTG      722
   Ile Phe Ala Phe Phe Ala Val Glu Met Val Ile Lys Met Val Ala Leu
           145                 150                 155
   GGG CTG TTC GGG CAG AAG TGT TAC CTG GGT GAC ACG TGG AAC AGG CTG      770
   Gly Leu Phe Gly Gln Lys Cys Tyr Leu Gly Asp Thr Trp Asn Arg Leu
       160                 165                 170
   GAT TTC TTC ATC GTC GTG GCG GGC ATG ATG GAG TAC TCG TTG GAC GGA      818
   Asp Phe Phe Ile Val Val Ala Gly Met Met Glu Tyr Ser Leu Asp Gly
   175                 180                 185                 190
   CAC AAC GTG AGC CTC TCG GCT ATC AGG ACC GTG CGG GTG CTG CGG CCC      866
   His Asn Val Ser Leu Ser Ala Ile Arg Thr Val Arg Val Leu Arg Pro
                   195                 200                 205
   CTC CGC GCC ATC AAC CGC GTG CCT AGC ATG CGG ATC CTG GTC ACT CTG      914
   Leu Arg Ala Ile Asn Arg Val Pro Ser Met Arg Ile Leu Val Thr Leu
               210                 215                 220
   CTG CTG GAT ACG CTG CCC ATG CTC GGG AAC GTC CTT CTG CTG TGC TTC      962
   Leu Leu Asp Thr Leu Pro Met Leu Gly Asn Val Leu Leu Leu Cys Phe
           225                 230                 235
   TTC GTC TTC TTC ATT TTC GGC ATC GTT GGC GTC CAG CTC TGG GCT GGC     1010
   Phe Val Phe Phe Ile Phe Gly Ile Val Gly Val Gln Leu Trp Ala Gly
       240                 245                 250
   CTC CTG CGG AAC CGC TGC TTC CTG GAC AGT GCC TTT GTC AGG AAC AAC     1058
   Leu Leu Arg Asn Arg Cys Phe Leu Asp Ser Ala Phe Val Arg Asn Asn
   255                 260                 265                 270
   AAC CTG ACC TTC CTG CGG CCG TAC TAC CAG ACG GAG GAG GGC GAG GAG      1106
   Asn Leu Thr Phe Leu Arg Pro Tyr Tyr Gln Thr Glu Glu Gly Glu Glu
                   275                 280                 285
   AAC CCG TTC ATC TGC TCC TCA CGC CGA GAC AAC GGC ATG CAG AAG TGC      1154
   Asn Pro Phe Ile Cys Ser Ser Arg Arg Asp Asn Gly Met Gln Lys Cys
               290                 295                 300
   TCG CAC ATC CCC GGC CGC CGC GAG CTG CGC ATG CCC TGC ACC CTG GGC      1202
   Ser His Ile Pro Gly Arg Arg Glu Leu Arg Met Pro Cys Thr Leu Gly
           305                 310                 315
   TGG GAG GCC TAC ACG CAG CCG CAG GCC GAG GGG GTG GGC GCT GCA CGC      1250
   Trp Glu Ala Tyr Thr Gln Pro Gln Ala Glu Gly Val Gly Ala Ala Arg
       320                 325                 330
   AAC GCC TGC ATC AAC TGG AAC CAG TAC TAC AAC GTG TGC CGC TCG GGT      1298
   Asn Ala Cys Ile Asn Trp Asn Gln Tyr Tyr Asn Val Cys Arg Ser Gly
   335                 340                 345                 350
   GAC TCC AAC CCC CAC AAC GGT GCC ATC AAC TTC GAC AAC ATC GGC TAC      1346
   Asp Ser Asn Pro His Asn Gly Ala Ile Asn Phe Asp Asn Ile Gly Tyr
                   355                 360                 365
   GCC TGG ATT GCC ATC TTC CAG GTG ATC ACG CTG GAA GGC TGG GTG GAC      1394
   Ala Trp Ile Ala Ile Phe Gln Val Ile Thr Leu Glu Gly Trp Val Asp
               370                 375                 380
   ATC ATG TAC TAC GTC ATG GAC GCC CAC TCA TTC TAC AAC TTC ATC TAT      1442
   Ile Met Tyr Tyr Val Met Asp Ala His Ser Phe Tyr Asn Phe Ile Tyr
           385                 390                 395
   TTC ATC CTG CTC ATC ATC GTG GGC TCC TTC TTC ATG ATC AAC CTG TGC      1490
   Phe Ile Leu Leu Ile Ile Val Gly Ser Phe Phe Met Ile Asn Leu Cys
       400                 405                 410
   CTG GTG GTG ATT GCC ACG CAG TTC TCG GAG ACG AAG CAG CGG GAG AGT      1538
   Leu Val Val Ile Ala Thr Gln Phe Ser Glu Thr Lys Gln Arg Glu Ser
   415                 420                 425                 430
   CAG CTG ATG CGG GAG CAG CGG GCA CGC CAC CTG TCC AAC GAC AGC ACG      1586
   Gln Leu Met Arg Glu Gln Arg Ala Arg His Leu Ser Asn Asp Ser Thr
                   435                 440                 445
   CTG GCC AGC TTC TCC GAG CCT GGC AGC TGC TAC GAA GAG CTG CTG AAG      1634
   Leu Ala Ser Phe Ser Glu Pro Gly Ser Cys Tyr Glu Glu Leu Leu Lys
               450                 455                 460
   TAC GTG GGC CAC ATA TTC CGC AAG GTC AAG CGG CGC AGC TTG CGC CTC      1682
   Tyr Val Gly His Ile Phe Arg Lys Val Lys Arg Arg Ser Leu Arg Leu
           465                 470                 475
   TAC GCC CGC TGG CAG AGC CGC TGG CGC AAG AAG GTG GAC CCC AGT GCT      1730
   Tyr Ala Arg Trp Gln Ser Arg Trp Arg Lys Lys Val Asp Pro Ser Ala
       480                 485                 490
   GTG CAA GGC CAG GGT CCC GGG CAC CGC CAG CGC CGG GCA GGC AGG CAC    1778
   Val Gln Gly Gln Gly Pro Gly His Arg Gln Arg Arg Ala Gly Arg His
   495                 500                 505                 510
   ACA GCC TCG GTG CAC CAC CTG GTC TAC CAC CAC CAT CAC CAC CAC CAC    1826
   Thr Ala Ser Val His His Leu Val Tyr His His His His His His His
                   515                 520                 525
   CAC CAC TAC CAT TTC AGC CAT GGC AGC CCC CGC AGG CCC GGC CCC GAG    1874
   His His Tyr His Phe Ser His Gly Ser Pro Arg Arg Pro Gly Pro Glu
               530                 535                 540
   CCA GGC GCC TGC GAC ACC AGG CTG GTC CGA GCT GGC GCG CCC CCC TCG    1922
   Pro Gly Ala Cys Asp Thr Arg Leu Val Arg Ala Gly Ala Pro Pro Ser
           545                 550                 555
   CCA CCT TCC CCA GGC CGC GGA CCC CCC GAC GCA GAG TCT GTG CAC AGC    1970
   Pro Pro Ser Pro Gly Arg Gly Pro Pro Asp Ala Glu Ser Val His Ser
       560                 565                 570
   ATC TAC CAT GCC GAC TGC CAC ATA GAG GGG CCG CAG GAG AGG GCC CGG    2018
   Ile Tyr His Ala Asp Cys His Ile Glu Gly Pro Gln Glu Arg Ala Arg
   575                 580                 585                 590
   GTG GCA CAT GCC GCA GCC ACT GCC GCT GCC AGC CTC AGG CTG GCC ACA    2066
   Val Ala His Ala Ala Ala Thr Ala Ala Ala Ser Leu Arg Leu Ala Thr
                   595                 600                 605
   GGG CTG GGC ACC ATG AAC TAC CCC ACG ATC CTG CCC TCA GGG GTG GGC    2114
   Gly Leu Gly Thr Met Asn Tyr Pro Thr Ile Leu Pro Ser Gly Val Gly
               610                 615                 620
   AGC GGC AAA GGC AGC ACC AGC CCC GGA CCC AAG GGG AAG TGG GCC GGT    2162
   Ser Gly Lys Gly Ser Thr Ser Pro Gly Pro Lys Gly Lys Trp Ala Gly
           625                 630                 635
   GGA CCG CCA GGC ACC GGG GGG CAC GGC CCG TTG AGC TTG AAC AGC CCT    2210
   Gly Pro Pro Gly Thr Gly Gly His Gly Pro Leu Ser Leu Asn Ser Pro
       640                 645                 650
   GAT CCC TAC GAG AAG ATC CCG CAT GTG GTC GGG GAG CAT GGA CTG GGC    2258
   Asp Pro Tyr Glu Lys Ile Pro His Val Val Gly Glu His Gly Leu Gly
   655                 660                 665                 670
   CAG GCC CCT GGC CAT CTG TCG GGC CTC AGT GTG CCC TGC CCC CTG CCC    2306
   Gln Ala Pro Gly His Leu Ser Gly Leu Ser Val Pro Cys Pro Leu Pro
                   675                 680                 685
   AGC CCC CCA GCG GGC ACA CTG ACC TGT GAG CTG AAG AGC TGC CCG TAC    2354
   Ser Pro Pro Ala Gly Thr Leu Thr Cys Glu Leu Lys Ser Cys Pro Tyr
               690                 695                 700
   TGC ACC CGT GCC CTG GAG GAC CCG GAG GGT GAG CTC AGC GGC TCG GAA    2402
   Cys Thr Arg Ala Leu Glu Asp Pro Glu Gly Glu Leu Ser Gly Ser Glu
           705                 710                 715
   AGT GGA GAC TCA GAT GGC CGT GGC GTC TAT GAA TTC ACG CAG GAC GTC    2450
   Ser Gly Asp Ser Asp Gly Arg Gly Val Tyr Glu Phe Thr Gln Asp Val
       720                     725                     730
   CGG CAC GGT GAC CGC TGG GAC CCC ACG CGA CCA CCC CGT GCG ACG GAC     2498
   Arg His Gly Asp Arg Trp Asp Pro Thr Arg Pro Pro Arg Ala Thr Asp
   735                 740                 745                 750
   ACA CCA GGC CCA GGC CCA GGC AGC CCC CAG CGG CGG GCA CAG CAG AGG     2546
   Thr Pro Gly Pro Gly Pro Gly Ser Pro Gln Arg Arg Ala Gln Gln Arg
                   755                 760                 765
   GCA GCC CCG GGC GAG CCA GGC TGG ATG GGC CGC CTC TGG GTT ACC TTC     2594
   Ala Ala Pro Gly Glu Pro Gly Trp Met Gly Arg Leu Trp Val Thr Phe
               770                 775                 780
   AGC GGC AAG CTG CGC CGC ATC GTG GAC AGC AAG TAC TTC AGC CGT GGC     2642
   Ser Gly Lys Leu Arg Arg Ile Val Asp Ser Lys Tyr Phe Ser Arg Gly
           785                 790                 795
   ATC ATG ATG GCC ATC CTT GTC AAC ACG CTG AGC ATG GGC GTG GAG TAC     2690
   Ile Met Met Ala Ile Leu Val Asn Thr Leu Ser Met Gly Val Glu Tyr
       800                 805                 810
   CAT GAG CAG CCC GAG GAG CTG ACT AAT GCT CTG GAG ATC AGC AAC ATC     2738
   His Glu Gln Pro Glu Glu Leu Thr Asn Ala Leu Glu Ile Ser Asn Ile
   815                 820                 825                 830
   GTG TTC ACC AGC ATG TTT GCC CTG GAG ATG CTG CTG AAG CTG CTG GCC     2786
   Val Phe Thr Ser Met Phe Ala Leu Glu Met Leu Leu Lys Leu Leu Ala
                   835                 840                 845
   TGC GGC CCT CTG GGC TAC ATC CGG AAC CCG TAC AAC ATC TTC GAC GGC     2834
   Cys Gly Pro Leu Gly Tyr Ile Arg Asn Pro Tyr Asn Ile Phe Asp Gly
               850                 855                 860
   ATC ATC GTG GTC ATC AGC GTC TGG GAG ATC GTG GGG CAG GCG GAC GGT     2882
   Ile Ile Val Val Ile Ser Val Trp Glu Ile Val Gly Gln Ala Asp Gly
           865                 870                 875
   GGC TTG TCT GTG CTG CGC ACC TTC CGG CTG CTG CGT GTG CTG AAG CTG     2930
   Gly Leu Ser Val Leu Arg Thr Phe Arg Leu Leu Arg Val Leu Lys Leu
       880                 885                 890
   GTG CGC TTT CTG CCA GCC CTG CGG CGC CAG CTC GTG GTG CTG GTG AAG     2978
   Val Arg Phe Leu Pro Ala Leu Arg Arg Gln Leu Val Val Leu Val Lys
   895                 900                 905                 910
   ACC ATG GAC AAC GTG GCT ACC TTC TGC ACG CTG CTC ATG CTC TTC ATT     3026
   Thr Met Asp Asn Val Ala Thr Phe Cys Thr Leu Leu Met Leu Phe Ile
                   915                 920                 925
   TTC ATC TTC AGC ATC CTG GGC ATG CAC CTT TTC GGC TGC AAG TTC AGC     3074
   Phe Ile Phe Ser Ile Leu Gly Met His Leu Phe Gly Cys Lys Phe Ser
               930                 935                 940
   CTG AAG ACA GAC ACC GGA GAC ACC GTG CCT GAC AGG AAG AAC TTC GAC     3122
   Leu Lys Thr Asp Thr Gly Asp Thr Val Pro Asp Arg Lys Asn Phe Asp
           945                 950                 955
   TCC CTG CTG TGG GCC ATC GTC ACC GTG TTC CAG ATC CTG ACC CAG GAG     3170
   Ser Leu Leu Trp Ala Ile Val Thr Val Phe Gln Ile Leu Thr Gln Glu
       960                 965                 970
   GAC TGG AAC GTG GTC CTG TAC AAC GGC ATG GCC TCC ACC TCC TCC TGG     3218
   Asp Trp Asn Val Val Leu Tyr Asn Gly Met Ala Ser Thr Ser Ser Trp
   975                 980                 985                 990
   GCC GCC CTC TAC TTC GTG GCC CTC ATG ACC TTC GGC AAC TAT GTG CTC     3266
   Ala Ala Leu Tyr Phe Val Ala Leu Met Thr Phe Gly Asn Tyr Val Leu
                   995                1000                1005
   TTC AAC CTG CTG GTG GCC ATC CTC GTG GAG GGC TTC CAG GCG GAG GGC     3314
   Phe Asn Leu Leu Val Ala Ile Leu Val Glu Gly Phe Gln Ala Glu Gly
              1010                1015                1020
   GAT GCC AAC AGA TCC GAC ACG GAC GAG GAC AAG ACG TCG GTC CAC TTC     3362
   Asp Ala Asn Arg Ser Asp Thr Asp Glu Asp Lys Thr Ser Val His Phe
          1025                1030                1035
   GAG GAG GAC TTC CAC AAG CTC AGA GAA CTC CAG ACC ACA GAG CTG AAG     3410
   Glu Glu Asp Phe His Lys Leu Arg Glu Leu Gln Thr Thr Glu Leu Lys
      1040                1045                1050
   ATG TGT TCC CTG GCC GTG ACC CCC AAC GGG CAC CTG GAG GGA CGA GGC     3458
   Met Cys Ser Leu Ala Val Thr Pro Asn Gly His Leu Glu Gly Arg Gly
   1055                1060                1065                1070
   AGC CTG TCC CCT CCC CTC ATC ATG TGC ACA GCT GCC ACG CCC ATG CCT     3506
   Ser Leu Ser Pro Pro Leu Ile Met Cys Thr Ala Ala Thr Pro Met Pro
                  1075                1080                1085
   ACC CCC AAG AGC TCA CCA TTC CTG GAT GCA GCC CCC AGC CTC CCA GAC     3554
   Thr Pro Lys Ser Ser Pro Phe Leu Asp Ala Ala Pro Ser Leu Pro Asp
              1090                1095                1100
   TCT CGG CGT GGC AGC AGC AGC TCC GGG GAC CCG CCA CTG GGA GAC CAG     3602
   Ser Arg Arg Gly Ser Ser Ser Ser Gly Asp Pro Pro Leu Gly Asp Gln
          1105                1110                1115
   AAG CCT CCG GCC AGC CTC CGA AGT TCT CCC TGT GCC CCC TGG GGC CCC     3650
   Lys Pro Pro Ala Ser Leu Arg Ser Ser Pro Cys Ala Pro Trp Gly Pro
      1120                1125                1130
   AGT GGC GCC TGG AGC AGC CGG CGC TCC AGC TGG AGC AGC CTG GGC CGT     3698
   Ser Gly Ala Trp Ser Ser Arg Arg Ser Ser Trp Ser Ser Leu Gly Arg
   1135                1140                1145                1150
   GCC CCC AGC CTC AAG CGC CGC GGC CAG TGT GGG GAA CGT GAG TCC CTG     3746
   Ala Pro Ser Leu Lys Arg Arg Gly Gln Cys Gly Glu Arg Glu Ser Leu
                  1155                1160                1165
   CTG TCT GGC GAG GGC AAG GGC AGC ACC GAC GAC GAA GCT GAG GAC GGC     3794
   Leu Ser Gly Glu Gly Lys Gly Ser Thr Asp Asp Glu Ala Glu Asp Gly
              1170                1175                1180
   AGG GCC GCG CCC GGG CCC CGT GCC ACC CCA CTG CGG CGG GCC GAG TCC     3842
   Arg Ala Ala Pro Gly Pro Arg Ala Thr Pro Leu Arg Arg Ala Glu Ser
         1185                1190                1195
   CTG GAC CCA CGG CCC CTG CGG CCG GCC GCC CTC CCG CCT ACC AAG TGC     3890
   Leu Asp Pro Arg Pro Leu Arg Pro Ala Ala Leu Pro Pro Thr Lys Cys
       1200                1205                1210
   CGC GAT CGC GAC GGG CAG GTG GTG GCC CTG CCC AGC GAC TTC TTC CTG     3938
   Arg Asp Arg Asp Gly Gln Val Val Ala Leu Pro Ser Asp Phe Phe Leu
   1215                1220                1225                1230
   CGC ATC GAC AGC CAC CGT GAG GAT GCA GCC GAG CTT GAC GAC GAC TCG     3986
   Arg Ile Asp Ser His Arg Glu Asp Ala Ala Glu Leu Asp Asp Asp Ser
                   1235                1240                1245
   GAG GAC AGC TGC TGC CTC CGC CTG CAT AAA GTG CTG GAG CCC TAC AAG     4034
   Glu Asp Ser Cys Cys Leu Arg Leu His Lys Val Leu Glu Pro Tyr Lys
               1250                1255                1260
   CCC CAG TGG TGC CGG AGC CGC GAG GCC TGG GCC CTC TAC CTC TTC TCC     4082
   Pro Gln Trp Cys Arg Ser Arg Glu Ala Trp Ala Leu Tyr Leu Phe Ser
           1265                1270                1275
   CCA CAG AAC CGG TTC CGC GTC TCC TGC CAG AAG GTC ATC ACA CAC AAG     4130
   Pro Gln Asn Arg Phe Arg Val Ser Cys Gln Lys Val Ile Thr His Lys
       1280                1285                1290
   ATG TTT GAT CAC GTG GTC CTC GTC TTC ATC TTC CTC AAC TGC GTC ACC     4178
   Met Phe Asp His Val Val Leu Val Phe Ile Phe Leu Asn Cys Val Thr
   1295                1300                1305                1310
   ATC GCC CTG GAG AGG CCT GAC ATT GAC CCC GGC AGC ACC GAG CGG GTC     4226
   Ile Ala Leu Glu Arg Pro Asp Ile Asp Pro Gly Ser Thr Glu Arg Val
                   1315                1320                1325
   TTC CTC AGC GTC TCC AAT TAC ATC TTC ACG GCC ATC TTC GTG GCG GAG     4274
   Phe Leu Ser Val Ser Asn Tyr Ile Phe Thr Ala Ile Phe Val Ala Glu
               1330                1335                1340
   ATG ATG GTG AAG GTG GTG GCC CTG GGG CTG CTG TCC GGC GAG CAC GCC     4322
   Met Met Val Lys Val Val Ala Leu Gly Leu Leu Ser Gly Glu His Ala
           1345                1350                1355
   TAC CTG CAG AGC AGC TGG AAC CTG CTG GAT GGG CTG CTG GTG CTG GTG     4370
   Tyr Leu Gln Ser Ser Trp Asn Leu Leu Asp Gly Leu Leu Val Leu Val
       1360                1365                1370
   TCC CTG GTG GAC ATT GTC GTG GCC ATG GCC TCG GCT GGT GGC GCC AAG     4418
   Ser Leu Val Asp Ile Val Val Ala Met Ala Ser Ala Gly Gly Ala Lys
   1375                1380                1385                1390
   ATC CTG GGT GTT CTG CGC GTG CTG CGT CTG CTG CGG ACC CTG CGG CCT     4466
   Ile Leu Gly Val Leu Arg Val Leu Arg Leu Leu Arg Thr Leu Arg Pro
                   1395                1400                1405
   CTA AGG GTC ATC AGC CGG GCC CCG GGC CTC AAG CTG GTG GTG GAG ACG     4514
   Leu Arg Val Ile Ser Arg Ala Pro Gly Leu Lys Leu Val Val Glu Thr
               1410                1415                1420
   CTG ATA TCG TCG CTC AGG CCC ATT GGG AAC ATC GTC CTC ATC TGC TGC    4562
   Leu Ile Ser Ser Leu Arg Pro Ile Gly Asn Ile Val Leu Ile Cys Cys
           1425                1430                1435
   GCC TTC TTC ATC ATT TTT GGC ATC TTG GGT GTG CAG CTC TTC AAA GGG    4610
   Ala Phe Phe Ile Ile Phe Gly Ile Leu Gly Val Gln Leu Phe Lys Gly
       1440                1445                1450
   AAG TTC TAC TAC TGC GAG GGC CCC GAC ACC AGG AAC ATC TCC ACC AAG    4658
   Lys Phe Tyr Tyr Cys Glu Gly Pro Asp Thr Arg Asn Ile Ser Thr Lys
   1455                1460                1465                1470
   GCA CAG TGC CGG GCC GCC CAC TAC CGC TGG GTG CGA CGC AAG TAC AAC    4706
   Ala Gln Cys Arg Ala Ala His Tyr Arg Trp Val Arg Arg Lys Tyr Asn
                   1475                1480                1485
   TTC GAC AAC CTG GGC CAG GCC CTG ATG TCG CTG TTC GTG CTG TCA TCC    4754
   Phe Asp Asn Leu Gly Gln Ala Leu Met Ser Leu Phe Val Leu Ser Ser
               1490                1495                1500
   AAG GAT GGA TGG GTG AAC ATC ATG TAC GAC GGG CTG GAT GCC GTG GGT    4802
   Lys Asp Gly Trp Val Asn Ile Met Tyr Asp Gly Leu Asp Ala Val Gly
           1505                1510                1515
   GTC GAC CAG CAG CCT GTG CAG AAC CAC AAC CCC TGG ATG CTG CTG TAC    4850
   Val Asp Gln Gln Pro Val Gln Asn His Asn Pro Trp Met Leu Leu Tyr
       1520                1525                1530
   TTC ATC TCC TTC CTG CTC ATC GTC AGC TTC TTC GTG CTC AAC ATG TTC    4898
   Phe Ile Ser Phe Leu Leu Ile Val Ser Phe Phe Val Leu Asn Met Phe
   1535                1540                1545                1550
   GTG GGC GTC GTG GTC GAG AAC TTC CAC AAG TGC CGG CAG CAC CAG GAG    4946
   Val Gly Val Val Val Glu Asn Phe His Lys Cys Arg Gln His Gln Glu
                   1555                1560                1565
   GCG GAG GAG GCG CGG CGG CGA GAG GAG AAG CGG CTG CGG CGC CTA GAG    4994
   Ala Glu Glu Ala Arg Arg Arg Glu Glu Lys Arg Leu Arg Arg Leu Glu
               1570                1575                1580
   AGG AGG CGC AGG AGC ACT TTC CCC AGC CCA GAG GCC CAG CGC CGG CCC    5042
   Arg Arg Arg Arg Ser Thr Phe Pro Ser Pro Glu Ala Gln Arg Arg Pro
           1585                1590                1595
   TAC TAT GCC GAC TAC TCG CCC ACG CGC CGC TCC ATT CAC TCG CTG TGC    5090
   Tyr Tyr Ala Asp Tyr Ser Pro Thr Arg Arg Ser Ile His Ser Leu Cys
       1600                1605                1610
   ACC AGC CAC TAT CTC GAC CTC TTC ATC ACC TTC ATC ATC TGT GTC AAC    5138
   Thr Ser His Tyr Leu Asp Leu Phe Ile Thr Phe Ile Ile Cys Val Asn
   1615                1620                1625                1630
   GTC ATC ACC ATG TCC ATG GAG CAC TAT AAC CAA CCC AAG TCG CTG GAC    5186
   Val Ile Thr Met Ser Met Glu His Tyr Asn Gln Pro Lys Ser Leu Asp
                   1635                1640                1645
   GAG GCC CTC AAG TAC TGC AAC TAC GTC TTC ACC ATC GTG TTT GTC TTC    5234
   Glu Ala Leu Lys Tyr Cys Asn Tyr Val Phe Thr Ile Val Phe Val Phe
                   1650                 1655                 1660
   GAG GCT GCA CTG AAG CTG GTA GCA TTT GGG TTC CGT CGG TTC TTC AAG     5282
   Glu Ala Ala Leu Lys Leu Val Ala Phe Gly Phe Arg Arg Phe Phe Lys
           1665                 1670                 1675
   GAC AGG TGG AAC CAG CTG GAC CTG GCC ATC GTG CTG CTG TCA CTC ATG     5330
   Asp Arg Trp Asn Gln Leu Asp Leu Ala Ile Val Leu Leu Ser Leu Met
       1680                 1685                 1690
   GGC ATC ACG CTG GAG GAG ATA GAG ATG AGC GCC GCG CTG CCC ATC AAC     5378
   Gly Ile Thr Leu Glu Glu Ile Glu Met Ser Ala Ala Leu Pro Ile Asn
   1695                 1700                 1705                 1710
   CCC ACC ATC ATC CGC ATC ATG CGC GTG CTT CGC ATT GCC CGT GTG CTG     5426
   Pro Thr Ile Ile Arg Ile Met Arg Val Leu Arg Ile Ala Arg Val Leu
                   1715                 1720                 1725
   AAG CTG CTG AAG ATG GCT ACG GGC ATG CGC GCC CTG CTG GAC ACT GTG     5474
   Lys Leu Leu Lys Met Ala Thr Gly Met Arg Ala Leu Leu Asp Thr Val
               1730                 1735                 1740
   GTG CAA GCT CTC CCC CAG GTG GGG AAC CTG GGC CTT CTT TTC ATG CTC     5522
   Val Gln Ala Leu Pro Gln Val Gly Asn Leu Gly Leu Leu Phe Met Leu
           1745                 1750                 1755
   CTG TTT TTT ATC TAT GCT GCG CTG GGA GTG GAG CTG TTC GGG AGG CTG     5570
   Leu Phe Phe Ile Tyr Ala Ala Leu Gly Val Glu Leu Phe Gly Arg Leu
       1760                 1765                 1770
   GAG TGC AGT GAA GAC AAC CCC TGC GAG GGC CTG AGC AGG CAC GCC ACC     5618
   Glu Cys Ser Glu Asp Asn Pro Cys Glu Gly Leu Ser Arg His Ala Thr
   1775                 1780                 1785                 1790
   TTC AGC AAC TTC GGC ATG GCC TTC CTC ACG CTG TTC CGC GTG TCC ACG     5666
   Phe Ser Asn Phe Gly Met Ala Phe Leu Thr Leu Phe Arg Val Ser Thr
                   1795                 1800                 1805
   GGG GAC AAC TGG AAC GGG ATC ATG AAG GAC ACG CTG CGC GAG TGC TCC     5714
   Gly Asp Asn Trp Asn Gly Ile Met Lys Asp Thr Leu Arg Glu Cys Ser
               1810                 1815                 1820
   CGT GAG GAC AAG CAC TGC CTG AGC TAC CTG CCG GCC CTG TCG CCC GTC     5762
   Arg Glu Asp Lys His Cys Leu Ser Tyr Leu Pro Ala Leu Ser Pro Val
           1825                 1830                 1835
   TAC TTC GTG ACC TTC GTG CTG GTG GCC CAG TTC GTG CTG GTG AAC GTG     5810
   Tyr Phe Val Thr Phe Val Leu Val Ala Gln Phe Val Leu Val Asn Val
       1840                 1845                 1850
   GTG GTG GCC GTG CTC ATG AAG CAC CTG GAG GAG AGC AAC AAG GAG GCA     5858
   Val Val Ala Val Leu Met Lys His Leu Glu Glu Ser Asn Lys Glu Ala
   1855                 1860                 1865                 1870
   CGG GAG GAT GCG GAG CTG GAC GCC GAG ATC GAG CTG GAG ATG GCG CAG     5906
   Arg Glu Asp Ala Glu Leu Asp Ala Glu Ile Glu Leu Glu Met Ala Gln
                   1875                 1880                 1885
   GGC CCC GGG AGT GCA CGC CGG GTG GAC GCG GAC AGG CCT CCC TTG CCC    5954
   Gly Pro Gly Ser Ala Arg Arg Val Asp Ala Asp Arg Pro Pro Leu Pro
               1890                1895                1900
   CAG GAG AGT CCG GGC GCC AGG GAT GCC CCA AAC CTG GTT GCA CGC AAG    6002
   Gln Glu Ser Pro Gly Ala Arg Asp Ala Pro Asn Leu Val Ala Arg Lys
           1905                1910                1915
   GTG TCC GTG TCC AGG ATG CTC TCG CTG CCC AAC GAC AGC TAC ATG TTC    6050
   Val Ser Val Ser Arg Met Leu Ser Leu Pro Asn Asp Ser Tyr Met Phe
       1920                1925                1930
   AGG CCC GTG GTG CCT GCC TCG GCG CCC CAC CCC CGC CCG CTG CAG GAG    6098
   Arg Pro Val Val Pro Ala Ser Ala Pro His Pro Arg Pro Leu Gln Glu
   1935                1940                1945                1950
   GTG GAG ATG GAG ACC TAT GGG GCC GGC ACC CCC TTG GGC TCC GTT GCC    6146
   Val Glu Met Glu Thr Tyr Gly Ala Gly Thr Pro Leu Gly Ser Val Ala
                   1955                1960                1965
   TCT GTG CAC TCT CCG CCC GCA GAG TCC TGT GCC TCC CTC CAG ATC CCA    6194
   Ser Val His Ser Pro Pro Ala Glu Ser Cys Ala Ser Leu Gln Ile Pro
               1970                1975                1980
   CTG GCT GTG TCG TCC CCA GCC AGG AGC GGC GAG CCC CTC CAC GCC CTG    6242
   Leu Ala Val Ser Ser Pro Ala Arg Ser Gly Glu Pro Leu His Ala Leu
           1985                1990                1995
   TCC CCT CGG GGC ACA GCC CGC TCC CCC AGT CTC AGC CGG CTG CTC TGC    6290
   Ser Pro Arg Gly Thr Ala Arg Ser Pro Ser Leu Ser Arg Leu Leu Cys
       2000                2005                2010
   AGA CAG GAG GCT GTG CAC ACC GAT TCC TTG GAA GGG AAG ATT GAC AGC    6338
   Arg Gln Glu Ala Val His Thr Asp Ser Leu Glu Gly Lys Ile Asp Ser
   2015                2020                2025                2030
   CCT AGG GAC ACC CTG GAT CCT GCA GAG CCT GGT GAG AAA ACC CCG GTG    6386
   Pro Arg Asp Thr Leu Asp Pro Ala Glu Pro Gly Glu Lys Thr Pro Val
                   2035                2040                2045
   AGG CCG GTG ACC CAG GGG GGC TCC CTG CAG TCC CCA CCA CGC TCC CCA    6434
   Arg Pro Val Thr Gln Gly Gly Ser Leu Gln Ser Pro Pro Arg Ser Pro
               2050                2055                2060
   CGG CCC GCC AGC GTC CGC ACT CGT AAG CAT ACC TTC GGA CAG CAC TGC    6482
   Arg Pro Ala Ser Val Arg Thr Arg Lys His Thr Phe Gly Gln His Cys
           2065                2070                2075
   GTC TCC AGC CGG CCG GCG GCC CCA GGC GGA GAG GAG GCC GAG GCC TCG    6530
   Val Ser Ser Arg Pro Ala Ala Pro Gly Gly Glu Glu Ala Glu Ala Ser
       2080                2085                2090
   GAC CCA GCC GAC GAG GAG GTC AGC CAC ATC ACC AGC TCC GCC TGC CCC    6578
   Asp Pro Ala Asp Glu Glu Val Ser His Ile Thr Ser Ser Ala Cys Pro
   2095                2100                2105                2110
   TGG CAG CCC ACA GCC GAG CCC CAT GGC CCC GAA GCC TCT CCG GTG GCC    6626
   Trp Gln Pro Thr Ala Glu Pro His Gly Pro Glu Ala Ser Pro Val Ala
             2115                2120                2125
   GGC GGC GAG CGG GAC CTG CGC AGG CTC TAC AGC GTG GAC GCT CAG GGC      6674
   Gly Gly Glu Arg Asp Leu Arg Arg Leu Tyr Ser Val Asp Ala Gln Gly
               2130                2135                2140
   TTC CTG GAC AAG CCG GGC CGG GCA GAC GAG CAG TGG CGG CCC TCG GCG      6722
   Phe Leu Asp Lys Pro Gly Arg Ala Asp Glu Gln Trp Arg Pro Ser Ala
           2145                2150                2155
   GAG CTG GGC AGC GGG GAG CCT GGG GAG GCG AAG GCC TGG GGC CCT GAG      6770
   Glu Leu Gly Ser Gly Glu Pro Gly Glu Ala Lys Ala Trp Gly Pro Glu
       2160                2165                2170
   GCC GAG CCC GCT CTG GGT GCG CGC AGA AAG AAG AAG ATG AGC CCC CCC      6818
   Ala Glu Pro Ala Leu Gly Ala Arg Arg Lys Lys Lys Met Ser Pro Pro
   2175                2180                2185                2190
   TGC ATC TCG GTG GAA CCC CCT GCG GAG GAC GAG GGC TCT GCG CGG CCC      6866
   Cys Ile Ser Val Glu Pro Pro Ala Glu Asp Glu Gly Ser Ala Arg Pro
                   2195                2200                2205
   TCC GCG GCA GAG GGC GGC AGC ACC ACA CTG AGG CGC AGG ACC CCG TCC      6914
   Ser Ala Ala Glu Gly Gly Ser Thr Thr Leu Arg Arg Arg Thr Pro Ser
               2210                2215                2220
   TGT GAG GCC ACG CCT CAC AGG GAA TCC CTG GAG CCC ACA GAG GGC TCA      6962
   Cys Glu Ala Thr Pro His Arg Glu Ser Leu Glu Pro Thr Glu Gly Ser
           2225                2230                2235
   GGC GCC GGG GGG GAC CCT GCA GCC AAG GGG GAG CGC TGG GGC CAG GCC      7010
   Gly Ala Gly Gly Asp Pro Ala Ala Lys Gly Glu Arg Trp Gly Gln Ala
       2240                2245                2250
   TCC TGC CGG GCT GAG CAC CTG ACC GTC CCC AGC TTT GCC TTT GAG CCG      7058
   Ser Cys Arg Ala Glu His Leu Thr Val Pro Ser Phe Ala Phe Glu Pro
   2255                2260                2265                2270
   CTG GAC CTC GGG GTC CCC AGT GGA GAC CCT TTT TTG GAC GGT AGC CAC      7106
   Leu Asp Leu Gly Val Pro Ser Gly Asp Pro Phe Leu Asp Gly Ser His
                   2275                2280                2285
   AGT GTG ACC CCA GAA TCC AGA GCT TCC TCT TCA GGG GCC ATA GTG CCC      7154
   Ser Val Thr Pro Glu Ser Arg Ala Ser Ser Ser Gly Ala Ile Val Pro
               2290                2295                2300
   CTG GAA CCC CCA GAA TCA GAG CCT CCC ATG CCC GTC GGT GAC CCC CCA      7202
   Leu Glu Pro Pro Glu Ser Glu Pro Pro Met Pro Val Gly Asp Pro Pro
           2305                2310                2315
   GAG AAG AGG CGG GGG CTG TAC CTC ACA GTC CCC CAG TGT CCT CTG GAG      7250
   Glu Lys Arg Arg Gly Leu Tyr Leu Thr Val Pro Gln Cys Pro Leu Glu
       2320                2325                2330
   AAA CCA GGG TCC CCC TCA GCC ACC CCT GCC CCA GGG GGT GGT GCA GAT      7298
   Lys Pro Gly Ser Pro Ser Ala Thr Pro Ala Pro Gly Gly Gly Ala Asp
   2335                2340                2345                2350
   GAC CCC GTG TAGCTCGGGG CTTGGTGCCG CCCACGGCTT TGGCCCTGGG GTCTGGGGGC
   7357
   Asp Pro Val
   CCCGCTGGGG TGGAGGCCCA GGCAGAACCC TGCATGGACC CTGACTTGGG TCCCGTCGTG 7417
   AGCAGAAAGG CCCGGGGAGG ATGACGGCCC AGGCCCTGGT TCTCTGCCCA GCGAAGCAGG 7477
   AGTAGCTGCC GGGCCCCACG AGCCTCCATC CGTTCTGGTT CGGGTTTCTC CGAGTTTTGC 7537
   TACCAGCCGA GGCTGTGCGG GCAACTGGGT CAGCCTCCCG TCAGGAGAGA AGCCGCGTCT 7597
   GTGGGACGAA GACCGGGCAC CCGCCAGAGA GGGGAAGGTA CCAGGTTGCG TCCTTTCAGG 7657
   CCCCGCGTTG TTACAGGACA CTCGCTGGGG GCCCTGTGCC CTTGCCGGCG GCAGGTTGCA 7717
   GCCACCGCGG CCCAATGTCA CCTTCACTCA CAGTCTGAGT TCTTGTCCGC CTGTCACGCC 7777
   CTCACCACCC TCCCCTTCCA GCCACCACCC TTTCCGTTCC GCTCGGGCCT TCCCAGAAGC 7837
   GTCCTGTGAC TCTGGGAGAG GTGACACCTC ACTAAGGGGC CGACCCCATG GAGTAACGCG 7897
   C 7898
(2) INFORMATION FOR SEQ ID NO:13:
   (i) SEQUENCE CHARACTERISTICS:
      (A) LENGTH: 1669 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:13:
   GTGCGGCTCC GAGCGCTGCA ACATCCTGGA GGCCTTTGAC GCCTTCATTT TCGCCTTTTT 60
   TGCGGTGGAG ATGGTCATCA AGATGGTGGC CTTGGGGCTG TTCGGGCAGA AGTGTTACCT 120
   GGGTGACACG TGGAACAGGC TGGATTTCTT CATCGTCGTG GCGGGCATGA TGGAGTACTC 180
   GTTGGACGGA CACAACGTGA GCCTCTCGGC TATCAGGACC GTGCGGGTGC TGCGGCCCCT 240
   CCGCGCCATC AACCGCGTGC CTAGCATGCG GATCCTGGTC ACTCTGCTGC TGGATACGCT 300
   GCCCATGCTC GGGAACGTCC TTCTGCTGTG CTTCTTCGTC TTCTTCATTT TCGGCATCGT 360
   TGGCGTCCAG CTCTGGGCTG GCCTCCTGCG GAACCGCTGC TTCCTGGACA GTGCCTTTGT 420
   CAGGAACAAC AACCTGACCT TCCTGCGGCC GTACTACCAG ACGGAGGAGG GCGAGGAGAA 480
   CCCGTTCATC TGCTCCTCAC GCCGAGACAA CGGCATGCAG AAGTGCTCGC ACATCCCCGG 540
   CCGCCGCGAG CTGCGCATGC CCTGCACCCT GGGCTGGGAG GCCTACACGC AGCCGCAGGC 600
   CGAGGGGGTG GGCGCTGCAC GCAACGCCTG CATCAACTGG AACCAGTACT ACAACGTGTG 660
   CCGCTCGGGT GACTCCAACC CCCACAACGG TGCCATCAAC TTCGACAACA TCGGCTACGC 720
   CTGGATTGCC ATCTTCCAGG TGATCACGCT GGAAGGCTGG GTGGACATCA TGTACTACGT 780
   CATGGACGCC CACTCATTCT ACAACTTCAT CTATTTCATC CTGCTCATCA TCGTGGGCTC 840
   CTTCTTCATG ATCAACCTGT GCCTGGTGGT GATTGCCACG CAGTTCTCGG AGACGAAGCA 900
   GCGGGAGAGT CAGCTGATGC GGGAGCAGCG GGCACGCCAC CTGTCCAACG ACAGCACGCT 960
   GGCCAGCTTC TCCGAGCCTG GCAGCTGCTA CGAAGAGCTG CCCGTACTGC ACCCGTGCCC 1020
   TGGAGGACCC GGAGGGTGAG CTCAGCGGCT CGGAAAGTGG AGACTCAGAT GGCCGTGGCG 1080
   TCTATGAATT CACGCAGGAC GTCCGGCACG GTGACCGCTG GGACCCCACG CGACCACCCC 1140
   GGGCGAGCCA GGCTGGATGG GCCGCCTCTG GGTTACCTTC AGCGGCAAGC TGCGCCGCAT 1200
   CGTGGACAGC AAGTACTTCA GCCGTGGCAT CATGATGGCC ATCCTTGTCA ACACGCTGAG 1260
   CATGGGCGTG GAGTACCATG AGCAGCCCGA GGAGCTGACT AATGCTCTGG AGATCAGCAA 1320
   CATCGTGTTC ACCAGCATGT TTGCCCTGGA GATGCTGCTG AAGCTGCTGG CCTGCGGCCC 1380
   TCTGGGCTAC ATCCGGAACC CGTACAACAT CTTCGACGGC ATCATCGTGG TCATCAGCGT 1440
   CTGGGAGATC GTGGGGCAGG CGGACGGTGG CTTGTCTGTG CTGCGCACCT TCCGGCTGCT 1500
   GCGTGTGCTG AAGCTGGTGC GCTTTCTGCC AGCCCTGCGG CGCCAGCTCG TGGTGCTGGT 1560
   GAAGACCATG GACAACGTGG CTACCTTCTG CACGCTGCTC ATGCTCTTCA TTTTCATCTT 1620
   CAGCATCCTG GGCATGCACC TTTTCGGCTG GCAAGTTCAG CCTGAAGAA 1669
(2) INFORMATION FOR SEQ ID NO:14:
   (i) SEQUENCE CHARACTERISTICS:
      (A) LENGTH: 1413 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (xi) SEQUENCE DESCRIPTION: SEQ ID NO:14:
   ACGGGCTCGA GGCTCGCTCG CTGCCTCACC GGTCCCCGGC CCGCGCCCCG CGCCCCGCGC 60
   CCCGCGCCCC GGCCTCACCC GTCCGCTCAG CGGCCTCCAC GCCGCGCCGA GGCCGCCGCC 120
   GTCGCCTCCG CCGGGCGAGC CGGAGCCGGA GTCGAGCCGC GGCCGGGAGC CGGGCGGGCT 180
   GGGGACGCGG GCCGGGGGCG GAGGCGCTGG GGGCCGGGGC CGGGGCCGGG CGCCGAGCGG 240
   GGTCCGCGGT GACCGCGCCG CCCGGGCGAT GCCCGCGGGG ACGCCGCCGG CCAGCAGAGC 300
   GAGGCATGCG GATCCTGGTC ACTCTGCTGC TGGATACGCT GCCCATGCTC GGGAACGTCC 360
   TTCTGCTGTG CTTCTTCGTC TTCTTCATTT TCGGCATCGT TGGCGTCCAG CTCTGGGCTG 420
   GCCTCCTGCG GAACCGCTGC TTCCTGGACA GTGCCTTTGT CAGGAACAAC AACCTGACCT 480
   TCCTGCGGCC GTACTACCAG ACGGAGGAGG GCGAGGAGAA CCCGTTCATC TGCTCCTCAC 540
   GCCGAGACAA CGGCATGCAG AAGTGCTCGC ACATCCCCGG CCGCCGCGAG CTGCGCATGC 600
   CCTGCACCCT GGGCTGGGAG GCCTACACGC AGCCGCAGGC CGAGGGGGTG GGCGCTGCAC 660
   GCAACGCCTG CATCAACTGG AACCAGTACT ACAACGTGTG CCGCTCGGGT GACTCCAACC 720
   CCCACAACGG TGCCATCAAC TTCGACAACA TCGGCTACGC CTGGATTGCC ATCTTCCAGG 780
   TGATCACGCT GGAAGGCTGG GTGGACATCA TGTACTACGT CATGGACGCC CACTCATTCT 840
   ACAACTTCAT CTATTTCATC CTGCTCATCA TCGTGGGCTC CTTCTTCATG ATCAACCTGT 900
   GCCTGGTGGT GATTGCCACG CAGTTCTCGG AGACGAAGCA GCGGGAGAGT CAGCTGATGC 960
   GGGAGCAGCG GGCACGCCAC CTGTCCAACG ACAGCACGCT GGCCAGCTTC TCCGAGCCTG 1020
   GCAGCTGCTA CGAAGAGCTG CTGAAGACTG GGCCAGGCCC CTGGCCATCT GTCGGGCCTC 1080
   AGTGTGCCCT GCCCCCTGCC CAGCCCCCCA GCGGGCACAC TGACCTGTGA GCTGAAGAGC 1140
   TGCCCGTACT GCACCCGTGC CCTGGAGGAC CCGGAGGGTG AGCTCAGCGG CTCGGAAAGT 1200
   GGAGACTCAG ATGGCCGTGG CGTCTATGAA TTCACGCAGG ACGTCCGGCA CGGTGACCGC 1260
   TGGGACCCCA CGCGACCACC CCGTGCGACG GACACACCAG GCCCAGGCCC AGGCAGCCCC 1320
   CAGCGGCGGG CACAGCAGAG GGCAGCCCCG GGCGAGCCAG GCTGGATGGG CCGCCTCTGG 1380
   GTTACTTCAG CGGCAAGCTG CGCGCATCGT GGA 1413
(2) INFORMATION FOR SEQ ID NO:15:
   (i) SEQUENCE CHARACTERISTICS:
      (A) LENGTH: 7898 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (ix) FEATURE:
      (A) NAME/KEY: Coding Sequence
      (B) LOCATION: 249...7307
      (D) OTHER INFORMATION: α_{1H-1}
   (xi) SEQUENCE DESCRIPTION: SEQ ID NO:15
(2) INFORMATION FOR SEQ ID NO:16:
   (i) SEQUENCE CHARACTERISTICS:
      (A) LENGTH: 6941 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTISENSE: NO
   (v) FRAGMENT TYPE:
   (vi) ORIGINAL SOURCE:
   (ix) FEATURE:
      (A) NAME/KEY: Coding Sequence
      (B) LOCATION: 249... 6353
      (D) OTHER INFORMATION: α_{1H-2}
   (xi) SEQUENCE DESCRIPTION: SEQ ID NO:16
   cgaggccgcc gccgtcgcct ccgccgggcg agccggagcc ggagtcgagc cgcggccggg 60
   agccgggcgg gctggggacg cgggccgggg gcggaggcgc tgggggccgg ggccggggcc 120
   gggggcggag gcgctggggg ccggggccgg ggccgggcgc cgagcggggt ccgcggtgac 180
   cgcgccgccc gggcgatgcc cgcggggacg ccgccggcca gcagagcgag gtgctgccgg 240
   ccgccacc atg acc gag ggc gca cgg gcc gcc gac gag gtc cgg gtg ccc   290
            Met Thr Glu Gly Ala Arg Ala Ala Asp Glu Val Arg Val Pro
             1               5                  10
   ctg ggc gcg ccg ccc cct ggc cct gcg gcg ttg gtg ggg gcg tcc ccg   338
   Leu Gly Ala Pro Pro Pro Gly Pro Ala Ala Leu Val Gly Ala Ser Pro
    15                  20                  25                  30
   gag agc ccc ggg gcg ccg gga cgc gag gcg gag cgg ggg tcc gag ctc   386
   Glu Ser Pro Gly Ala Pro Gly Arg Glu Ala Glu Arg Gly Ser Glu Leu
                    35                  40                  45
   ggc gtg tca ccc tcc gag agc ccg gcg gcc gag cgc ggc gcg gag ctg   434
   Gly Val Ser Pro Ser Glu Ser Pro Ala Ala Glu Arg Gly Ala Glu Leu
               50                  55                  60
   ggt gcc gac gag gag cag cgc gtc ccg tac ccg gcc ttg gcg gcc acg   482
   Gly Ala Asp Glu Glu Gln Arg Val Pro Tyr Pro Ala Leu Ala Ala Thr
           65                  70                  75
   gtc ttc ttc tgc ctc ggt cag acc acg cgg ccg cgc agc tgg tgc ctc   530
   Val Phe Phe Cys Leu Gly Gln Thr Thr Arg Pro Arg Ser Trp Cys Leu
       80                  85                  90
   cgg ctg gtc tgc aac cca tgg ttc gag cac gtg agc atg ctg gta atc   578
   Arg Leu Val Cys Asn Pro Trp Phe Glu His Val Ser Met Leu Val Ile
    95                 100                 105                 110
   atg ctc aac tgc gtg acc ctg ggc atg ttc cgg ccc tgt gag gac gtt   626
   Met Leu Asn Cys Val Thr Leu Gly Met Phe Arg Pro Cys Glu Asp Val
                   115                 120                 125
   gag tgc ggc tcc gag cgc tgc aac atc ctg gag gcc ttt gac gcc ttc   674
   Glu Cys Gly Ser Glu Arg Cys Asn Ile Leu Glu Ala Phe Asp Ala Phe
               130                 135                 140
   att ttc gcc ttt ttt gcg gtg gag atg gtc atc aag atg gtg gcc ttg   722
   Ile Phe Ala Phe Phe Ala Val Glu Met Val Ile Lys Met Val Ala Leu
           145                 150                 155
   ggg ctg ttc ggg cag aag tgt tac ctg ggt gac acg tgg aac agg ctg   770
   Gly Leu Phe Gly Gln Lys Cys Tyr Leu Gly Asp Thr Trp Asn Arg Leu
       160                 165                 170
   gat ttc ttc atc gtc gtg gcg ggc atg atg gag tac tcg ttg gac gga      818
   Asp Phe Phe Ile Val Val Ala Gly Met Met Glu Tyr Ser Leu Asp Gly
   175                 180                 185                 190
   cac aac gtg agc ctc tcg gct atc agg acc gtg cgg gtg ctg cgg ccc      866
   His Asn Val Ser Leu Ser Ala Ile Arg Thr Val Arg Val Leu Arg Pro
                   195                 200                 205
   ctc cgc gcc atc aac cgc gtg cct agc atg cgg atc ctg gtc act ctg      914
   Leu Arg Ala Ile Asn Arg Val Pro Ser Met Arg Ile Leu Val Thr Leu
               210                 215                 220
   ctg ctg gat acg ctg ccc atg ctc ggg aac gtc ctt ctg ctg tgc ttc      962
   Leu Leu Asp Thr Leu Pro Met Leu Gly Asn Val Leu Leu Leu Cys Phe
           225                 230                 235
   ttc gtc ttc ttc att ttc ggc atc gtt ggc gtc cag ctc tgg gct ggc     1010
   Phe Val Phe Phe Ile Phe Gly Ile Val Gly Val Gln Leu Trp Ala Gly
       240                 245                 250
   ctc ctg cgg aac cgc tgc ttc ctg gac agt gcc ttt gtc agg aac aac     1058
   Leu Leu Arg Asn Arg Cys Phe Leu Asp Ser Ala Phe Val Arg Asn Asn
   255                 260                 265                 270
   aac ctg acc ttc ctg cgg ccg tac tac cag acg gag gag ggc gag gag     1106
   Asn Leu Thr Phe Leu Arg Pro Tyr Tyr Gln Thr Glu Glu Gly Glu Glu
                   275                 280                 285
   aac ccg ttc atc tgc tcc tca cgc cga gac aac ggc atg cag aag tgc     1154
   Asn Pro Phe Ile Cys Ser Ser Arg Arg Asp Asn Gly Met Gln Lys Cys
               290                 295                 300
   tcg cac atc ccc ggc cgc cgc gag ctg cgc atg ccc tgc acc ctg ggc     1202
   Ser His Ile Pro Gly Arg Arg Glu Leu Arg Met Pro Cys Thr Leu Gly
           305                 310                 315
   tgg gag gcc tac acg cag ccg cag gcc gag ggg gtg ggc gct gca cgc     1250
   Trp Glu Ala Tyr Thr Gln Pro Gln Ala Glu Gly Val Gly Ala Ala Arg
       320                 325                 330
   aac gcc tgc atc aac tgg aac cag tac tac aac gtg tgc cgc tcg ggt     1298
   Asn Ala Cys Ile Asn Trp Asn Gln Tyr Tyr Asn Val Cys Arg Ser Gly
   335                 340                 345                 350
   gac tcc aac ccc cac aac ggt gcc atc aac ttc gac aac atc ggc tac     1346
   Asp Ser Asn Pro His Asn Gly Ala Ile Asn Phe Asp Asn Ile Gly Tyr
                   355                 360                 365
   gcc tgg att gcc atc ttc cag gtg atc acg ctg gaa ggc tgg gtg gac     1394
   Ala Trp Ile Ala Ile Phe Gln Val Ile Thr Leu Glu Gly Trp Val Asp
               370                 375                 380
   atc atg tac tac gtc atg gac gcc cac tca ttc tac aac ttc atc tat     1442
   Ile Met Tyr Tyr Val Met Asp Ala His Ser Phe Tyr Asn Phe Ile Tyr
           385                 390                 395
   ttc atc ctg ctc atc atc gtg ggc tcc ttc ttc atg atc aac ctg tgc     1490
   Phe Ile Leu Leu Ile Ile Val Gly Ser Phe Phe Met Ile Asn Leu Cys
       400                 405                 410
   ctg gtg gtg att gcc acg cag ttc tcg gag acg aag cag cgg gag agt     1538
   Leu Val Val Ile Ala Thr Gln Phe Ser Glu Thr Lys Gln Arg Glu Ser
   415                 420                 425                 430
   cag ctg atg cgg gag cag cgg gca cgc cac ctg tcc aac gac agc acg     1586
   Gln Leu Met Arg Glu Gln Arg Ala Arg His Leu Ser Asn Asp Ser Thr
                   435                 440                 445
   ctg gcc agc ttc tcc gag cct ggc agc tgc tac gaa gag ctg ctg aag     1634
   Leu Ala Ser Phe Ser Glu Pro Gly Ser Cys Tyr Glu Glu Leu Leu Lys
               450                 455                 460
   tac gtg ggc cac ata ttc cgc atc gtg gac agc aag tac ttc agc cgt     1682
   Tyr Val Gly His Ile Phe Arg Ile Val Asp Ser Lys Tyr Phe Ser Arg
           465                 470                 475
   ggc atc atg atg gcc atc ctt gtc aac acg ctg agc atg ggc gtg gag     1730
   Gly Ile Met Met Ala Ile Leu Val Asn Thr Leu Ser Met Gly Val Glu
       480                 485                 490
   tac cat gag cag ccc gag gag ctg act aat gct ctg gag atc agc aac     1778
   Tyr His Glu Gln Pro Glu Glu Leu Thr Asn Ala Leu Glu Ile Ser Asn
   495                 500                 505                 510
   atc gtg ttc acc agc atg ttt gcc ctg gag atg ctg ctg aag ctg ctg     1826
   Ile Val Phe Thr Ser Met Phe Ala Leu Glu Met Leu Leu Lys Leu Leu
                   515                 520                 525
   gcc tgc ggc cct ctg ggc tac atc cgg aac ccg tac aac atc ttc gac     1874
   Ala Cys Gly Pro Leu Gly Tyr Ile Arg Asn Pro Tyr Asn Ile Phe Asp
               530                 535                 540
   ggc atc atc gtg gtc atc agc gtc tgg gag atc gtg ggg cag gcg gac     1922
   Gly Ile Ile Val Val Ile Ser Val Trp Glu Ile Val Gly Gln Ala Asp
           545                 550                 555
   ggt ggc ttg tct gtg ctg cgc acc ttc cgg ctg ctg cgt gtg ctg aag     1970
   Gly Gly Leu Ser Val Leu Arg Thr Phe Arg Leu Leu Arg Val Leu Lys
       560                 565                 570
   ctg gtg cgc ttt ctg cca gcc ctg cgg cgc cag ctc gtg gtg ctg gtg     2018
   Leu Val Arg Phe Leu Pro Ala Leu Arg Arg Gln Leu Val Val Leu Val
   575                 580                 585                 590
   aag acc atg gac aac gtg gct acc ttc tgc acg ctg ctc atg ctc ttc     2066
   Lys Thr Met Asp Asn Val Ala Thr Phe Cys Thr Leu Leu Met Leu Phe
                   595                 600                 605
   att ttc atc ttc agc atc ctg ggc atg cac ctt ttc ggc tgc aag ttc     2114
   Ile Phe Ile Phe Ser Ile Leu Gly Met His Leu Phe Gly Cys Lys Phe
               610                 615                 620
   agc ctg aag aca gac acc gga gac acc gtg cct gac agg aag aac ttc     2162
   Ser Leu Lys Thr Asp Thr Gly Asp Thr Val Pro Asp Arg Lys Asn Phe
           625                 630                 635
   gac tcc ctg ctg tgg gcc atc gtc acc gtg ttc cag atc ctg acc cag      2210
   Asp Ser Leu Leu Trp Ala Ile Val Thr Val Phe Gln Ile Leu Thr Gln
       640                 645                 650
   gag gac tgg aac gtg gtc ctg tac aac ggc atg gcc tcc acc tcc tcc      2258
   Glu Asp Trp Asn Val Val Leu Tyr Asn Gly Met Ala Ser Thr Ser Ser
   655                 660                 665                 670
   tgg gcc gcc ctc tac ttc gtg gcc ctc atg acc ttc ggc aac tat gtg      2306
   Trp Ala Ala Leu Tyr Phe Val Ala Leu Met Thr Phe Gly Asn Tyr Val
                   675                 680                 685
   ctc ttc aac ctg ctg gtg gcc atc ctc gtg gag ggc ttc cag gcg gag      2354
   Leu Phe Asn Leu Leu Val Ala Ile Leu Val Glu Gly Phe Gln Ala Glu
               690                 695                 700
   ggc gat gcc aac aga tcc gac acg gac gag gac aag acg tcg gtc cac      2402
   Gly Asp Ala Asn Arg Ser Asp Thr Asp Glu Asp Lys Thr Ser Val His
           705                 710                 715
   ttc gag gag gac ttc cac aag ctc aga gaa ctc cag acc aca gag ctg      2450
   Phe Glu Glu Asp Phe His Lys Leu Arg Glu Leu Gln Thr Thr Glu Leu
       720                 725                 730
   aag atg tgt tcc ctg gcc gtg acc ccc aac ggg cac ctg gag gga cga      2498
   Lys Met Cys Ser Leu Ala Val Thr Pro Asn Gly His Leu Glu Gly Arg
   735                 740                 745                 750
   ggc agc ctg tcc cct ccc ctc atc atg tgc aca gct gcc acg ccc atg      2546
   Gly Ser Leu Ser Pro Pro Leu Ile Met Cys Thr Ala Ala Thr Pro Met
                   755                 760                 765
   cct acc ccc aag agc tca cca ttc ctg gat gca gcc ccc agc ctc cca      2594
   Pro Thr Pro Lys Ser Ser Pro Phe Leu Asp Ala Ala Pro Ser Leu Pro
               770                 775                 780
   gac tct cgg cgt ggc agc agc agc tcc ggg gac ccg cca ctg gga gac      2642
   Asp Ser Arg Arg Gly Ser Ser Ser Ser Gly Asp Pro Pro Leu Gly Asp
           785                 790                 795
   cag aag cct ccg gcc agc ctc cga agt tct ccc tgt gcc ccc tgg ggc      2690
   Gln Lys Pro Pro Ala Ser Leu Arg Ser Ser Pro Cys Ala Pro Trp Gly
       800                 805                 810
   ccc agt ggc gcc tgg agc agc cgg cgc tcc agc tgg agc agc ctg ggc      2738
   Pro Ser Gly Ala Trp Ser Ser Arg Arg Ser Ser Trp Ser Ser Leu Gly
   815                 820                 825                 830
   cgt gcc ccc agc ctc aag cgc cgc ggc cag tgt ggg gaa cgt gag tcc      2786
   Arg Ala Pro Ser Leu Lys Arg Arg Gly Gln Cys Gly Glu Arg Glu Ser
                   835                 840                 845
   ctg ctg tct ggc gag ggc aag ggc agc acc gac gac gaa gct gag gac      2834
   Leu Leu Ser Gly Glu Gly Lys Gly Ser Thr Asp Asp Glu Ala Glu Asp
               850                 855                 860
   ggc agg gcc gcg ccc ggg ccc cgt gcc acc cca ctg cgg cgg gcc gag    2882
   Gly Arg Ala Ala Pro Gly Pro Arg Ala Thr Pro Leu Arg Arg Ala Glu
           865                 870                 875
   tcc ctg gac cca cgg ccc ctg cgg ccg gcc gcc ctc ccg cct acc aag    2930
   Ser Leu Asp Pro Arg Pro Leu Arg Pro Ala Ala Leu Pro Pro Thr Lys
       880                 885                 890
   tgc cgc gat cgc gac ggg cag gtg gtg gcc ctg ccc agc gac ttc ttc    2978
   Cys Arg Asp Arg Asp Gly Gln Val Val Ala Leu Pro Ser Asp Phe Phe
   895                 900                 905                 910
   ctg cgc atc gac agc cac cgt gag gat gca gcc gag ctt gac gac gac    3026
   Leu Arg Ile Asp Ser His Arg Glu Asp Ala Ala Glu Leu Asp Asp Asp
                   915                 920                 925
   tcg gag gac agc tgc tgc ctc cgc ctg cat aaa gtg ctg gag ccc tac    3074
   Ser Glu Asp Ser Cys Cys Leu Arg Leu His Lys Val Leu Glu Pro Tyr
               930                 935                 940
   aag ccc cag tgg tgc cgg agc cgc gag gcc tgg gcc ctc tac ctc ttc    3122
   Lys Pro Gln Trp Cys Arg Ser Arg Glu Ala Trp Ala Leu Tyr Leu Phe
           945                 950                 955
   tcc cca cag aac cgg ttc cgc gtc tcc tgc cag aag gtc atc aca cac    3170
   Ser Pro Gln Asn Arg Phe Arg Val Ser Cys Gln Lys Val Ile Thr His
       960                 965                 970
   aag atg ttt gat cac gtg gtc ctc gtc ttc atc ttc ctc aac tgc gtc    3218
   Lys Met Phe Asp His Val Val Leu Val Phe Ile Phe Leu Asn Cys Val
   975                 980                 985                 990
   acc atc gcc ctg gag agg cct gac att gac ccc ggc agc acc gag cgg    3266
   Thr Ile Ala Leu Glu Arg Pro Asp Ile Asp Pro Gly Ser Thr Glu Arg
                   995                 1000                1005
   gtc ttc ctc agc gtc tcc aat tac atc ttc acg gcc atc ttc gtg gcg    3314
   Val Phe Leu Ser Val Ser Asn Tyr Ile Phe Thr Ala Ile Phe Val Ala
               1010                1015                1020
   gag atg atg gtg aag gtg gtg gcc ctg ggg ctg ctg tcc ggc gag cac    3362
   Glu Met Met Val Lys Val Val Ala Leu Gly Leu Leu Ser Gly Glu His
           1025                1030                1035
   gcc tac ctg cag agc agc tgg aac ctg ctg gat ggg ctg ctg gtg ctg    3410
   Ala Tyr Leu Gln Ser Ser Trp Asn Leu Leu Asp Gly Leu Leu Val Leu
       1040                1045                1050
   gtg tcc ctg gtg gac att gtc gtg gcc atg gcc tcg gct ggt ggc gcc    3458
   Val Ser Leu Val Asp Ile Val Val Ala Met Ala Ser Ala Gly Gly Ala
   1055                1060                1065                1070
   aag atc ctg ggt gtt ctg cgc gtg ctg cgt ctg ctg cgg acc ctg cgg    3506
   Lys Ile Leu Gly Val Leu Arg Val Leu Arg Leu Leu Arg Thr Leu Arg
                   1075                1080                1085
   cct cta agg gtc atc agc cgg gcc ccg ggc ctc aag ctg gtg gtg gag    3554
   Pro Leu Arg Val Ile Ser Arg Ala Pro Gly Leu Lys Leu Val Val Glu
               1090                1095                1100
   acg ctg ata tcg tcg ctc agg ccc att ggg aac atc gtc ctc atc tgc    3602
   Thr Leu Ile Ser Ser Leu Arg Pro Ile Gly Asn Ile Val Leu Ile Cys
           1105                1110                1115
   tgc gcc ttc ttc atc att ttt ggc atc ttg ggt gtg cag ctc ttc aaa    3650
   Cys Ala Phe Phe Ile Ile Phe Gly Ile Leu Gly Val Gln Leu Phe Lys
       1120                1125                1130
   ggg aag ttc tac tac tgc gag ggc ccc gac acc agg aac atc tcc acc    3698
   Gly Lys Phe Tyr Tyr Cys Glu Gly Pro Asp Thr Arg Asn Ile Ser Thr
   1135                1140                1145                1150
   aag gca cag tgc cgg gcc gcc cac tac cgc tgg gtg cga cgc aag tac    3746
   Lys Ala Gln Cys Arg Ala Ala His Tyr Arg Trp Val Arg Arg Lys Tyr
                   1155                1160                1165
   aac ttc gac aac ctg ggc cag gcc ctg atg tcg ctg ttc gtg ctg tca    3794
   Asn Phe Asp Asn Leu Gly Gln Ala Leu Met Ser Leu Phe Val Leu Ser
               1170                1175                1180
   tcc aag gat gga tgg gtg aac atc atg tac gac ggg ctg gat gcc gtg    3842
   Ser Lys Asp Gly Trp Val Asn Ile Met Tyr Asp Gly Leu Asp Ala Val
           1185                1190                1195
   ggt gtc gac cag cag cct gtg cag aac cac aac ccc tgg atg ctg ctg    3890
   Gly Val Asp Gln Gln Pro Val Gln Asn His Asn Pro Trp Met Leu Leu
       1200                1205                1210
   tac ttc atc tcc ttc ctg ctc atc gtc agc ttc ttc gtg ctc aac atg    3938
   Tyr Phe Ile Ser Phe Leu Leu Ile Val Ser Phe Phe Val Leu Asn Met
   1215                1220                1225                1230
   ttc gtg ggc gtc gtg gtc gag aac ttc cac aag tgc cgg cag cac cag    3986
   Phe Val Gly Val Val Val Glu Asn Phe His Lys Cys Arg Gln His Gln
                   1235                1240                1245
   gag gcg gag gag gcg cgg cgg cga gag gag aag cgg ctg cgg cgc cta    4034
   Glu Ala Glu Glu Ala Arg Arg Arg Glu Glu Lys Arg Leu Arg Arg Leu
               1250                1255                1260
   gag agg agg cgc agg agc act ttc ccc agc cca gag gcc cag cgc cgg    4082
   Glu Arg Arg Arg Arg Ser Thr Phe Pro Ser Pro Glu Ala Gln Arg Arg
           1265                1270                1275
   ccc tac tat gcc gac tac tcg ccc acg cgc cgc tcc att cac tcg ctg    4130
   Pro Tyr Tyr Ala Asp Tyr Ser Pro Thr Arg Arg Ser Ile His Ser Leu
       1280                1285                1290
   tgc acc agc cac tat ctc gac ctc ttc atc acc ttc atc atc tgt gtc    4178
   Cys Thr Ser His Tyr Leu Asp Leu Phe Ile Thr Phe Ile Ile Cys Val
   1295                1300                1305                1310
   aac gtc atc acc atg tcc atg gag cac tat aac caa ccc aag tcg ctg    4226
   Asn Val Ile Thr Met Ser Met Glu His Tyr Asn Gln Pro Lys Ser Leu
                   1315                1320                1325
   gac gag gcc ctc aag tac tgc aac tac gtc ttc acc atc gtg ttt gtc    4274
   Asp Glu Ala Leu Lys Tyr Cys Asn Tyr Val Phe Thr Ile Val Phe Val
               1330                1335                1340
   ttc gag gct gca ctg aag ctg gta gca ttt ggg ttc cgt cgg ttc ttc    4322
   Phe Glu Ala Ala Leu Lys Leu Val Ala Phe Gly Phe Arg Arg Phe Phe
           1345                1350                1355
   aag gac agg tgg aac cag ctg gac ctg gcc atc gtg ctg ctg tca ctc    4370
   Lys Asp Arg Trp Asn Gln Leu Asp Leu Ala Ile Val Leu Leu Ser Leu
       1360                1365                1370
   atg ggc atc acg ctg gag gag ata gag atg agc gcc gcg ctg ccc atc    4418
   Met Gly Ile Thr Leu Glu Glu Ile Glu Met Ser Ala Ala Leu Pro Ile
   1375                1380                1385                1390
   aac ccc acc atc atc cgc atc atg cgc gtg ctt cgc att gcc cgt gtg    4466
   Asn Pro Thr Ile Ile Arg Ile Met Arg Val Leu Arg Ile Ala Arg Val
                   1395                1400                1405
   ctg aag ctg ctg aag atg gct acg ggc atg cgc gcc ctg ctg gac act    4514
   Leu Lys Leu Leu Lys Met Ala Thr Gly Met Arg Ala Leu Leu Asp Thr
               1410                1415                1420
   gtg gtg caa gct ctc ccc cag gtg ggg aac ctg ggc ctt ctt ttc atg    4562
   Val Val Gln Ala Leu Pro Gln Val Gly Asn Leu Gly Leu Leu Phe Met
           1425                1430                1435
   ctc ctg ttt ttt atc tat gct gcg ctg gga gtg gag ctg ttc ggg agg    4610
   Leu Leu Phe Phe Ile Tyr Ala Ala Leu Gly Val Glu Leu Phe Gly Arg
       1440                1445                1450
   ctg gag tgc agt gaa gac aac ccc tgc gag ggc ctg agc agg cac gcc    4658
   Leu Glu Cys Ser Glu Asp Asn Pro Cys Glu Gly Leu Ser Arg His Ala
   1455                1460                1465                1470
   acc ttc agc aac ttc ggc atg gcc ttc ctc acg ctg ttc cgc gtg tcc    4706
   Thr Phe Ser Asn Phe Gly Met Ala Phe Leu Thr Leu Phe Arg Val Ser
                   1475                1480                1485
   acg ggg gac aac tgg aac ggg atc atg aag gac acg ctg cgc gag tgc    4754
   Thr Gly Asp Asn Trp Asn Gly Ile Met Lys Asp Thr Leu Arg Glu Cys
               1490                1495                1500
   tcc cgt gag gac aag cac tgc ctg agc tac ctg ccg gcc ctg tcg ccc    4802
   Ser Arg Glu Asp Lys His Cys Leu Ser Tyr Leu Pro Ala Leu Ser Pro
           1505                1510                1515
   gtc tac ttc gtg acc ttc gtg ctg gtg gcc cag ttc gtg ctg gtg aac    4850
   Val Tyr Phe Val Thr Phe Val Leu Val Ala Gln Phe Val Leu Val Asn
       1520                1525                1530
   gtg gtg gtg gcc gtg ctc atg aag cac ctg gag gag agc aac aag gag    4898
   Val Val Val Ala Val Leu Met Lys His Leu Glu Glu Ser Asn Lys Glu
   1535                1540                1545                1550
   gca cgg gag gat gcg gag ctg gac gcc gag atc gag ctg gag atg gcg    4946
   Ala Arg Glu Asp Ala Glu Leu Asp Ala Glu Ile Glu Leu Glu Met Ala
                   1555                1560                1565
   cag ggc ccc ggg agt gca cgc cgg gtg gac gcg gac agg cct ccc ttg   4994
   Gln Gly Pro Gly Ser Ala Arg Arg Val Asp Ala Asp Arg Pro Pro Leu
               1570                1575                1580
   ccc cag gag agt ccg ggc gcc agg gat gcc cca aac ctg gtt gca cgc   5042
   Pro Gln Glu Ser Pro Gly Ala Arg Asp Ala Pro Asn Leu Val Ala Arg
           1585                1590                1595
   aag gtg tcc gtg tcc agg atg ctc tcg ctg ccc aac gac agc tac atg   5090
   Lys Val Ser Val Ser Arg Met Leu Ser Leu Pro Asn Asp Ser Tyr Met
       1600                1605                1610
   ttc agg ccc gtg gtg cct gcc tcg gcg ccc cac ccc cgc ccg ctg cag   5138
   Phe Arg Pro Val Val Pro Ala Ser Ala Pro His Pro Arg Pro Leu Gln
   1615                1620                1625                1630
   gag gtg gag atg gag acc tat ggg gcc ggc acc ccc ttg ggc tcc gtt   5186
   Glu Val Glu Met Glu Thr Tyr Gly Ala Gly Thr Pro Leu Gly Ser Val
                   1635                1640                1645
   gcc tct gtg cac tct ccg ccc gca gag tcc tgt gcc tcc ctc cag atc   5234
   Ala Ser Val His Ser Pro Pro Ala Glu Ser Cys Ala Ser Leu Gln Ile
               1650                1655                1660
   cca ctg gct gtg tcg tcc cca gcc agg agc ggc gag ccc ctc cac gcc   5282
   Pro Leu Ala Val Ser Ser Pro Ala Arg Ser Gly Glu Pro Leu His Ala
           1665                1670                1675
   ctg tcc cct cgg ggc aca gcc cgc tcc ccc agt ctc agc cgg ctg ctc   5330
   Leu Ser Pro Arg Gly Thr Ala Arg Ser Pro Ser Leu Ser Arg Leu Leu
       1680                1685                1690
   tgc aga cag gag gct gtg cac acc gat tcc ttg gaa ggg aag att gac   5378
   Cys Arg Gln Glu Ala Val His Thr Asp Ser Leu Glu Gly Lys Ile Asp
   1695                1700                1705                1710
   agc cct agg gac acc ctg gat cct gca gag cct ggt gag aaa acc ccg   5426
   Ser Pro Arg Asp Thr Leu Asp Pro Ala Glu Pro Gly Glu Lys Thr Pro
                   1715                1720                1725
   gtg agg ccg gtg acc cag ggg ggc tcc ctg cag tcc cca cca cgc tcc   5474
   Val Arg Pro Val Thr Gln Gly Gly Ser Leu Gln Ser Pro Pro Arg Ser
               1730                1735                1740
   cca cgg ccc gcc agc gtc cgc act cgt aag cat acc ttc gga cag cac   5522
   Pro Arg Pro Ala Ser Val Arg Thr Arg Lys His Thr Phe Gly Gln His
           1745                1750                1755
   tgc gtc tcc agc cgg ccg gcg gcc cca ggc gga gag gag gcc gag gcc   5570
   Cys Val Ser Ser Arg Pro Ala Ala Pro Gly Gly Glu Glu Ala Glu Ala
       1760                1765                1770
   tcg gac cca gcc gac gag gag gtc agc cac atc acc agc tcc gcc tgc   5618
   Ser Asp Pro Ala Asp Glu Glu Val Ser His Ile Thr Ser Ser Ala Cys
   1775                1780                1785                1790
   ccc tgg cag ccc aca gcc gag ccc cat ggc ccc gaa gcc tct ccg gtg    5666
   Pro Trp Gln Pro Thr Ala Glu Pro His Gly Pro Glu Ala Ser Pro Val
                   1795                1800                1805
   gcc ggc ggc gag cgg gac ctg cgc agg ctc tac agc gtg gac gct cag    5714
   Ala Gly Gly Glu Arg Asp Leu Arg Arg Leu Tyr Ser Val Asp Ala Gln
               1810                1815                1820
   ggc ttc ctg gac aag ccg ggc cgg gca gac gag cag tgg cgg ccc tcg    5762
   Gly Phe Leu Asp Lys Pro Gly Arg Ala Asp Glu Gln Trp Arg Pro Ser
           1825                1830                1835
   gcg gag ctg ggc agc ggg gag cct ggg gag gcg aag gcc tgg ggc cct    5810
   Ala Glu Leu Gly Ser Gly Glu Pro Gly Glu Ala Lys Ala Trp Gly Pro
       1840                1845                1850
   gag gcc gag ccc gct ctg ggt gcg cgc aga aag aag aag atg agc ccc    5858
   Glu Ala Glu Pro Ala Leu Gly Ala Arg Arg Lys Lys Lys Met Ser Pro
   1855                1860                1865                1870
   ccc tgc atc tcg gtg gaa ccc cct gcg gag gac gag ggc tct gcg cgg    5906
   Pro Cys Ile Ser Val Glu Pro Pro Ala Glu Asp Glu Gly Ser Ala Arg
                   1875                1880                1885
   ccc tcc gcg gca gag ggc ggc agc acc aca ctg agg cgc agg acc ccg    5954
   Pro Ser Ala Ala Glu Gly Gly Ser Thr Thr Leu Arg Arg Arg Thr Pro
               1890                1895                1900
   tcc tgt gag gcc acg cct cac agg gac tcc ctg gag ccc aca gag ggc    6002
   Ser Cys Glu Ala Thr Pro His Arg Asp Ser Leu Glu Pro Thr Glu Gly
           1905                1910                1915
   tca ggc gcc ggg ggg gac cct gca gcc aag ggg gag cgc tgg ggc cag    6050
   Ser Gly Ala Gly Gly Asp Pro Ala Ala Lys Gly Glu Arg Trp Gly Gln
       1920                1925                1930
   gcc tcc tgc cgg gct gag cac ctg acc gtc ccc agc ttt gcc ttt gag    6098
   Ala Ser Cys Arg Ala Glu His Leu Thr Val Pro Ser Phe Ala Phe Glu
   1935                1940                1945                1950
   ccg ctg gac ctc ggg gtc ccc agt gga gac cct ttc ttg gac ggt agc    6146
   Pro Leu Asp Leu Gly Val Pro Ser Gly Asp Pro Phe Leu Asp Gly Ser
                   1955                1960                1965
   cac agt gtg acc cca gaa tcc aga gct tcc tct tca ggg gcc ata gtg    6194
   His Ser Val Thr Pro Glu Ser Arg Ala Ser Ser Ser Gly Ala Ile Val
               1970                1975                1980
   ccc ctg gaa ccc cca gaa tca gag cct ccc atg ccc gtc ggt gac ccc    6242
   Pro Leu Glu Pro Pro Glu Ser Glu Pro Pro Met Pro Val Gly Asp Pro
           1985                1990                1995
   cca gag aag agg cgg ggg ctg tac ctc aca gtc ccc cag tgt cct ctg    6290
   Pro Glu Lys Arg Arg Gly Leu Tyr Leu Thr Val Pro Gln Cys Pro Leu
       2000                2005                2010
   gag aaa cca ggg tcc ccc tca gcc acc cct gcc cca ggg ggt ggt gca    6338
   Glu Lys Pro Gly Ser Pro Ser Ala Thr Pro Ala Pro Gly Gly Gly Ala
   2015                2020                2025                2030
   gat gac ccc gtg tag ctcggggctt ggtgccgccc acggctttgg ccctggggtc   6393
   Asp Asp Pro Val
               2035
   tgggggcccc gctggggtgg aggcccaggc agaaccctgc atggaccctg acttgggtcc 6453
   cgtcgtgagc agaaaggccc ggggaggatg acggcccagg ccctggttct ctgcccagcg 6513
   aagcaggagt agctgccggg ccccacgagc ctccatccgt tctggttcgg gtttctccga 6573
   gttttgctac cagccgaggc tgtgcgggca actgggtcag cctcccgtca ggagagaagc 6633
   cgcgtctgtg ggacgaagac cgggcacccg ccagagaggg gaaggtacca ggttgcgtcc 6693
   tttcaggccc cgcgttgtta caggacactc gctgggggcc ctgtgccctt gccggcggca 6753
   ggttgcagcc accgcggccc aatgtcacct tcactcacag tctgagttct tgtccgcctg 6813
   tcacgccctc accaccctcc ccttccagcc accacccttt ccgttccgct cgggccttcc 6873
   cagaagcgtc ctgtgactct gggagaggtg acacctcact aaggggccga ccccatggag 6933
   taacgcgc                                                          6941